(19) **Europäisches Patentamt**
**European Patent Office**
**Office européen des brevets**

(11) **EP 3 754 018 A1**

(12) **EUROPEAN PATENT APPLICATION**
published in accordance with Art. 153(4) EPC

(43) Date of publication:
**23.12.2020 Bulletin 2020/52**

(21) Application number: **19753818.4**

(22) Date of filing: **15.02.2019**

(51) Int Cl.:
**C12N 15/09** (2006.01)     **C12N 5/10** (2006.01)
**C12N 15/11** (2006.01)     **C12Q 1/04** (2006.01)
**G01N 33/53** (2006.01)

(86) International application number:
**PCT/JP2019/005524**

(87) International publication number:
**WO 2019/160077 (22.08.2019 Gazette 2019/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.02.2018   JP 2018026421**

(71) Applicant: **Kyoto University**
**Kyoto-shi, Kyoto 606-8501 (JP)**

(72) Inventors:
• **HOTTA, Akitsu**
**Kyoto-shi, Kyoto 606-8501 (JP)**

• **XU, Huaigeng**
**Kyoto-shi, Kyoto 606-8501 (JP)**
• **KANEKO, Shin**
**Kyoto-shi, Kyoto 606-8501 (JP)**
• **WANG, Bo**
**Kyoto-shi, Kyoto 606-8501 (JP)**

(74) Representative: **Potter Clarkson**
**The Belgrave Centre**
**Talbot Street**
**Nottingham NG1 5GG (GB)**

Remarks:
The complete document including Reference Tables
and the Sequence Listing can be downloaded from
the EPO website

(54) **METHOD FOR PRODUCING LOW-ANTIGENIC CELL**

(57)     A method for producing, from a donor cell, a low-antigenic cell in which a rejection reaction is reduced in a case where the cell is allogeneically transplanted into a recipient, the method including: determining human leukocyte antigen (HLA) alleles for the donor cell and the recipient, respectively; specifying an HLA allele that is present in the donor cell but is not present in the recipient; and disrupting or modifying the specified HLA allele to obtain a cell population including a cell not expressing an HLA protein specific to the donor cell, in which the cell not expressing the HLA protein specific to the donor cell is the low-antigenic cell.

**Description**

Technical Field

The present invention relates to a method for producing a low-antigenic cell. More specifically, the present invention relates to a method for producing a low-antigenic cell, a kit for detecting a low-antigenic cell, a cell, a gRNA, and a method for specifying a target base sequence. Priority is claimed on Japanese Patent Application No. 2018-026421, filed February 16, 2018, the content of which is incorporated herein by reference.

Background Art

In a case of allogeneic transplantation in which donor cells are transplanted into a recipient (patient) who is the other person, transplanted cells are rejected due to an immune reaction.

A protein that plays the most important role in distinguishing between self cells and non-self cells is a cell surface protein called a human leukocyte antigen (HLA) or a major histocompatibility complex (MHC).

HLA is classified into class I and class II. Class I HLA proteins are expressed in most cell types in a body. The class I HLA protein has a function of forming a heterodimer with β2-Microglobulin (B2M) to be expressed on a cell surface, and presenting a peptide with respect to CD8-positive cytotoxic T cells to induce activation. The antigenic peptides presented are endogenous and have a length of 8 to 10 amino acids in many cases.

Genes classified into class I HLA are mainly six genes of an HLA-A gene, an HLA-B gene, an HLA-C gene, an HLA-E gene, an HLA-F gene, and an HLA-G gene. In addition, many pseudogenes (HLA-H, HLA-J, HLA-K, HLA-L, HLA-P, HLA-T, HLA-U, HLA-V, HLA-W, HLA-X, HLA-Y, and the like) are also known. Among the genes, three genes of the HLA-A gene, HLA-B gene, and HLA-C gene have particularly great sequence diversity among individuals and play a major role in identifying self cells and non-self cells in transplantation immunity.

Class II HLA proteins are mainly expressed in immune cells such as macrophages, dendritic cells, activated T cells and B cells, and the like. The class II HLA protein has a function of forming a heterodimer with an α chain and a β chain and presenting a peptide with respect to CD4 helper T cells to induce activation. The antigenic peptides presented are exogenous and have a length of 15 to 24 amino acids in many cases.

Genes classified into class II HLA are HLA-DR (α chain: HLA-DRA, β chain: HLA-DRB), HLA-DQ (α chain: HLA-DQA1, β chain: HLA-DQB1), HLA-DP (α chain: HLA-DPA1 or HLA-DPA2, β chain: HLA-DPB 1 or HLA-DPB2). In addition, many pseudogenes (HLA-DMA, HLA-DMB, HLA-DOA, HLA-DOB) are also known to exist.

HLA genes have sequence diversity and thus are involved in recognition of self and non-self cells at the cellular level. HLA matching is also particularly important for reducing immune rejection during allogeneic transplantation. For example, in transplantation of hematopoietic stem cells, it is recommended to find and transplant donors in which antigenicities in both alleles of HLA-A, HLA-B, HLA-C, and HLA-DR, that is, alleles at a total of 8 loci, are as consistent as possible.

In addition, in consideration of results of engraftment rates of kidney transplantation and the like, it has been reported that, as the degree of consistency of HLA antigenicity becomes high, engraftment efficiency becomes significantly high.

There is also an immune system induced by the absence of HLA antigens on a cell surface. NK cells express multiple inhibitory receptors. For example, it is known that a complex receptor of CD94 and NKG2A, which recognizes HLA-E and inhibits the action of NK cells, induces activation of NK cells when a cell without HLA-E is found, and attacks the cells. In addition, there is also a receptor family called Killer cell Immunoglobulin-like Receptor (KIR), which is classified into 2D and 3D according to the number of extracellular domains, and is also divided into L (long) and S (short) depending on lengths of intracellular domain. It is known that a 2DL1 receptor recognizes HLA-C2, 2DL2 and 2DL3 receptors recognize HLA-C 1, a 2DL4 receptor recognizes HLA-G, a 3DL1 recognizes HLA-Bw4, and a 3DL2 recognizes HLA-A3 or HLA-A11 to inhibit activity of NK cells. There are polymorphisms in KIR, and for example, almost all Japanese (98% or more) have 2DL1, 2DL3, or 3DL4, but it has been reported that a proportion of persons having 2DL2 is about 15%, which is rare.

In addition, because pluripotent stem cells such as iPS cells and ES cells have pluripotency capable of differentiating into various cell types, cell therapy and regenerative medicine in which pluripotent stem cells are differentiated into various cells and then transplanted to patients are attracting attention.

There have been reported cases in which ES cells are differentiated into nerve cells and transplanted into a bone marrow injury patient, and cases in which iPS cells are differentiated into retinal pigment epithelial cells and transplanted into an age-related macular degeneration patient. It has been reported that, in a case of transplanting such pluripotent stem cell-derived cells, it is important to match transplanted cells with an HLA type of a patient.

However, pluripotent stem cells require a great deal of costs and time for establishment, and thus there is a problem in that it is difficult to prepare cells with matching HLA types for each patient. As one means for solving this problem, for example, Patent Literature 1 discloses cells deficient in a B2M gene required for displaying class I HLA proteins on a cell surface.

In recent years, by genome editing technology that uses CRISPR-Cas9 and guide RNA (gRNA), it became possible to insert an arbitrary DNA fragment by inducing double-stranded DNA breaks (Double strand break, DSB) in genomic DNA depending on target base sequences of gRNA to delete part of genomic DNA, and by co-introducing Cas9 and gRNA with a template DNA having a partially homologous sequence near the double-stranded DNA cleavage induction site.

However, DSB induction in the genome editing technique is determined by a spacer base sequence of about 20 bases of gRNA that recognizes and binds to a target sequence and by a base sequence called a PAM sequence of about 3 to 5 bases. Accordingly, unless a base sequence of gRNA matches a base sequence of a target site, efficient DSB induction is difficult.

In addition, a risk is known, in which, in a case where a base sequence that is very similar to a base sequence of a target site, for example, a base sequence that differs by only one base, is present outside the target site, it will induce a DSB to sites other than the target site due to errors, resulting in induction of undesired genomic sequence mutations.

Citation List

Patent Literature

[Patent Literature 1]
PCT International Publication No. WO2012/145384

Summary of Invention

Technical Problem

Because the cells disclosed in Patent Literature 1 are deficient in a B2M gene, class I HLA proteins are not displayed on the cell surface. Accordingly, it is considered that an immune reaction in the case where the cells are allogeneically transplanted is inhibited. However, in a case where an HLA protein is not displayed on the cell surface, an antigen-presenting ability of the cell is lost. In a case where the cell loses its antigen-presenting ability, a B2M-deficient cell cannot present virus- or tumor-derived antigens when they are infected with viruses or the like or when they become tumors, and as a result, there is a risk in which it may help growth of viruses and tumors. In addition, a cell in which an HLA protein is not presented on the cell surface is attacked by NK cells that recognize "missing self."

Accordingly, it is possible to selectively disrupt only an arbitrary HLA gene by using the genome editing technique. However, there are multiple HLA genes, and there is a high degree of sequence homology between HLA genes, including many pseudogenes. In addition, because sequence diversity among individuals is large even with the same HLA genes, even when a target base sequence that matches a certain HLA gene sequence is identified, it cannot always be used in cells derived from other donors. For this reason, it is not easy to determine an appropriate target sequence for genome editing. Furthermore, in the genome editing technique of the related art, it takes a great deal of time to search for a cell in which a desired genome has been edited.

An object of the present invention is to provide a technique for producing a low-antigenic cell in which a rejection reaction is reduced in a case where the cell is allogeneically transplanted into a recipient.

Solution to Problem

The present invention includes the following aspects.

[1] A method for producing, from a donor cell, a low-antigenic cell in which a rejection reaction is reduced in a case where the cell is allogeneically transplanted into a recipient, the method including: determining human leukocyte antigen (HLA) alleles for the donor cell and the recipient, respectively; specifying an HLA allele that is present in the donor cell but is not present in the recipient; and disrupting or modifying the specified HLA allele to obtain a cell population including a cell not expressing an HLA protein specific to the donor cell, in which the cell not expressing the HLA protein specific to the donor cell is the low-antigenic cell.

[2] The production method according to [1], in which the HLA allele determined in the determining the HLA alleles for the donor cell and the recipient, respectively, includes an HLA-A allele, an HLA-B allele, and an HLA-C allele.

[3] The production method according to [1] or [2], further including: recovering the cell not expressing the HLA protein specific to the donor cell from the cell population after the obtaining of the cell population including the cell not expressing the HLA protein specific to the donor cell, in which the recovering includes bringing the cell population into contact with an HLA protein expression-inducing agent, and recovering the cell not expressing the HLA protein specific to the donor cell from the cell population using, as an index, expression of the HLA protein specific to the donor cell in the cell population brought into contact with the HLA protein expression-inducing agent.

[4] The production method according to [3], in which the HLA protein expression-inducing agent is interferon (IFN)-γ, IFN-α, IFN-β, interleukin (IL)-4, granulocyte macrophage colony-stimulating factor (GM-CSF), transforming growth factor (TGF)-α, or TGF-β.

[5] The production method according to any one of [1] to [4], in which the low-antigenic cell is a pluripotent stem cell.

[6] A kit for detecting a low-antigenic cell in which a rejection reaction is reduced in a case where the cell is allogeneically transplanted into a recipient, the kit comprising an HLA protein expression-inducing agent.

[7] The kit according to [6], in which the HLA protein expression-inducing agent is IFN-γ, IFN-α, IFN-β, IL-4, GM-CSF, TGF-α, or TGF-β.

[8] The kit according to [6] or [7], in which the low-antigenic cell is a pluripotent stem cell.

[9] An HLA protein expression-inducing agent consisting of IFN-γ, IFN-α, IFN-β, IL-4, GM-CSF, TGF-α, or TGF-β.

[10] A cell in which at least one HLA allele is disrupted or modified and at least one kind of HLA protein can be expressed.

[11] The cell according to [10], in which the HLA allele includes an HLA-A allele, an HLA-B allele, or an HLA-C allele.

[12] The cell according to [10] or [11], which is a low-antigenic cell in which a rejection reaction is reduced in a case where the cell is allogeneically transplanted into a recipient, and in which the disrupted or modified HLA allele is an HLA allele that is not present in the recipient.

[13] The cell according to any one of [10] to [12], which is a pluripotent stem cell.

[14] The cell according to any one of [10] to [13], in which at least one allele of a class II major histocompatibility complex transactivator (CIITA) allele, a regulatory factor X5 (RFX5) allele, a regulatory factor X associated protein (RFXAP) allele, or a regulatory factor X associated ankyrin containing protein (RFXANK) allele is further disrupted or modified.

[15] The cell according to any one of [10] to [14], in which the HLA-A allele and the HLA-B allele are disrupted and at least one kind of HLA-C protein can be expressed.

[16] The cell according to [15], in which the HLA-C protein is a protein encoded by one kind of allele selected from the group consisting of an HLA-C*01:02 allele, an HLA-C*02:02 allele, an HLA-C*03:03 allele, an HLA-C*03:04 allele, an HLA-C*04:01 allele, an HLA-C*05:01 allele, an HLA-C*06:02 allele, an HLA-C*07:01 allele, an HLA-C*07:02 allele, an HLA-C*08:01 allele, an HLA-C* 12:02 allele, and an HLA-C*16:01 allele.

[17] A method for producing, from a donor cell, a low-antigenic cell in which a rejection reaction is reduced in a case where the cell is allogeneically transplanted into a recipient, the method including: disrupting or modifying an allele including at least one of a CIITA allele, an RFX5 allele, an RFXAP allele, and an RFXANK allele of the donor cell, in which a cell in which the CIITA allele, the RFX5 allele, the RFXAP allele, or the RFXANK allele is disrupted or modified is the low-antigenic cell.

[18] A cell according to [17], in which the low-antigenic cell is a pluripotent stem cell.

[19] A cell in which an allele including at least one of a CIITA allele, an RFX5 allele, an RFXAP allele, and an RFXANK allele is disrupted or modified.

[20] The cell according to [19], which is a pluripotent stem cell.

[21] A gRNA targeting, as a target base sequence, a base sequence that is mapped to only one target HLA haplotype in a case where the base sequence is mapped to base sequence data of genomic DNA of all HLA haplotypes, but that is not mapped in a case where the base sequence is mapped to base sequence data of all genomic DNA except for HLA alleles.

[22] The gRNA according to [21], in which the target base sequence consists of a base sequence set forth in any of SEQ ID NOs: 3, 4, 7, 45 to 52, and 72 to 2459, or a base sequence in which one or several bases are deleted, substituted, or added at a 5' end of a base sequence set forth in any of SEQ ID NOs: 3, 4, 7, 45 to 52, and 72 to 2459.

[23] The gRNA according to [21] or [22], in which the target base sequence consists of a base sequence set forth in any of SEQ ID NOs: 3, 4, 7, and 45 to 52, or a base sequence in which one or several bases are deleted, substituted, or added at a 5' end of a base sequence set forth in any of SEQ ID NOs: 3, 4, 7, and 45 to 52.

[24] The gRNA according to any one of [21] to [23], in which the target base sequence is mapped to an exon 2 or 3 of an HLA gene.

[25] A gRNA targeting, as a target base sequence, a base sequence that is mapped to two or more target HLA haplotypes in a case where the base sequence is mapped to base sequence data of genomic DNA of all HLA haplotypes, but that is not mapped in a case where the base sequence is mapped to base sequence data of all genomic DNA except for HLA alleles.

[26] The gRNA according to [25], in which the target base sequence consists of a base sequence set forth in any of SEQ ID NOs: 53 to 55 and 2460 to 8013, or a base sequence in which one or several bases are deleted, substituted, or added at a 5' end of a base sequence set forth in any of SEQ ID NOs: 53 to 55 and 2460 to 8013.

[27] The gRNA according to [25] or [26], in which the target base sequence consists of a base sequence set forth in any of SEQ ID NOs: 53 to 55, or a base sequence in which one or several bases are deleted, substituted, or added at a 5' end of a base sequence set forth in any of SEQ ID NOs: 53 to 55.

[28] The gRNA according to any one of [25] to [27], in which the target base sequence is mapped to an exon 2 or 3 of an HLA gene.

[29] A method for specifying a target base sequence for producing, by genome editing, a low-antigenic cell in which a rejection reaction is reduced in a case where the cell is allogeneically transplanted into a recipient, the method including: mapping a candidate base sequence to base sequence data of genomic DNA of all HLA haplotypes; mapping the candidate base sequence to base sequence data of all genomic DNA except for HLA alleles; and specifying, as a target base sequence, the candidate base sequence that is mapped to only one target HLA haplotype in a case where the base sequence is mapped to base sequence data of genomic DNA of all HLA haplotypes, but that is not mapped in a case where the base sequence is mapped to base sequence data of all genomic DNA except for the HLA alleles.

[30] A method for specifying a target base sequence for producing, by genome editing, a low-antigenic cell in which a rejection reaction is reduced in a case where the cell is allogeneically transplanted into a recipient, the method including: mapping a candidate base sequence to base sequence data of genomic DNA of all HLA haplotypes; mapping the candidate base sequence to base sequence data of all genomic DNA except for HLA alleles; and specifying, as a target base sequence, the candidate base sequence that is mapped to two or more target HLA haplotypes in a case where the base sequence is mapped to base sequence data of genomic DNA of all HLA haplotypes, but that is not mapped in a case where the base sequence is mapped to base sequence data of all genomic DNA except for the HLA alleles.

Advantageous Effects of Invention

According to the present invention, it is possible to provide a technique for producing a low-antigenic cell in which a rejection reaction is reduced in a case where the cell is allogeneically transplanted into a recipient.

Brief Description of Drawings

Fig. 1(a) is a Venn diagram showing HLA alleles targeted by sgRNAs extracted in Experimental Example 1. Fig. 1(b) is a diagram showing which sites of an HLA-A allele, an HLA-B allele, and an HLA-C allele sgRNAs identified in Experimental Example 1 target.

Fig. 2 is a graph showing results of flow cytometry in Experimental Example 2.

Fig. 3 is a graph showing results of flow cytometry in Experimental Example 3.

Fig. 4(a) is a graph showing results of flow cytometry in Experimental Example 4. Fig. 4(b) is a diagram showing a schedule for an IFN-γ treatment of iPS cells and analysis of an expression level of an HLA-A protein in Experimental Example 4.

Fig. 5(a) is a diagram showing target bases and target sites of sgRNAs in Experimental Example 6. Fig. 5(b) is a photograph showing results of a T7 endonuclease I assay in Experimental Example 6.

Fig. 6(a) and Fig. 6(b) are graphs showing results of flow cytometry in Experimental Example 7.

Fig. 7 is a graph showing a proportion of base sequence mutation of each clone in Experimental Example 7.

Fig. 8(a) and Fig. 8(b) are graphs showing results of flow cytometry in Experimental Example 8.

Fig. 9(a) and Fig. 9(b) are graphs showing results of flow cytometry in Experimental Example 8.

Fig. 10(a) is a table showing a base mutation pattern of each clone recovered in Experimental Example 8. Fig. 10(b) shows a base sequence of a representative clone recovered in Experimental Example 8.

Fig. 11 is a graph showing results of flow cytometry in Experimental Example 9.

Fig. 12(a) is a table showing a base mutation pattern of each clone recovered in Experimental Example 9. Fig. 12(b) shows a base sequence of a representative clone recovered in Experimental Example 9.

Fig. 13(a) and Fig. 13(b) are tables showing a base mutation pattern of each clone recovered in Experimental Example 10. Fig. 13(c) shows a base sequence of a representative clone recovered in Experimental Example 10.

Fig. 14(a) is a table showing a base mutation pattern of each clone recovered in Experimental Example 11. Fig. 14(b) shows a base sequence of a representative clone recovered in Experimental Example 11.

Fig. 15(a) is a graph showing results of flow cytometry in Experimental Example 11. Fig. 15(b) is a table showing a base mutation pattern of each clone recovered in Experimental Example 11. Fig. 15(c) shows a base sequence of a representative clone recovered in Experimental Example 11.

Fig. 16(a) is a diagram showing target sites of sgRNAs in Experimental Example 12. Fig. 16(b) and Fig. 16(c) are graphs showing results of flow cytometry in Experimental Example 12.

Fig. 17(a) is a table showing a base mutation pattern of a clone recovered in Experimental Example 12. Fig. 17(b) shows a base sequence of a clone recovered in Experimental Example 12.

Fig. 18(a) is a graph showing results of flow cytometry in Experimental Example 13. Fig. 18(b) and Fig. 18(d) are tables showing a base mutation pattern of each clone recovered in Experimental Example 13. Fig. 18(c) and Fig. 18(e) show a base sequence of a representative clone recovered in Experimental Example 13.

Fig. 19(a) and Fig. 19(b) are graphs showing results of flow cytometry in Experimental Example 14. Fig. 19(c) is a table showing a base mutation pattern of each clone recovered in Experimental Example 14. Fig. 19(d) shows a base sequence of a representative clone recovered in Experimental Example 14.

Fig. 20(a) is a diagram showing target sites of sgRNAs in Experimental Example 15. Fig. 20(b) and Fig. 20(c) are graphs showing results of flow cytometry in Experimental Example 15.

Fig. 21(a) and Fig. 21(b) are schematic diagrams illustrating homologous recombination in Experimental Example 16. Fig. 21(c) is a graph showing results of flow cytometry in Experimental Example 15.

Fig. 22(a) and Fig. 22(b) are diagrams showing results of analysis of a base sequence in Experimental Example 16.

Fig. 23(a) and Fig. 23(b) are graphs showing results of flow cytometry in Experimental Example 17. Fig. 23(c) is a diagram showing results of analysis of a base sequence in Experimental example 17.

Fig. 24(a) is a graph showing results of flow cytometry in Experimental Example 18. Fig. 24(b) is a diagram showing results of analysis of a base sequence in Experimental example 18.

Fig. 25(a) to Fig. 25(e) are graphs showing results of flow cytometry in Experimental Example 19.

Fig. 26(a) to Fig. 26(f) are graphs showing results of flow cytometry in Experimental Example 20.

Fig. 27(a) to Fig. 27(e) are graphs showing results of flow cytometry in Experimental Example 21.

Fig. 28 is a schematic diagram showing a procedure of Experimental Example 22.

Fig. 29(a) to Fig. 29(e) are graphs showing results of flow cytometry in Experimental Example 22.

Fig. 30 is a schematic diagram showing a procedure of Experimental Example 23.

Fig. 31 is a graph showing results of Experimental Example 23.

Fig. 32 is a graph showing results of Experimental Example 24.

Fig. 33 is a graph showing results of Experimental Example 25.

Fig. 34(a) and Fig. 34(b) are graphs showing results of flow cytometry in Experimental Example 26. Fig. 34(c) is a graph showing results of Experimental Example 26.

Fig. 35(a) to Fig. 35(g) are graphs showing results of flow cytometry in Experimental Example 27.

Fig. 36 is a graph showing results of Experimental Example 28.

Fig. 37(a) to Fig. 37(f) are phase-contrast micrographs showing results of Experimental Example 29.

Fig. 38(a) to Fig. 38(f) are phase-contrast micrographs showing results of Experimental Example 30.

Fig. 39(a) and Fig. 39(b) are graphs showing results of flow cytometry in Experimental Example 31. Fig. 39(c) is a graph showing results of Experimental Example 31.

Fig. 40(a) and Fig. 40(b) are graphs showing results of flow cytometry in Experimental Example 32. Fig. 40(c) is a graph showing results of Experimental Example 32.

Fig. 41(a) is a diagram illustrating an experimental schedule in Experimental Example 33. Fig. 41(b) shows a photograph showing results of capturing fluorescence of EB-derived blood-cell-like cells in Experimental Example 33. Fig. 41(c) is a graph showing a survival rate of the EB-derived blood-cell-like cells in Experimental Example 33.

Fig. 42(a) is a graph showing results of flow cytometric analysis in Experimental Example 34. Fig. 42(b) is a graph showing results of Experimental Example 34.

Fig. 43(a) is a graph showing results of flow cytometric analysis in Experimental Example 35. Fig. 43(b) is a graph showing results of Experimental Example 35.

Fig. 44(a) and Fig. 44(b) are graphs showing results of flow cytometric analysis in Experimental Example 36.

Fig. 45(a) is a diagram illustrating an experimental schedule in Experimental Example 37. Fig. 45(b) shows a photograph showing results of capturing fluorescence of EB-derived blood-cell-like cells in Experimental Example 37. Fig. 45(c) is a graph showing a relative survival rate of the EB-derived blood-cell-like cells in Experimental Example 37.

Fig. 46 is a graph showing results of Experimental Example 38.

Fig. 47(a) is a table showing an HLA haplotype of 585A1-C7 residual cells used in Experimental Example 39, and an HLA haplotype of 585A1-C7 residual cells in which a CIITA gene was knocked out and which were produced in Experimental Example 39. Fig. 47(b) is a table showing a base mutation pattern of each clone obtained in Experimental Example 39. Fig. 47(c) shows a base sequence of a representative clone obtained in Experimental Example 39.

Fig. 48(a) to Fig. 48(d) are graphs showing results of flow cytometry in Experimental Example 40.

Fig. 49(a) to Fig. 49(c) are graphs showing results of flow cytometry in Experimental Example 41.

Fig. 50(a) to Fig. 50(c) are diagrams illustrating a procedure of Experimental Example 42.

Fig. 51(a) is a graph showing results of flow cytometry in Experimental Example 42. Fig. 51(b) is a table showing a base mutation pattern of each subclone obtained in Experimental Example 42.

Fig. 52(a) and Fig. 52(b) are tables showing a base mutation pattern of each subclone obtained in Experimental Example 42.

Fig. 53 is a table showing allele frequency of an HLC-C allele in various races.

Fig. 54 is a table showing a base mutation pattern of each subclone obtained in Experimental Example 43.

Fig. 55 is a table showing a base mutation pattern of each subclone obtained in Experimental Example 44. Fig. 56(a) and Fig. 56(b) are graphs showing results of flow cytometry in Experimental Example 45.

Fig. 57 is a graph showing results of flow cytometry in Experimental Example 46.

Fig. 58 is a table showing a base mutation pattern of each subclone obtained in Experimental Example 47.

Fig. 59 is a table showing a base mutation pattern of each subclone obtained in Experimental Example 48.

Fig. 60 is a table showing a base mutation pattern of each subclone obtained in Experimental Example 49.

Description of Embodiments

[Method for producing a low-antigenic cell with reduced rejection reaction]

(First embodiment)

A production method of the first embodiment is a method for producing, from a donor cell, a low-antigenic cell in which a rejection reaction is reduced in a case where the cell is allogeneically transplanted into a recipient, the method including: (a) determining HLA alleles for the donor cell and the recipient, respectively; (b) specifying an HLA allele that is present in the donor cell but is not present in the recipient; and (c) disrupting or modifying the specified HLA allele to obtain a cell population including a cell not expressing an HLA protein specific to the donor cell, in which the cell not expressing the HLA protein specific to the donor cell is the low-antigenic cell.

As described below in Examples, according to the production method of the first embodiment, it is possible to produce low-antigenic cells with reduced rejection reaction (graft-versus-host disease) when the cells are allogeneically transplanted into a recipient. Furthermore, as will be described later in Examples, low-antigenic cells produced by the production method of the first embodiment are less likely to be attacked by NK cells of a recipient even in a case where the cells are allogeneically transplanted into the recipient.

In the present specification, an HLA genetic locus may be referred to as an "HLA allele", and antigenic diversity of HLA proteins displayed on a cell surface may be referred to as an "HLA type." Hereinafter, each of the steps will be described.

(Step (a))

In the present step, HLA alleles of a donor cell and a recipient are respectively determined. The donor cell may be a human cell or a non-human animal cell. Furthermore, the donor cell may be a pluripotent stem cell or a differentiated cell. In the present specification, pluripotent stem cells mean embryonic stem cells (ES cells), induced pluripotent stem cells (iPS cells), and the like.

Furthermore, low-antigenic cells produced by the production method of the first embodiment are cells in which an HLA allele of donor cells has been disrupted or modified. Accordingly, the low-antigenic cell may be a pluripotent stem cell similar to the donor cell, or may be a differentiated cell.

Furthermore, a recipient is preferably an animal of the same species as the low-antigenic cells. The recipient may be a patient who is actually scheduled for transplantation, or may be a recipient having a hypothetical HLA allele that can be a recipient in the future.

HLA alleles can be determined by methods of the related art such as a PCR-rSSO method (PCR-reverse Sequence Specific Oligonucleotide), a PCR-SSP (Sequence specific primer) method, a PCR-SBT (Sequence based typing) method, and a next-generation sequencing method, and the determination can be carried out using a commercially available HLA typing kit or the like. In addition, HLA reporter, HLA-PRG, hla-genotyper, PHLAT, Optitype, neXtype, Athlates, HLAforest, SOAP-HLA, HLAminer, seq2HLA, GATK HLA Caller, and the like are known as software for performing HLA typing from sequence data of next-generation sequencers such as whole genome sequence (WGS), exome sequence (WES), and RNA-seq.

The HLA allele determined in the present step is preferably an HLA allele highly relevant to a rejection reaction upon cell transplantation, and preferably includes an HLA-A allele, an HLA-B allele, and an HLA-C allele.

The HLA allele determined in the present step may further include an HLA-DR allele, an HLA-DQ allele, and an HLA-DP allele, and may further include other HLA alleles.

(Step (b))

In the present step, an HLA allele not present in a recipient but present in donor cells (hereinafter, may be referred to as "donor-specific HLA allele") is specified. Donor-specific HLA alleles can be specified by comparing an HLA allele of donor cells and an HLA allele of a recipient.

The HLA allele is a base sequence encoded on genomic DNA and encodes the HLA protein. Serological classification of HLA proteins is called HLA serotype. Examples of methods for identifying an HLA serotype include a method of analyzing reactivity with a serum or an antibody that recognizes a specific HLA serotype; a method of collating a base sequence of DNA of an HLA allele or an RNA transcribed from the HLA allele with existing correspondence tables, IPD-IMGT database (https://www.ebi.ac.uk/ipd/imgt/hla/), and the like; and the like.

The HLA serotype generally refers to a difference in a first section (two-digit notation) of the HLA allele notation (http://hla.alleles.org/nomenclature/naming.html), but in the present specification, even in a case where an HLA serotype is the same serologically, in a case where amino acid sequences are different (non-synonymous substitution), it is determined that HLA alleles are different HLA alleles.

Specifically, it is determined whether alleles are the same HLA alleles or different HLA alleles from a difference between the first section and the second section (four-digit notation) in the HLA allele notation. Then, it is determined that whether HLA alleles in which the first section and the second section indicated by the HLA allele notation are the same HLA alleles. Furthermore, in a case where it is necessary to distinguish base sequences of genome such as in a case of determining a target sequence for genome editing, differences in the third section (a base substitution in a translation region not associated with an amino acid mutation) and the fourth section (a base substitution outside the translation region) may also be considered.

(Step (c))

In a case where cells having an HLA protein of an HLA serotype that are not present in a recipient are allogeneically transplanted into the recipient, a rejection reaction occurs, and thereby the transplanted cells are rejected. Accordingly, in the present step, a cell population is obtained, which contains cells that disrupt or modify the donor-specific HLA allele and do not express the donor cell-specific HLA protein (hereinafter, also referred to as "donor-specific HLA protein").

A cell that does not express a donor-specific HLA protein refers to a cell in which the expression of a specific HLA protein is negative due to the disruption of an HLA allele of donor cells, or a cell changed from a specific HLA type to another HLA type by modifying the HLA allele of the donor cells.

A cell that does not express the donor-specific HLA protein obtained in the present step is a low-antigenic cell. In a case where the donor-specific HLA protein is not present, the present step is unnecessary.

It is preferable that a B2M gene be not disrupted in low-antigenic cells produced by the production method of the first embodiment. As a result, a class I HLA protein is present on a cell surface, and even in a case where low-antigenic cells are allogeneically transplanted into a recipient, the cells are less likely to be attacked by NK cells of the recipient. In addition, because only part of HLA is disrupted and the remaining HLA retains an antigen-presenting ability, the antigen-presenting ability can be maintained even in a case where the cell is infected with a virus or becomes a tumor.

<Genome editing>

As will be described later in Examples, disruption or modification of HLA alleles of donor cells can be performed by, for example, genome editing. Disruption of HLA alleles can be performed by specifically cleaving the HLA alleles and inducing double-stranded DNA break (DSB). In a case where a base is deleted or added to cause a frameshift mutation in a DSB repairing process, HLA alleles are disrupted, and thereby an HLA protein is not expressed. In the present specification, disruption of the HLA alleles may be referred to as knockout of the HLA alleles.

Furthermore, by specifically cleaving the HLA alleles in the presence of donor DNA and inducing DSB, it is possible to induce homologous recombination in the DSB repairing process and modify the HLA alleles. Specifically, for example, an HLA-A*02:07 allele can be modified to an HLA-A*01:01 allele, as will be described later in Examples.

As the donor DNA, donor DNA having a sequence identity with a region including before and after a position of double-stranded DNA cleavage of genomic DNA and encoding a desired HLA allele may be used. The donor DNA may be single-stranded DNA or double-stranded DNA. The donor DNA may be a DNA having a single base sequence or a mixture of DNAs having a plurality of base sequences.

The phrase "having a sequence identity" means that 90% or more of base sequences match a region including double-strand break positions of donor DNA and target genomic DNA. In the donor DNA, 95% or more of base sequences preferably match and 99% or more of base sequences more preferably match the region including the double-strand break positions in the genomic DNA.

The donor DNA may be single-stranded DNA of about 50 to 5,000 bases or double-stranded DNA of about 50 to 5,000 base pairs. In a case where the donor DNA is single-stranded DNA, the donor DNA may have sequence identity with any of the double strands of genomic DNA.

<Sequence-specific DNA-cleaving enzyme>

In general, sequence-specific DNA-cleaving enzymes used for inducing DSB to perform genome editing are roughly classified into RNA-induced nucleases and artificial nucleases. The sequence-specific DNA-cleaving enzyme may be an RNA-induced nuclease or an artificial nuclease.

The sequence-specific DNA-cleaving enzyme is not particularly limited as long as it cleaves genomic DNA in a target sequence-specific manner to form a double-strand break. A length of a target sequence recognized by the sequence-specific DNA-cleaving enzyme may be, for example, about 10 to 60 bases.

Furthermore, the sequence-specific DNA-cleaving enzyme may be, for example, in a form in which a plurality of nickases are combined to cleave DNA. The nickase means an enzyme that forms a nick in a single strand of double-stranded DNA. For example, a double-strand break can be formed by forming nickases at both strands of double-stranded DNA at close locations on genomic DNA.

The RNA-induced nuclease is an enzyme that guides a short-chain RNA that binds to a target sequence and recruits a nuclease having two DNA cleavage domains (nuclease domains) to induce sequence-specific cleavage. Examples of RNA-induced nucleases include CRISPR-Cas family proteins. The CRISPR-Cas family proteins are roughly classified into class 1 and class 2. Class 1 includes type I, type III, and type IV, and class 2 includes type II, type V, and type VI.

Examples of CRISPR-Cas family protein include Cas9, Cpf1, CasX, CasY, Cas12, Cas13, C2C2, and the like. The RNA-derived nuclease may be a homologue of a CRISPR-Cas family protein or a modified CRISPR-Cas family protein. For example, it may be a nickase-modified nuclease in which one of two existing wild-type nuclease domains is modified to be inactive. Alternatively, it may be Cas9-HF, HiFi-Cas9, eCas9, or the like having improved target specificity.

Examples of Cas9 include those derived from Pseudomonas aeruginosa, Staphylococcus aureus, Streptococcus thermophilus, Geobacillus stearothermophilus, and the like. Examples of Cpf1 include those derived from Acidaminococcus, Lachnospira, Chlamydomonas, Francicella-Novicida, and the like.

The artificial nuclease is an artificial restriction enzyme having a DNA binding domain designed/produced to specifically bind to a target sequence and a nuclease domain (such as a DNA cleavage domain of FokI which is a restriction enzyme). Examples of artificial nucleases include Zinc finger nuclease (ZFN), Transcription activator-like effector nuclease (TALEN), meganucleases, and the like, but examples are not limited thereto.

«Introduction of sequence-specific DNA-cleaving enzyme»

For example, in a case where CRISPR-Cas is used as the sequence-specific DNA-cleaving enzyme introduced into donor cells, a target base sequence is determined by gRNA. Details of gRNA will be described later. A gRNA may be introduced into donor cells in the form of RNA, or may be introduced into the donor cells in the form of an expression vector and expressed intracellularly.

Examples of methods of preparing a gRNA in the form of RNA include a method of producing a construct in which a promoter such as T7 is added upstream of a nucleic acid fragment encoding gRNA, and synthesizing by an in vitro transcription reaction; a method of chemical synthesis; and the like. In the case of chemically synthesizing gRNA, chemically modified RNA may be used.

Examples of expression vectors include a plasmid vector or a viral vector which transcribes gRNA from Pol III promoter such as HI promoter or U6 promoter. In a case where gRNA is expressed using an expression vector, gRNA may be expressed constitutively or may be expressed under the control of an expression-inducible promoter.

Subsequently, Cas9 is prepared. Cas9 may be introduced into donor cells in the form of an expression vector that is expressed from a Pol II promoter, or may be introduced into the donor cells in the form of a purified protein. Examples of expression vectors include transposon vectors, virus vectors, episomal vectors, plasmid vectors, and the like.

In introduction of gRNA and Cas9 into donor cells, in a case where the gRNA and Cas9 are in the form of a viral vector, it is sufficient for the gRNA and Cas9 be added to a medium of the donor cells. Examples of viral vectors include adeno-associated virus vectors, adenovirus vectors, retrovirus vectors, lentivirus vectors, Sendai virus vectors, baculovirus vectors, and the like.

In a case where gRNA and Cas9 are transposon vectors, episomal vectors, plasmid vectors, or the like, or in a case where the gRNA is RNA and the Cas9 is a purified protein, they may be introduced into donor cells by a transfection reagent or an electroporation method.

As the transfection reagent, for example, it is possible to use Lipofectamine 2000, Lipofectamine 3000, CRISPRMAX, and RNAMAX (all of which are from Thermo Fisher Scientific); FuGENE 6 and FuGENE HD (both are from Promega); and the like.

In addition, the electroporation method can be performed using a device such as NEPA21 (Neppagene Co., Ltd.), Neon (Thermo Fisher Scientific), and 4D-Nucleofector (Lonza Co., Ltd.).

(Step (d))

In the step (c), it is possible to obtain the cell population which includes low-antigenic cells and which does not express a donor-specific HLA protein. However, the cell population obtained in step (c) may include, in addition to cells (low-antigenic cells) in which desired HLA alleles are disrupted or modified, cells in which HLA alleles are not disrupted or modified, cells in which HLA alleles are not completely disrupted or modified, and the like. Accordingly, after the step (c), a step (d) of recovering cells (low-antigenic cells) that do not express the donor-specific HLA protein may be further performed.

The step (d) includes step of bringing the cell population including low-antigenic cells into contact with an agent for inducing HLA protein expression (HLA protein expression-inducing agent), and recovering the cell not expressing the HLA protein specific to the donor cell from the cell population using, as an index, expression of the HLA protein specific to the donor cell in the cell population brought into contact with the HLA protein expression-inducing agent. The step of bringing the HLA protein expression-inducing agent into contact with the cell population can be performed, for example, by adding the HLA protein expression-inducing agent to a medium of the cell population.

In the present step, to recover cells that do not express the donor-specific HLA protein from the cell population using the expression of the donor-specific HLA protein as an index means to detect the presence of the expressed HLA protein, to detect the presence or absence of the expressed HLA protein, and to recover cells that do not express the donor-specific HLA protein.

Examples of HLA protein expression-inducing agents include cytokines such as IFN-γ, IFN-α, IFN-β, IL-4, GM-CSF, TGF-α, and TGF-β. These expression-inducing agents may be used alone or in combination of two or more kinds thereof.

In a case where the above-mentioned cytokines are used in human cells, the cytokines are preferably human-derived cytokines. The NCBI accession numbers of human IFN-γ protein are NP_000610.2 and the like. The NCBI accession numbers of human IFN-α protein are NP_076918.1, NP_000596.2, NP_066546.1, and the like. The NCBI accession numbers of human IFN-β protein are NP_002167.1 and the like. The NCBI accession numbers of human IL-4 protein are NP_000580.1, NP_758858.1, NP_001341919.1, and the like. The NCBI accession numbers of human GM-CSF protein are NP_000749.2, and the like. The NCBI accession numbers of human TGF-α protein are NP_001093161.1, NP_003227.1, NP_001295088.1, NP_001295087.1, and the like. The NCBI accession numbers of human TGF-β protein are NP_000651.3, XP_011525544.1, and the like.

The above-mentioned cytokine may have a mutation in an amino acid sequence described in each of the above-mentioned accession numbers as long as it has an activity of inducing the expression of HLA protein. More specifically, it may have an amino acid sequence in which one or several amino acids are deleted, substituted, or added in an amino acid sequence described in each of the above accession numbers. One or several amino acids may be, for example, 1 to 10 amino acids, for example, 1 to 5 amino acids, and for example, 1 to 3 amino acids. The above-mentioned cytokine may further have an amino acid sequence from which a signal peptide has been removed.

It is known that pluripotent stem cells generally do not express the HLA protein on the cell surface in an undifferentiated state, and therefore it is difficult to detect the HLA protein. In order to display the HLA protein, it is necessary to induce differentiation. However, a differentiation induction process is generally complicated and time-consuming. In addition, because cells that have once been differentiation-induced do not spontaneously return to an undifferentiated state, even in a case where an HLA protein-negative differentiated cell population is recovered, it is no longer a pluripotent stem cell.

The inventors of the present invention have revealed that it is possible induce the expression of HLA protein while maintaining pluripotency of pluripotent stem cells by causing the above-mentioned HLA protein expression-inducing agent to act on pluripotent stem cells.

Accordingly, the expression of the HLA protein in the cell population obtained in the step (d) is induced, and thereby pluripotent stem cells (low-antigenic cells) in which desired HLA alleles are disrupted or modified can be efficiently detected and recovered.

For example, after inducing the expression of HLA protein in cells, by staining the cells with an anti-HLA protein antibody, it is possible to distinguish HLA-expressing cells or HLA-non-expressing cells with a flow cytometer and recover them by sorting.

Alternatively, an anti-HLA protein antibody may be adsorbed on magnetic beads and brought into contact with HLA protein-expressing cells. Then, the cells to which the magnetic beads are bonded can be recovered using magnetic force.

Alternatively, cells expressing a specific HLA protein on a culture dish are labeled with an anti-HLA protein antibody or the like, unnecessary undesired cells are removed by suction or laser irradiation, and thereby only target low-antigenic cells may be recovered.

Alternatively, HLA protein-expressing cells on a culture dish are mixed with T cells that recognize an undesired HLA protein, only cells expressing the undesired HLA protein are attacked and removed, and thereby only target low-antigenic cells may be recovered.

The production method of the first embodiment may further include a step of disrupting or modifying an allele including at least one allele of a CIITA allele, an RFX5 allele, an RFXAP allele, and an RFXANK allele of the donor cell. A CIITA gene is a gene encoding a Class II Major Histocompatibility Complex Transactivator protein. Furthermore, an RFX5 gene is a gene encoding a Regulatory Factor X5 protein. Furthermore, an RFXAP gene is a gene encoding a Regulatory Factor X Associated Protein. Furthermore, an RFXANK gene is a gene encoding a Regulatory Factor X Associated Ankyrin Containing Protein.

The CIITA gene, together with the RFX5 gene, the RFXAP gene, and the RFXANK gene, encodes a transcription factor that controls transcriptional activation of HLA class II. Accordingly, for cells in which at least one of the CIITA allele, the RFX5 allele, the RFXAP allele, and the RFXANK allele is disrupted do not express the HLA class II protein, a rejection reaction in a case where the cells are allogeneically transplanted into a recipient is further reduced.

The NCBI accession numbers of the human CIITA gene are NM_000246.3., NM_001286402.1, NM_001286403.1, and the like. The NCBI accession numbers of human RFX5 gene are NM_000449.3, NM_001025603.1, and the like. The NCBI accession numbers of the human RFXAP gene is NM_000538.3, and the like. The NCBI accession numbers of the human RFXANK gene are NM_001278727.1, NM_001278728.1, NM_003721.3, NM_134440.2, and the like.

(Second embodiment)

A production method according to a second embodiment is a method for producing, from a donor cell, a low-antigenic cell in which a rejection reaction is reduced in a case where the cell is allogeneically transplanted into a recipient, the method including: disrupting or modifying an allele including at least one of a CIITA allele, an RFX5 allele, an RFXAP allele, and an RFXANK allele of the donor cell, in which a cell in which an allele including at least one of the CIITA allele, the RFX5 allele, the RFXAP allele, or the RFXANK allele is disrupted or modified is the low-antigenic cell. The production method of the present embodiment mainly differs from the production method of the first embodiment described above in that HLA alleles are not disrupted.

As described above, expression of the CIITA gene, the RFX5 gene, the RFXAP gene, and the RFXANK gene is known to be essential for expression of class II HLA protein. Accordingly, for cells in which at least one of the CIITA allele, the RFX5 allele, the RFXAP allele, and the RFXANK allele is disrupted do not express the HLA class II protein, a rejection reaction in a case where the cells are allogeneically transplanted into a recipient is reduced.

In the production method of the second embodiment, donor cells may be pluripotent stem cells similar to those described above, or may be differentiated cells.

Furthermore, a recipient is preferably an animal of the same species as the low-antigenic cells. The recipient may be a patient who is actually scheduled for transplantation, or may be a recipient having a hypothetical HLA allele that can be a recipient in the future.

Furthermore, as described above, cells in which a B2M allele is disrupted do not express an HLA class I protein even in a case where an HLA allele is wild type. The production method of the second embodiment may further include a step of disrupting a B2M allele of a donor cell.

Disruption of the CIITA allele or the B2M allele of the donor cell can be performed by, for example, genome editing. The genome editing is the same as described above.

[Kit for detecting low-antigenic cell]

In one embodiment, the present invention provides a kit for detecting a low-antigenic cell in which a rejection reaction is reduced in a case where the cell is allogeneically transplanted into a recipient, the kit including an HLA protein expression-inducing agent. Low-antigenic cells can be detected and recovered by the kit of the present embodiment. Accordingly, it can be said that the kit of the present embodiment is a kit for recovering low-antigenic cells.

In the kit of the present embodiment, an HLA protein expression-inducing agent is the same as described above, and examples thereof include cytokines such as IFN-γ, IFN-α, IFN-β, IL-4, GM-CSF, TGF-α, and TGF-β. The kit of the present embodiment may include one of these expression-inducing agent alone or may include two or more kinds thereof.

In the kit of this embodiment, low-antigenic cells may be pluripotent stem cells. As will be described later in Examples, according to the kit of the present embodiment, even in a case where low-antigenic cells are pluripotent stem cells, expression of the HLA protein is induced while still maintaining pluripotency of the low-antigenic cells.

[Cell]

(First embodiment)

A cell according to the first embodiment is a cell in which at least one HLA allele is disrupted or modified and at least one kind of HLA protein can be expressed. The cell of the first embodiment may be a pluripotent stem cell or a differentiated cell. In the cell of the first embodiment, at least one HLA allele that has been disrupted or modified is preferably a class I HLA allele.

Pluripotent stem cells generally have weak expression of HLA protein. The term "weak expression" means that, for example, expression of the HLA protein cannot be clearly detected even though cells are stained with an anti-HLA protein antibody and subjected to flow cytometric analysis.

However, as will be described later in Examples, in a case where the HLA protein expression-inducing agent is brought into contact with pluripotent stem cells, expression of an HLA protein is enhanced. In the cell of the first embodiment, the phrase "capable of expressing at least one HLA protein" means that the cell is a cell in which expression of the HLA protein is enhanced by bringing the HLA protein expression-inducing agent into contact with the cells even in a case where the cell is a cell in which expression of the HLA protein generally usually weak, such as pluripotent stem cells. Furthermore, the cell of the first embodiment may be a differentiated cell in which the HLA protein is expressed.

As will be described later in Examples, for example, cells in which a B2M allele is disrupted do not express an HLA class I protein even in a case where an HLA allele is wild type. The cell of the first embodiment do not include such cells.

In the cell of the first embodiment, a disrupted or modified HLA allele preferably includes a class I HLA allele, and preferably includes an HLA-A allele, an HLA-B allele, or an HLA-C allele.

Since the cell of the first embodiment expresses the at least one class I HLA protein, the cell is less likely to be attacked by NK cells of a recipient even in a case where the cell is allogeneically transplanted into the recipient. The at least one class I HLA protein includes an HLA-C protein, an HLA-E protein, an HLA-G protein, and the like. In the cell of the first embodiment, because only part of HLA is disrupted and the remaining HLA retains an antigen-presenting ability, the antigen-presenting ability can be maintained even in a case where the cell is infected with a virus or becomes a tumor.

The cell of the first embodiment may be a low-antigenic cell in which a rejection reaction is reduced in a case where the cell is allogeneically transplanted into a recipient, in which the disrupted or modified HLA allele is an HLA allele that is not present in the recipient. In the above-mentioned low-antigenic cell, a rejection reaction is reduced in a case where the cells is allogeneically transplanted to a recipient.

The low-antigenic cell of the first embodiment is preferably a cell of the same species as a recipient, and may be a human cell or a non-human animal cell. The recipient may be a patient who is actually scheduled for transplantation, or may be a recipient having a hypothetical HLA allele that can be a recipient in the future.

In the cell of the first embodiment, an allele including at least one of a CIITA allele, an RFX5 allele, an RFXAP allele, and an RFXANK allele may be further disrupted or modified. As described above, expression of the CIITA gene, the RFX5 gene, the RFXAP gene, and the RFXANK gene is known to be essential for expression of class II HLA protein. Accordingly, for cells in which at least one of the CIITA allele, the RFX5 allele, the RFXAP allele, and the RFXANK allele is disrupted do not express the HLA class II protein, a rejection reaction in a case where the cells are allogeneically transplanted into a recipient is further reduced.

(Second embodiment)

A cell according to the second embodiment is a cell in which an allele including at least one of a CIITA allele, an RFX5 allele, an RFXAP allele, and an RFXANK allele is disrupted or modified. As described above, a cell, in which an allele including at least one of the CIITA allele, the RFX5 allele, the RFXAP allele, and the RFXANK allele is disrupted or modified do not express the HLA class II protein, is a low-antigenic cell in which a rejection reaction in a case where the cell is allogeneically transplanted into a recipient is reduced.

The cell according to the second embodiment may express an HLA class I protein. The cell of the second embodiment may be useful for allogeneic transplantation into a recipient.

The cell of the second embodiment may be a pluripotent stem cell or a differentiated cell. Furthermore, as described above, cells in which a B2M allele is disrupted do not express an HLA class I protein even in a case where an HLA allele is wild type. In the cell of the second embodiment, a B2M allele may be further disrupted in addition to the at least one of the CIITA allele, the RFX5 allele, the RFXAP allele, and the RFXANK allele.

A low-antigenic cell of the second embodiment is preferably a cell of the same species as a recipient, and may be a human cell or a non-human animal cell. The recipient may be a patient who is actually scheduled for transplantation, or may be a recipient having a hypothetical HLA allele that can be a recipient in the future.

(Usage applications of low-antigenic cells)

The cells of the first embodiment and the second embodiment described above are low-antigenic cells in which a rejection reaction in a case where the cells are allogeneically transplanted into a recipient is reduced. Accordingly, the cells can be used for cell therapy and regenerative medicine.

In a case where low-antigenic cells are pluripotent stem cells, for example, the cells may be differentiated into nerve cells, hepatocytes, pancreatic islet cells, cardiomyocytes, kidney cells, hematopoietic stem cells, cytotoxic T cells, and the like, and then the cells may be transplanted into a patient.

Alternatively, the low-antigenic cells may be introduced into a patient after gene transfection. For example, after differentiation of pluripotent stem cells into T cells, gene transfection of a chimeric antigen receptor (CAR) is performed, and transplantation into cancer patients as CAR-T cells is performed.

[Method of specifying target base sequence]

(First embodiment)

In one embodiment, the present invention provides a method for specifying a target base sequence for producing, by genome editing, a low-antigenic cell in which a rejection reaction is reduced in a case where the cell is allogeneically transplanted into a recipient, the method including: mapping a candidate base sequence to base sequence data of genomic DNA of all HLA haplotypes; mapping the candidate base sequence to base sequence data of all genomic DNA except for HLA alleles; and specifying, as a target base sequence, the candidate base sequence that is mapped to only one target HLA haplotype in a case where the base sequence is mapped to base sequence data of genomic DNA of all HLA haplotypes, but that is not mapped in a case where the base sequence is mapped to base sequence data of all genomic DNA except for the HLA alleles.

By the method of the first embodiment, a target base sequence of the gRNA of the first embodiment to be described later can be specified. As described below, the gRNA of the first embodiment can specifically induce DSB in an HLA haplotype-specific manner, which makes the risk of off-target mutation introduction low.

As described above, an HLA gene has many pseudogenes, HLA gene sequences are highly homologous to each other, and sequence diversity among individuals is large. Accordingly, it is not easy to determine an appropriate target base sequence for genome editing using a sequence-specific DNA-cleaving enzyme.

A target base sequence of a sequence-specific DNA-cleaving enzyme specific to an HLA gene can be specified, for example, as follows. First, base sequence data of genomic DNA of all HLA haplotypes (base sequence data of all known HLA genes) is obtained. The base sequence data of all HLA genes can be obtained from, for example, IPD-IMGT/HLA database (https://www.ebi.ac.uk/ipd/imgt/hla/). As of July 2017, 12,544 base sequences of HLA class I genes and 4,622 base sequences of HLA class II genes have been registered in the present database.

Subsequently, a base sequence which is a target of a sequence-specific DNA-cleaving enzyme to be used is extracted from a base sequence of each HLA allele. For example, in a case where CRISPR-Cas is used as a sequence-specific DNA-cleaving enzyme, a base sequence, which includes a PAM sequence of about 3 to 5 bases (for example, "NGG" for Cas9 derived from Streptococcus pyogenes, "TTTN" for Cpf1 derived from Acidaminococcus) and which is usable as a target base sequence of gRNA, is extracted as a candidate base sequence.

In the method of the present embodiment, a target base sequence is assumed to include the PAM sequence. A length of the target base sequence including the PAM sequence varies depending on sequence-specific DNA-cleaving enzymes. For example, in a case where a sequence-specific DNA-cleaving enzyme is Cas 9 derived from Pseudomonas aeruginosa, a length of the target base sequence including the PAM sequence is preferably 19 to 33 bases, and is more preferably 20 to 24 bases. Furthermore, in a case where a sequence-specific DNA-cleaving enzyme is Cpf1 derived from Acidaminococcus, a length of the target base sequence including the PAM sequence is preferably 20 to 34 bases, and more preferably about 24 bases.

Subsequently, a candidate base sequence including the PAM sequence is mapped to the base sequence data of the genomic DNA of all HLA haplotypes.

Mapping refers to an operation of specifying a position having high sequence identity in a query base sequence (meaning a candidate base sequence herein) on a reference base sequence (meaning base sequence data of genomic DNA of all HLA haplotypes herein). A sequence identity between the query base sequence and the reference base sequence is preferably 90% or more, more preferably 95% or more, even more preferably 99% or more, and particularly preferably 100%.

The sequence identity of the query base sequence with respect to the reference base sequence can be determined as follows, for example. First, the reference base sequence and the query base sequence are aligned. Subsequently, the number of bases of matched bases in the reference base sequence and the query base sequence can be calculated, and a sequence identity can be determined according to Formula (1).

Sequence identity (%) = number of matched bases/total number of bases in

query base sequence x 100(1)

As will be described later in Examples, mapping can be performed using, for example, a Bowtie program (http://bowtie-bio.sourceforge.net/index.shtml). In addition, mapping may be performed using a sequence homology (sequence identity) search program other than the Bowtie program, such as BWA, BLAST, BLAT, SOAP, Novoalign, and TopHat.

Subsequently, the candidate base sequence is mapped to base sequence data of all genomic DNA except for HLA alleles. A reference human genome sequence (Hg19) or the like can be used as base sequence data of total genomic DNA. In addition, for mapping, the Bowtie program or a sequence homology search program other than the Bowtie program can be used.

Subsequently, a candidate base sequence, that is mapped to only one target HLA haplotype in a case where the base sequence is mapped to base sequence data of genomic DNA of all HLA haplotypes, but that is not mapped in a case where the base sequence is mapped to base sequence data of all genomic DNA except for HLA alleles, is specified as a target base sequence. The target HLA haplotype means a target HLA haplotype to be disrupted or modified.

As will be described later in Examples, a gRNA targeting the target base sequence specified by the method of the first embodiment as a target base sequence (the gRNA of the first embodiment to be described later) can induce a DSB specific to a target specific HLA haplotype, which makes the risk of off-target mutagenesis low.

Even though an HLA gene is composed of eight exons in many cases, the target base sequence preferably targets a protein coding region of the HLA gene. The target base sequence preferably targets an exon 1, 2, 3, or 4 encoding an extracellular domain of the HLA protein, and particularly preferably targets the exon 2 or 3.

Alternatively, in a case where the target base sequence is set at two positions, a base sequence between therebetween can be largely deleted. In order to utilize this deletion, two or more target base sequences may be designed to include a gene region encoding a protein of HLA gene.

Furthermore, the target base sequence preferably does not target other genomic regions other than the HLA gene or mitochondrial DNA. Furthermore, even in a case where a mismatch of several bases is allowed, it is preferable to select a target base sequence that has few sites targeting regions other than the HLA gene.

(Second embodiment)

In one embodiment, the present invention provides a method for specifying a target base sequence for producing, by genome editing, a low-antigenic cell in which a rejection reaction is reduced in a case where the cell is allogeneically transplanted into a recipient, the method including: mapping a candidate base sequence to base sequence data of genomic DNA of all HLA haplotypes; mapping the candidate base sequence to base sequence data of all genomic DNA except for HLA alleles; and specifying, as a target base sequence, the candidate base sequence that is mapped to two or more target HLA haplotypes in a case where the base sequence is mapped to base sequence data of genomic DNA of all HLA haplotypes, but that is not mapped in a case where the base sequence is mapped to base sequence data of all genomic DNA except for the HLA alleles.

The method of the second embodiment mainly differs from the above-described first embodiment in that, in the method of the second embodiment, the candidate base sequence that is mapped to two or more target HLA haplotypes is specified as a target base sequence when the candidate base sequence is mapped to base sequence data of genomic DNA of all HLA haplotypes.

In the method of the second embodiment, the candidate base sequence, the base sequence data of all genomic DNA of HLA haplotypes, the base sequence data of all genomic DNA, and mapping are the same as those of the above-described first embodiment.

By the method of the present embodiment, a target base sequence of the gRNA of the second embodiment to be described later can be specified. As will be described later, the gRNA of the second embodiment can cleave multiple target HLA genes (or alleles). Accordingly, it is possible to reduce the types of gRNA required in a case of disrupting or modifying multiple HLA genes. Thereby, costs of producing gRNAs can be reduced. In addition, the risk of off-target mutagenesis can be reduced by reducing the types of gRNA used.

[GRNA]

(First embodiment)

In one embodiment, the present invention provides a gRNA targeting, as a target base sequence, a base sequence that is mapped to only one target HLA haplotype in a case where the base sequence is mapped to base sequence data of genomic DNA of all HLA haplotypes, but that is not mapped in a case where the base sequence is mapped to base sequence data of all genomic DNA except for HLA alleles.

The gRNA of the first embodiment can specifically induce DSB in an HLA haplotype-specific manner, which makes the risk of off-target mutation introduction low.

In the present embodiment, the gRNA may be a complex of CRISPR RNA (crRNA) and trans-activated CRISPR RNA (tracrRNA), or a single synthetic gRNA (sgRNA) obtained by combining tracrRNA and crRNA. With any structure of gRNA, DSB can be induced in a target base sequence-specific manner.

A target base sequence is preferably specified by the method described above. As described above, in the present specification, the target base sequence is a base sequence including a PAM sequence. Accordingly, a base sequence obtained by removing the PAM sequence from the target base sequence is used as a spacer base sequence of crRNA or sgRNA.

In a case where the gRNA of the present embodiment is sgRNA, a base sequence of sgRNA can be the following base sequence.

First, a base sequence in which the PAM sequence has been removed from the target base sequence is used as a spacer base sequence. Then, a base sequence in which a scaffold sequence is linked to a 3' end of the spacer base sequence is designed. As the scaffold sequence, for example, a base sequence set forth in SEQ ID NO:39 can be used, but it is not limited thereto.

The base sequence set forth in SEQ ID NO: 39 may be a base sequence in which one or several bases have been deleted, substituted, or added in the base sequence set forth in SEQ ID NO: 39, as long as the base sequence set forth in SEQ ID NO: 39 functions as a scaffold sequence. One or several bases may be, for example, 1 to 10 bases, for example, 1 to 5 bases, and for example, 1 to 3 bases.

The designed sgRNA can be prepared by chemical synthesis or the like. The sgRNA may be prepared as RNA and directly introduced into a donor cell, or may be prepared as DNA and incorporated into an expression vector, introduced into a donor cell in the form of the expression vector, and expressed intracellularly.

In a case where the sgRNA is expressed in cells, a polymerase III promoter such as U6 promoter or HI promoter can be used. In this case, a base sequence at a 5' end of the sgRNA may be changed to G or GG in order to improve transcription efficiency. Even in a case where the base sequence at the 5' end of the sgRNA is changed to G or GG, a cleavage activity of Cas9 is hardly affected.

For example, in a case where a base sequence from which the PAM sequence is removed from the target base sequence is "5'-NNNNNNNNNNNNNNNNNNNN-3'" (SEQ ID NO: 62), a base sequence of the sgRNA that specifically cleaves the target base sequence is "5'-NNNNNNNNNNNNNNNNNNNNGUUUUAGAGCUAGAAAUAGCAAGUUAAAA UAAGGCUAGUCCGUUAUCAACUUGAAAAAGUGGCACCGAGUCGGUGCUUU UUUU-3'" (SEQ ID NO: 63).

The gRNA of the present embodiment may be a complex of crRNA and tracrRNA. In addition, the crRNA and tracrRNA may be directly introduced into a donor cell, or may be introduced into a donor cell in the form of an expression vector and expressed in the cell. In a case where the crRNA and tracrRNA are expressed in cells, a polymerase III promoter such as U6 promoter or HI promoter can be used. In this case, a base sequence at a 5' end of the crRNA or tracrRNA may be changed to G or GG in order to improve transcription efficiency. Even in a case where the base sequence at the 5' end of the crRNA or tracrRNA is changed to G or GG, a cleavage activity of Cas9 is hardly affected.

In a case where the gRNA of the present embodiment is a complex of crRNA and tracrRNA, base sequences of crRNA and tracrRNA can be the following base sequences.

First, a base sequence in which the PAM sequence has been removed from the target base sequence is used as a spacer base sequence. Then, a base sequence in which a scaffold sequence is linked to a 3' end of the spacer base sequence is designed to be a base sequence of the crRNA. For example, in a case where a base sequence from which the PAM sequence is removed from the target base sequence is "5'-NNNNNNNNNNNNNNNNNNNN-3'" (SEQ ID NO: 62), a base sequence of the crRNA is "5'-NNNNNNNNNNNNNNNNNNNNGUUUUAGAGCUAUGCUGUUUUG-3'" (SEQ ID NO: 64). In addition, a base sequence of the tracrRNA can be, for example, "5'-CAAAACAGCAUAGCAAGUUAAAAUAAGGCUAGUCCGUUAUCAACUUGAAA AAGUGGCACCGAGUCGGUGC-3'" (SEQ ID NO: 65).

The base sequence of the crRNA may have a mutation with respect to the base sequence set forth in SEQ ID NO:64 as long as it functions as crRNA. More specifically, it may be a base sequence in which one or several bases are deleted, substituted, or added in the base sequence of SEQ ID NO: 64. One or several bases may be, for example, 1 to 10 bases, for example, 1 to 5 bases, and for example, 1 to 3 bases.

In addition, the base sequence of the tracrRNA may be a base sequence in which one or several bases are deleted, substituted, or added in the base sequence of SEQ ID NO: 65, as long as it functions as tracrRNA. One or several bases may be, for example, 1 to 10 bases, for example, 1 to 5 bases, and for example, 1 to 3 bases.

The designed crRNA and tracrRNA can be prepared by chemical synthesis or the like. The crRNA and tracrRNA may be prepared as RNA and directly introduced into a donor cell, or may be prepared as DNA and incorporated into an expression vector, introduced into a donor cell in the form of the expression vector, and expressed intracellularly.

Specific examples of gRNAs of the first embodiment include a gRNA targeting a base sequence set forth in any of SEQ ID NOs: 3, 4, 7, 45 to 52, and 72 to 2459 as a target base sequence; or a gRNA targeting, as a target base sequence, a base sequence in which one or several bases are deleted, substituted, or added at a 5' end of a base sequence set forth in any of SEQ ID NOs: 3, 4, 7, 45 to 52, and 72 to 2459. One or several bases may be, for example, 1 to 10 bases, for example, 1 to 5 bases, and for example, 1 to 3 bases. For example, by shortening a 5' end of a spacer sequence by 2 to 3 bases, a binding ability to DNA can be reduced, and thereby sequence recognition specificity of CRISPR-Cas9 can be increased.

Among the examples, a gRNA is preferably a gRNA targeting a base sequence set forth in any of SEQ ID NOs: 3, 4, 7, and 45 to 52 as a target base sequence; or a gRNA targeting, as a target base sequence, a base sequence in which one or several bases are deleted, substituted, or added at a 5' end of a base sequence set forth in any of SEQ ID NOs: 3, 4, 7, and 45 to 52. As will be described later in Examples, gRNAs having these base sequences as a target base sequence disrupts or modifies HLA alleles in an HLA haplotype-specific manner, and thereby it is possible to produce low-antigenic cells in which a rejection reaction in a case where the cells are allogeneically transplanted into a recipient is reduced.

(Second embodiment)

In one embodiment, the present invention provides a gRNA targeting, as a target base sequence, a base sequence that is mapped to two or more target HLA haplotypes in a case where the base sequence is mapped to base sequence data of genomic DNA of all HLA haplotypes, but that is not mapped in a case where the base sequence is mapped to base sequence data of all genomic DNA except for HLA alleles.

Specific examples of gRNAs of the second embodiment include a gRNA targeting a base sequence set forth in any of SEQ ID NOs: 53 to 55 and 2460 to 8013 as a target base sequence; or a gRNA targeting, as a target base sequence, a base sequence in which one or several bases are deleted, substituted, or added at a 5' end of a base sequence set forth in any of SEQ ID NOs: 53 to 55 and 2460 to 8013. One or several bases may be, for example, 1 to 10 bases, for example, 1 to 5 bases, and for example, 1 to 3 bases. For example, by shortening a 5' end of a spacer sequence by 2 to 3 bases, a binding ability to DNA can be reduced, and thereby sequence recognition specificity of CRISPR-Cas9 can be increased.

Among the examples, a gRNA is preferably a gRNA targeting a base sequence set forth in any of SEQ ID NOs: 53 to 55 as a target base sequence; or a gRNA targeting, as a target base sequence, a base sequence in which one or several bases are deleted, substituted, or added at a 5' end of a base sequence set forth in any of SEQ ID NOs: 53 to 55.

Furthermore, as described above, a base sequence at a 5' end of the sgRNA may be changed to G or GG in order to improve transcription efficiency from a polymerase III promoter.

For example, in a case of focusing on the HLA-A allele, the HLA-A allele has two genes, which are an HLA-A gene derived from a father and an HLA-A gene derived from a mother, and the HLA-A genes may be the same as or different from each other in depending on cases. In order to retain an antigen-presenting ability of HLA-A, it is necessary to cleave only one HLA-A gene and induce knockout, and leave the opposite HLA-A gene. Alternatively, in a case where it is desired to induce complete knockout, it is necessary to cleave both HLA-A genes.

In general, in a case of targeting two genes to CRISPR-gRNA, it is general to design two gRNAs and induce DNA cleavage separately. Accordingly, in a case of knockout of multiple HLA genes, it is possible to use a gRNA specific to each HLA gene.

As will be described later in Examples, when the inventors of the present invention utilized a feature, in which HLA genes have high DNA sequence homology with each other, to search for a gRNA capable of inducing cleavage of multiple HLA genes with one gRNA (for example, both the father-derived sequence and the mother-derived sequence of the HLA-A gene, or the HLA-A gene and the HLA-B gene), such gRNA sequences could be identified.

As will be described later in Examples, by using the gRNA of the second embodiment, it is possible to reduce the types of gRNA required in a case of cleaving multiple HLA genes (or alleles). Thereby, costs of producing gRNAs can be reduced. In addition, the risk of off-target mutagenesis can be reduced by reducing the types of gRNA used.

[Other Embodiments]

In one embodiment, the present invention provides an HLA protein expression-inducing agent which includes IFN-γ, IFN-α, IFN-β, IL-4, GM-CSF, TGF-α, or TGF-β as an active ingredient, and which induces HLA protein expression while still maintaining pluripotency of pluripotent stem cells.

In one embodiment, the present invention provides a method for inducing expression of an HLA protein while still maintaining pluripotency of pluripotent stem cells, the method including a step of adding IFN-γ, IFN-α, IFN-β, IL-4, GM-CSF, TGF-α, or TGF-β in a medium of pluripotent stem cells.

Examples

Next, the present invention will be described in more detail with reference to examples, but the present invention is not limited to the following examples.

[Experimental Example 1]

(Design of gRNA)

It is difficult to design a base sequence of gRNA that cleaves only a specific HLA haplotype because an HLA gene has a large individual difference in sequence and homology between HLA genes is high. Specifically, for example, it was difficult to design a gRNA sequence that recognizes only an HLA-A*01:01 allele and does not recognize other HLA-A alleles, HLA genes other than HLA-A, and other genomic regions.

Accordingly, a target base sequence of gRNA that recognizes the HLA-A allele, the HLA-B allele, and the HLA-C allele was determined by the following method. First, from the IPD-IMGT/HLA database (https://www.ebi.ac.uk/ipd/imgt/hla/), an "hla_gen.fasta" file including base sequences of genomic DNA of all HLA haplotypes was downloaded.

Then, from the base sequences of genomic DNA of all HLA haplotypes, a base sequence corresponding to "NNNNNNNNNNNNNNNNNNNNNGG" (SEQ ID NO:40) was extracted as a candidate of a target base sequence of a gRNA of SpCas9 (sgRNA).

Then, base sequences of each candidate were mapped to the base sequences of all HLA haplotypes using a Bowtie program (http://bowtie-bio.sourceforge.net/index.shtml), and it was analyzed how many sites and to which HLA gene the base sequences of each candidate were mapped. The same operation can be performed using a sequence homology search program other than the Bowtie program.

Then, based on results of the Bowtie program, the results were classified into a case in which each sgRNA was bonded to only one of the HLA-A allele, the HLA-B allele or the HLA-C allele, and a case in which each sgRNA was bonded to two or more HLA alleles such as the HLA-A allele and the HLA-B allele, the HLA-A allele and the HLA-C allele, the HLA-B allele and the HLA-C allele, and the HLA-A allele, the HLA-B allele, and the HLA-C allele.

Then, all candidate base sequences were mapped to a reference human genome sequence (Hg19) using Bowtie, and it was checked whether or not the candidate base sequences were mapped to a genomic region other than the HLA allele. As a result, a candidate base sequence mapped only to the HLA allele was extracted as an intended target base sequence.

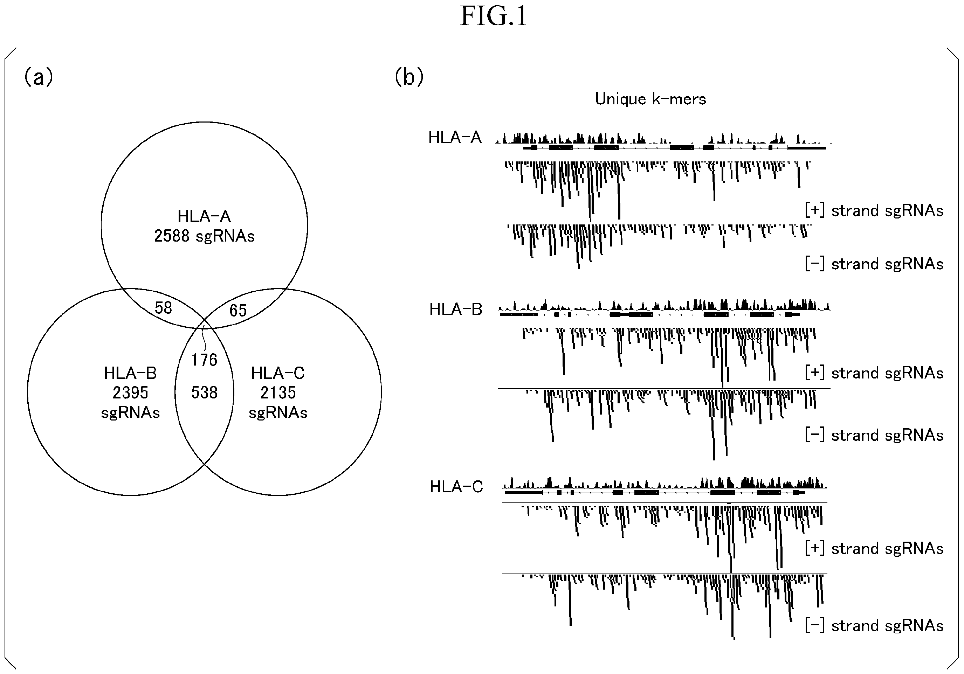

Fig. 1(a) is a Venn diagram showing which of the HLA-A allele, the HLA-B allele, and the HLA-C allele is targeted by a target base sequence extracted in the present experimental example. The numbers in the figure indicate the number of target base sequences of sgRNA. As a result, it was possible to determine a large number of target base sequences of sgRNA targeting only one of the HLA-A allele, the HLA-B allele, or the HLA-C allele, and target base sequences of sgRNA targeting two or more HLA alleles such as the HLA-A allele and the HLA-B allele, the HLA-A allele and the HLA-C allele, the HLA-B allele and the HLA-C allele, and the HLA-A allele, the HLA-B allele, and the HLA-C allele.

Fig. 1(b) is a diagram showing which sites of an HLA-A allele, an HLA-B allele, and an HLA-C allele a target base sequence of an sgRNA identified in the present experimental example targets. In Fig. 1(b), boxes connected by lines indicate exons of each HLA gene, and the HLA-A allele has an exon 1 on the left side, and the HLA-B allele and the HLA-C allele have an exon 1 on the right side. In addition, the term "[+] strand sgRNAs" indicates target base sequences targeting plus-strand DNA, the term "[-] strand sgRNAs" indicates target base sequences targeting minus-strand DNA, and the term "Unique k-mers" indicates a set of 10 to 16 mer base sequences (also referred to as "k-mer sequences") that is present only at one site on the human genome.

As a result, it was revealed that a large number of sgRNA target sequences could be found at a site at which a peak of unique k-mers was high. A considerable number of target base sequences could be found in both the plus-strand DNA and the minus-strand DNA.

On SEQ ID NOs: 3, 4, 7, 45 to 52, and 72 to 2459, a base sequence is shown, which is extracted as a base sequence that is mapped to only one target HLA haplotype in a case where the base sequence is mapped to base sequence data of genomic DNA of all HLA haplotypes, but that is not mapped in a case where the base sequence is mapped to base sequence data of all genomic DNA except for HLA alleles. In addition, Table 1 shows, as an example, the number of target HLA alleles and target HLA alleles for some base sequences.

[Table 1]

| SEQ ID NO. | Number of target HLA alleles | Target HLA alleles |
|---|---|---|
| 72 | 1 | A*11:01:01:02 |

(continued)

| SEQ ID NO. | Number of target HLA alleles | Target HLA alleles |
|---|---|---|
| 73 | 1 | A*11:05 |
| 74 | 1 | A*11:01:01:02 |
| 75 | 1 | A*02:43N |
| 76 | 1 | B*73:01 |

In addition, on SEQ ID NOs: 53 to 55 and 2460 to 8013, a base sequence is shown, which is extracted as a base sequence that is mapped to two or more target HLA haplotypes in a case where the base sequence is mapped to base sequence data of genomic DNA of all HLA haplotypes, but that is not mapped in a case where the base sequence is mapped to base sequence data of all genomic DNA except for HLA alleles. In addition, Table 2 shows, as an example, the number of target HLA alleles and target HLA alleles for some base sequences.

[Table 2]

| SEQ ID NO. | Number of target HLA alleles | Target HLA alleles |
|---|---|---|
| 2460 | 44 | A*02:01:01:01, A*02:01:01:02L, A*02:01:01:03, A*02:01:01:04, A *02:01:01:05, A*02: 01:01:06, A*02:01:120, A*02:01:121, A*02:02: 01:01, A*02:02:01:02, A*02:03:01, A*02: 03:03, A*02:05:01, A*02 :06:01:01, A*02:06:01:02, A*02:06:01:03, A*02:06:22, A*02: 07:01, A*02:10, A*02:193, A*02:251, A*02:259, A*02:264, A*02:265, A*02:266, A*02: 269, A*02:279, A*02:32N, A*02:376, A*02:43N, A*02:455, A*02:48, A*02:51, A*02:533, A*02:53N, A*02:602, A *02:603, A*02:60:01, A*02:68, A*02:77, A*02:81, A*02:89, A*02 : 95, A*69:01 |
| 2461 | 2 | A*80:01:01:01, A*80:01:01:02 |
| 2462 | 138 | A*01:01:01:01, A*01:01:01:02N, A*01:01:38L, A*01:01:71, A*01: 01:73, A*01:02, A*01: 03, A*01:04N, A*01:09, A*01:11N, A*01:1 4, A*01:16N, A*01:194, A*01:20, A*02:57, A*02:65, A*03:01:01: 01, A*03:01:01:02N, A*03:01:01:03, A*03:01:01:04, A*03:01:01: 0 5, A*03:01:01:06, A*03:02:01, A*03:11N, A*03:213, A*03:218, A *03:21N, A*03:224, A*03:231, A*03:234Q, A*03:36N, A*03:89:0 2, A*11:01:01:01, A*11:01:18, A*11:02:01, A*11:05, A*11:110, A*11:183, A*11:190, A*11:210N, A*11:25:01, A*11:50Q, A*11:6 0, A*11:69N, A*11:74, A*11:75, A*11:77, A*23:01:01, A*23:09, A*23:17, A*23:19N, A*23: 38N, A*24:02:01:01, A*24:02:01:02L, A*24:02:01:03, A*24:02:01:04, A*24:02:01:05, A*24:02:03Q, A*2 4:02:10, A*24:03:01, A*24:07:01, A*24:08, A*24:09N, A*24:10:0 1, A*24:11N, A*24:152, A*24:20, A*24:215, A*24:252N, A*24:2 93, A*24:56, A*24:61, A*24:86N, A*25:01:01, A*26:01:01:01, A* 26:01:01:02, A*26:01:39, A*26:04, A*26:07: 02, A*26:107N, A*26 :115, A*26:11N, A*26:15, A*26:50, A*29:01:01:01, A*29:01:01:0 2N, A*29:02:01:01, A*29:02:01:02, A*29:02:01:03, A*29:02:17, A *29:03, A*29:46, A*31: 01:02:01, A*31:01:02:02, A*31:01:02:03N, A*31:01:04, A*31:01:23, A*31:01:24, A*31: 04, A*31:14N, A*31: 36, A*31:46, A*32:01:01, A*32:06, A*32:69, A*33:01:01, A*33:0 3: 01, A*33:07, A*34:01:01, A*34:02:01, A*34:14, A*36:01, A*43 :01, A*66:01:01, A*66: 17, A*68:01:01:01, A*68:01:01:02, A*68:0 1:02:01, A*68:01:02:02, A*68:01:02:03, A*68: 02:01:01, A*68:02:0 1:02, A*68:02:01:03, A*68:02:02, A*68:03:01, A*68:07, A*68:08: 01, A*68:139, A*68:140, A*68:17, A*68:18N, A*68:22, A*68:71, A*74:01:01, A*74:01: 02, A*74:01:03, A*74:02:01:01, A*74:02:01: 02 |
| 2463 | 2 | A*80:01:01:01, A*80:01:01:02 |
| 2464 | 7 | A*30:01:01, A*30:01:11, A*30:02:01:01, A*30:02:01:02, A*30:02: 01:03, A*30:04:01, A*30:89 |

Furthermore, Table 3 shows the number of target HLA alleles and the number of extracted target base sequences, regarding a base sequence extracted as a base sequence that is mapped to two or more target HLA haplotypes in a case where the base sequence is mapped to base sequence data of genomic DNA of all HLA haplotypes, but that is not mapped in a case where the base sequence is mapped to base sequence data of all genomic DNA except for HLA alleles.

[Table 3]

| Number of target HLA alleles | Number of target base sequences |
| --- | --- |
| 2 | 378 |
| 3 | 115 |
| 4 | 88 |
| 5~10 | 537 |
| 11~100 | 2604 |
| 101~200 | 926 |
| 201~300 | 547 |
| 301~400 | 173 |
| 401~500 | 65 |
| 501 or more | 121 |

[Experimental Example 2]

(Induction 1 of HLA protein expression by stimulation of iPS cells)

An attempt was made to stimulate undifferentiated iPS cells to induce HLA protein expression. Specifically, 100 ng/mL of lipopolysaccharide (LPS), 100 ng/mL of TNF-$\alpha$, or 50 ng/mL of IFN-$\gamma$ was added to a medium of iPS cells and treated for 48 hours. Thereafter, flow cytometric analysis was performed using an anti-HLA-ABC antibody (product number: 311418, BIOLEGEND) to examine expression of HLA protein.

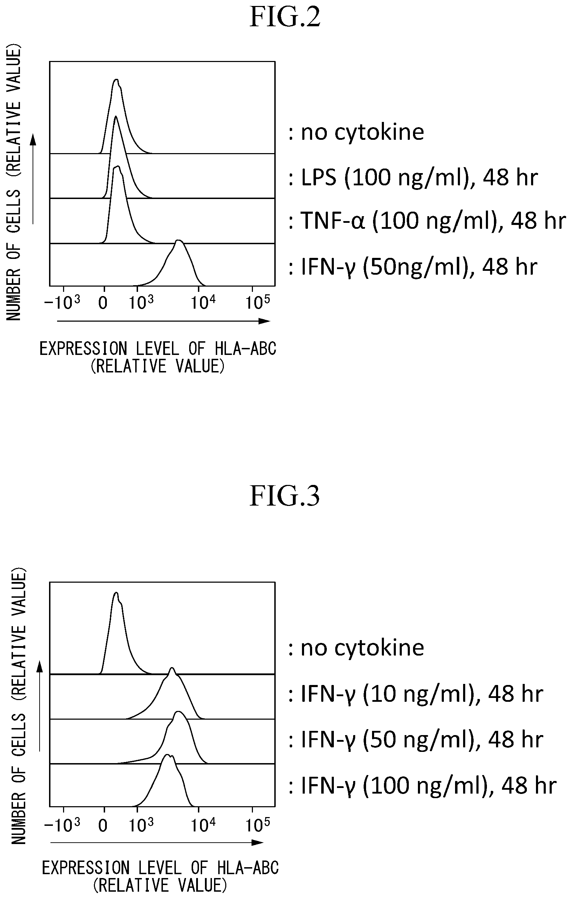

Fig. 2 is a graph showing results of flow cytometry. As a result, it was revealed that the addition of IFN-$\gamma$ increased the expression of HLA protein.

[Experimental Example 3]

(Induction 2 of HLA protein expression by stimulation of iPS cells)

10 ng/mL, 50 ng/mL, or 100 ng/mL of IFN-$\gamma$ was added to a medium of iPS cells and treated for 48 hours. Thereafter, flow cytometric analysis was performed using an anti-HLA-ABC antibody (product number: 311418, BIOLEGEND) to examine expression of HLA protein.
Fig. 3 is a graph showing results of flow cytometry. As a result, it was revealed that the addition of IFN-$\gamma$ increased the expression of HLA protein. As a result, it was revealed that the addition of any concentration of IFN-$\gamma$ increased the expression of HLA protein.

[Experimental Example 4]

(Induction 3 of HLA protein expression by stimulation of iPS cells)

50 ng/mL of IFN-$\gamma$ was added to a medium of iPS cells and treated for 4 hours, 8 hours, 16 hours, 24 hours, and 48 hours. Thereafter, flow cytometric analysis was performed using anti-HLA-A2 antibody (product number:740082, BD) to examine expression of HLA-A2 protein.

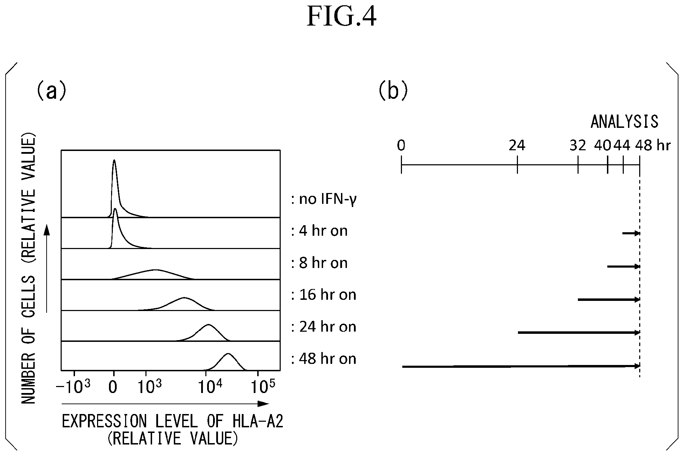

Fig. 4(a) is a graph showing results of flow cytometry. In addition, Fig. 4(b) is a diagram showing a schedule for an IFN-$\gamma$ treatment of iPS cells and analysis of an expression level of an HLA-A protein. As a result, it was revealed that the longer the IFN-$\gamma$ treatment time, the higher the expression of HLA protein. Furthermore, as a result of further examination, it was revealed that after the iPS cells were treated with IFN-$\gamma$ for 48 hours, the expression of HLA protein was maintained even 7 days after removal of IFN-$\gamma$ from the medium.

[Experimental Example 5]

(Determination of HLA haplotype of cells)

An HLA haplotype of each cell (a pair of each HLA allele inherited from parents) was determined by the following method.

First, a FASTQ sequence file was created for each of 604B1 iPS cells and 1383D2 iPS cells based on Whole Exome Sequencing (WES) analysis results of the 604B1 iPS cells and Whole Genome Sequencing (WGS) analysis results of the 1383D2 iPS cells.

Subsequently, each FASTQ sequence file was mapped to a reference human genome sequence (Hg19) to create a BAM file. Subsequently, each of the above-described BAM files was analyzed using software (name "HLA-genotyper," https://pypi.python.org/pypi/hla-genotyper/0.4.2b1) to determine an HLA haplotype of each cell.

In addition, for 585A1 iPS cells, extracted genomic DNA was sent to HLA Research Institute (http://hla.or.jp/), and an HLA haplotype was determined by a fluorescent bead method (PCR-rSSO/Luminex method). Furthermore, for 604B1 iPS cells, an HLA haplotype was determined in the same manner as for 585A1 iPS cells.

Furthermore, regarding peripheral blood mononuclear cells (PBMC), cells for which HLA haplotype information was provided by the vendors (CTL, Wako) were used.

[Experimental Example 6]

(Examination on genomic cleavage activity of sgRNA)

Among the sgRNAs targeting the target base sequence specified in Experimental Example 1, A0207-ex3-g1 (where a target base sequence is shown in SEQ ID NO: 3), A0207-ex3-g2 (where a target base sequence is shown in SEQ ID NO: 4), and A0207-ex3-g4 (where a target base sequence is shown in SEQ ID NO: 7), which were sgRNAs which cleaved only an A*02:07 allele and of which base sequences did not match an A*32:01 allele, were selected.

A base sequence of each sgRNA used in each of the following experimental examples was a base sequence in which a scaffold sequence set forth in SEQ ID NO:39 was linked to a 3' side of a base sequence from which a PAM sequence was removed from a target base sequence of each sgRNA. For example, in a case where a base sequence from which a PAM sequence was removed from a target base sequence was "5'-NNNNNNNNNNNNNNNNNNNN-3"' (SEQ ID NO:62), a base sequence of an sgRNA used was "5'-NNNNNNNNNNNNNNNNNNNNGUUUUAGAGCUAGAAAUAGCAAGUUAAAA UAAGGCUAGUCCGUUAUCAACUUGAAAAAGUGGCACCGAGUCGGUGCUUU UUUU-3"' (SEQ ID NO:63). Furthermore, in each of the following experimental examples, each sgRNA was introduced into a cell in the form of an expression vector and expressed in the cell to be used.

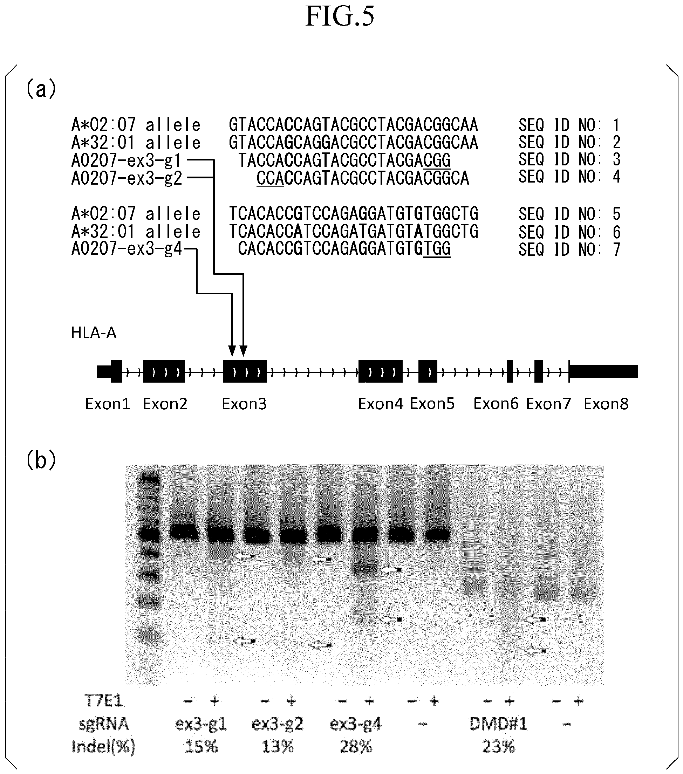

Fig. 5(a) is a diagram showing target bases and target sites of sgRNAs found in an exon 3 region of HLA-A. In Fig. 5 (a), a box indicates an exon of an HLA-A*02:07 gene, and an arrow indicates a position of a target base sequence of each sgRNA. Furthermore, Fig. 5(a) also shows a base sequence of a wild type A*02:07 allele and a base sequence of a wild type A*32:01 allele. Portions of both alleles at which sequences differ are shown with capital letters, and a PAM sequence of sgRNA is shown with an underline.

Subsequently, A0207-ex3-g1, A0207-ex3-g2, and A0207-ex3-g4 were each transfected together with purified Cas9 protein into 1383D2 iPS cells having an HLA haplotype of Table 4. The transfection was carried out using a commercially available kit (model "CRISPR-MAX," product number: CMAX00003, Thermo Fisher Scientific).

[Table 4]

| HLA haplotype of 1383D2 iPS cell | | | | | |
|---|---|---|---|---|---|
| | HLA-A | | HLA-B | | HLA-C | |
| HLA allele | A*02:07 | A*32:01 | B*15:02 | B*51:01 | C*08:01 | C*14:02 |

Then, after extracting genomic DNA, gene mutation introduction efficiency in each cell was examined by T7 endonuclease I (T7EI) assay. The T7EI assay was performed as follows.

First, Nested PCR was performed using a primer that specifically amplifies the A*02:07 allele of the HLA-A2 region. Primers shown in Table 5 were used for Nested PCR.

[Table 5]

| Primers for 1st PCR | | |
| --- | --- | --- |
| Primer name | Base sequence | SEQ ID NO. |
| 1383D2-HLA-A-be-exl-fwd | TTGGGGATTCCCCAACTCC | 41 |
| HLA-A-in7-rev | GTCCACTGTTCCGCCCAA | 42 |
| Primers for 2nd PCR | | |
| Primer name | Base sequence | SEQ ID NO. |
| HLA-A0207 -ex2-fwd | GGTCCGGAGTATTGGGACGG | 43 |
| HLA-A0207-ex3-rev | TCTCAACTGCTCCGCCACAT | 44 |

Then, a PCR product was purified, and 1/10 volume of 10 × NEBuffer2 (NEB) buffer was added to the purified PCR product (400 ng) and heated at 95°C for 5 minutes to heat-modify double-stranded DNA. Thereafter, re-annealing was performed by gradually lowering the temperature. More specifically, cooling was performed from 95°C to 85°C at -2°C/sec, and from 85°C to 25°C at -0.1°C/sec.

Then, 10 units of T7 endonuclease I (T7EI, Cat. No. M0302S, NEB) was added to the PCR product after reannealing and treated at 37°C for 15 minutes. Subsequently, activity of T7EI was stopped by adding a 0.25 M EDTA solution at 1/10 volume of the reaction solution, and then a sample was maintained at a low temperature (on ice).

Subsequently, the T7EI-treated PCR product was subjected to 2% agarose gel electrophoresis, and DNA signal intensities of a cleaved band and an uncut band were quantified with ImageJ software.

Fig. 5(b) shows the results of analysis of the genomic DNA mutation introduction efficiency in a case where each sgRNA was used by the T7EI assay. In Fig. 5(b), "T7EI" indicates T7 endonuclease I, "-" indicates that no addition was performed, and "+" indicates that addition was performed. Furthermore, "ex3-g1" indicates a result of introducing A0207-ex3-g1, "ex3-g2" indicates a result of introducing A0207-ex3-g2, and "ex3-g4" indicates a result of introducing A0207-ex3-g4, and "DMD#1" indicates a result of introducing an sgRNA specific to a dystrophin gene, which was used as a positive control. Furthermore, an arrowhead indicates a band cleaved by T7EI.

As a result, it was revealed that A0207-ex3-g4 sgRNA has the highest genome cleavage activity.

[Experimental Example 7]

(HLA-allele-specific knockout 1)

«Confirmation of HLA protein expression»

In order to confirm the expression of HLA on a cell surface, two iPS cell strains having HLA serotype A2 (1383D2 strain and 404C2 strain) were selected. Furthermore, a 604B1 strain was selected as an iPS cell strain not having the HLA serotype A2. Regarding undifferentiated iPS cells of a total of three strains, for in a case where the cells were treated with cytokine and a case where the cells were treated with 50 ng/mL of IFN-γ for 48 hours, the cells were stained using an anti-HLA-A2 antibody (product number: 740082, BD), and flow cytometric analysis was performed thereon.

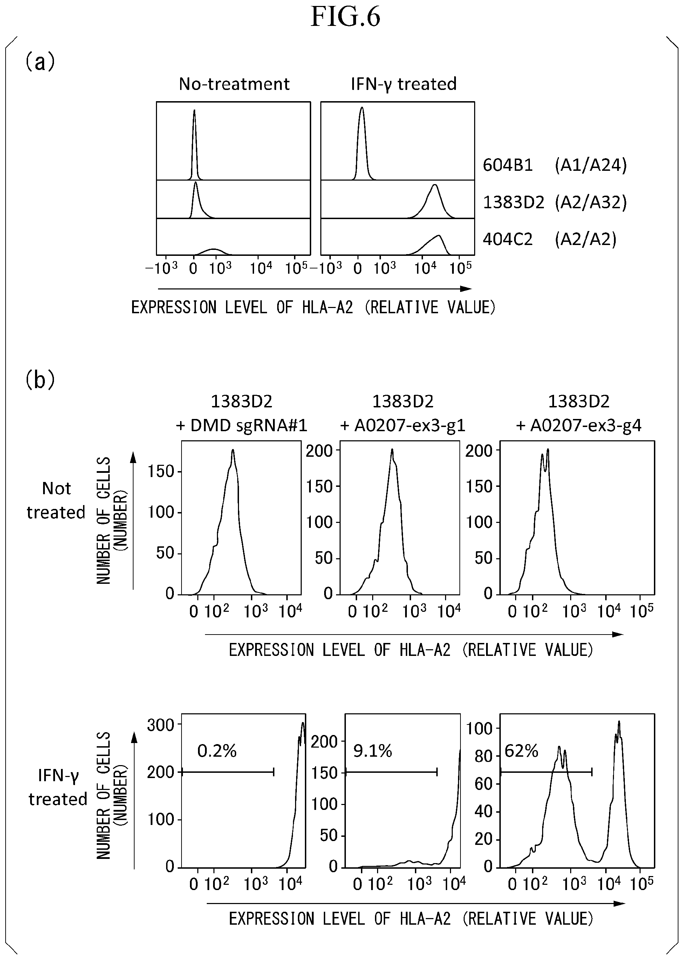

Fig. 6(a) is a graph showing results of flow cytometric analysis. As a result, by IFN-γ treatment, specific expression of HLA-A2 was confirmed in the 1383D2 cells and the 404C2 cells.

<<HLA-allele-specific knockout>>

Subsequently, A0207-ex3-g1 (where a target base sequence is shown in SEQ ID NO: 3), A0207-ex3-g2 (where a target base sequence is shown in SEQ ID NO: 4), and A0207-ex3-g4 (where a target base sequence is shown in SEQ ID NO: 7), which were the above-described sgRNAs, were respectively transfected into 1383D2 iPS cells together with a purified Cas9 protein. The transfection was carried out using a commercially available kit (model "CRISPR-MAX," product number: CMAX00003, Thermo Fisher Scientific).

In addition, as a control sgRNA that does not disrupt an HLA gene, sgRNA-DMD#1 (where a target base sequence is shown in SEQ ID NO: 61) targeting a dystrophin (DMD) gene on the X chromosome was used.

Subsequently, each transfected iPS cell was passaged in 2 wells, one well kept untreated with cytokines and the other well was treated with 50 ng/mL of IFN-γ added to a medium for 48 hours. Thereafter, flow cytometric analysis was performed on the cells using an anti-HLA-A2 antibody (product number: 740082, BD).

Fig. 6(b) is a graph showing results of flow cytometric analysis. As a result, it was revealed that, in a case where A0207-ex3-g4 sgRNA targeting the highest genome cleaving activity was used, the cells in which the largest number of an HLA-A2 antigen was disrupted appeared.

<<Analysis of base sequence of target site of sgRNA>>

Then, genomic DNA was extracted from 1383D2 iPS cells into which sgRNA (A0207-ex3-g4) had been introduced, and using a primer specifically amplifying the A*02:07 allele and not amplifying the A*32:01 allele, Nested PCR was performed to amplify a target site of sgRNA. Primers shown in Table 6 were used for Nested PCR.

[Table 6]

| Primers for 1st PCR | | |
|---|---|---|
| Primer name | Base sequence | SEQ ID NO. |
| 1383D2-HLA-A-be-exl-fwd | TTGGGGATTCCCCAACTCC | 41 |
| HLA-A-in7-rev | GTCCACTGTTCCGCCCAA | 42 |
| Primers for 2nd PCR | | |
| Primer name | Base sequence | SEQ ID NO. |
| HLA-A0207-ex2-fwd | GGTCCGGAGTATTGGGACGG | 43 |
| HLA-A0207-ex3-rev | TCTCAACTGCTCCGCCACAT | 44 |

Then, a PCR product was cloned by TA cloning into a pGEM-T-Easy vector (Promega). Subsequently, 21 strains of Escherichia coli colonies were recovered, and a base sequence was determined by Sanger sequence. As a result, it was revealed that 11 strains had a wild-type base sequence, but 10 strains (4 strains of deletion and 6 strains of insertion) had some insertion deletion mutation (Indel mutation).

In addition, 1383D2 iPS cells to which sgRNA (A0207-ex3-g4) was introduced were stained with an anti-HLA-A2 antibody (product number:740082, BD), and cells in which an HLA-A2 antigen became negative were sorted by a flow cytometer. Genomic DNA was extracted from the sorted iPS cells, and Nested PCR was performed using each of the primers (described above) that specifically amplify the A*02:07 allele which was the target allele. In addition, Nested PCR was performed using each of primers that specifically amplify the A*32:01 allele which was not the target. Primers shown in Table 7 were used for Nested PCR of the A*32:01 allele.

[Table 7]

| Primers for 1st PCR | | |
|---|---|---|
| Primer name | Base sequence | SEQ ID NO. |
| 1383D2-HLA-A-be-exl-fwd | TTGGGGATTCCCCAACTCC | 41 |
| HLA-A-in7-rev | GTCCACTGTTCCGCCCAA | 42 |
| Primers for 2nd PCR | | |
| Primer name | Base sequence | SEQ ID NO. |
| HLA-A3201-ex2-fwd | GGGCCGGAGTATTGGGACCA | 70 |
| HLA-A3201-ex3-rev | CTCAACTGCTCCGCCACAT | 71 |

Then, a PCR product was cloned by TA cloning into a pGEM-T-Easy vector (Promega). Escherichia coli colonies were recovered from 16 strains (A*02:07 allele) and 19 strains (A*32:01 allele), and base sequences were determined by Sanger sequence. Then, based on the determined base sequence, the number of clones with a deletion in the sequence, the number of clones with insertion, and the number of clones without sequence change were counted.

As a result, the A*02:07 allele had some insertion deletion mutation (Indel mutation) in all subclones of the 16 strains. On the other hand, the A*32:01 allele was confirmed to be a wild-type base sequence in all subclones of the 19 strains.

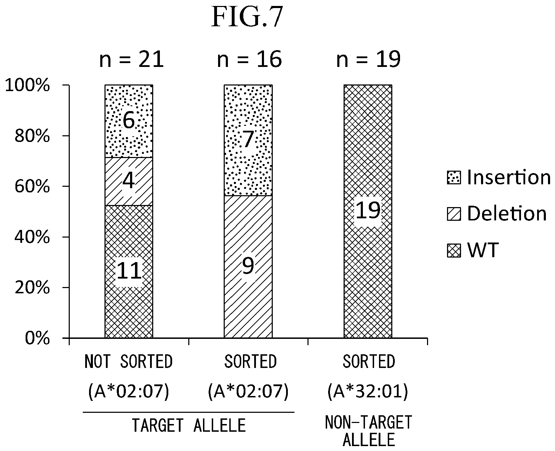

Fig. 7 shows a graph showing genomic DNA mutation introduction efficiency in a case where sorting by HLA-A2 antigen expression was not performed and in a case where sorting a cell group in which HLA-A2 antigen expression was negative was performed, after introducing sgRNA into the iPS cell 1383D2 strain to induce genome editing.

As a result, it was revealed that proportion of cells in which the A*02:07 target allele was genome-edited was increased by sorting HLA antigen-negative cells. In addition, it was also confirmed that no mutation was introduced into the HLA-A*32:01 allele, which is a non-target allele.

[Experimental Example 8]

(HLA-allele-specific knockout 2)

By disrupting the HLA allele of 604B1 iPS cells having the HLA haplotype shown in Table 8, low-antigenic cells were produced in which a rejection reaction was reduced in a case where the cells were allogeneically transplanted into a recipient A having a hypothetical HLA haplotype shown in Table 9. HLA haplotypes shown in Table 9 are HLA haplotypes of peripheral blood mononuclear cells (PBMC, type "LP_194," CTL).

As shown in Table 8 and Table 9, 604B1 iPS cells have A*01:01 and B*07:02 alleles. In addition, these HLA alleles are not present in the recipient A. Accordingly, in a case where the 604B1 iPS cells or cells differentiation-induced from the 604B1 iPS cells are transplanted into a recipient, T cells of the recipient A attack the cells and immune rejection occurs.

For this reason, in order to reduce a rejection reaction, the HLA alleles that are present in the donor cells but not present in the recipient A, that is, the A*01:01 allele and the B*07:02 allele, are disrupted such that cells do not express an HLA protein specific to donor cells.

[Table 8]

| HLA haplotype of 604B1 iPS cell | | | | | |
|---|---|---|---|---|---|
| HLA-A | | HLA-B | | HLA-C | |
| A*01:01 | A*24:02 | B*07:02 | B*37:01 | C*06:02 | C*07:02 |

[Table 9]

| HLA haplotype of virtual recipient A (PBMC "LP_194," CTL) | | | | | |
|---|---|---|---|---|---|
| HLA-A | | HLA-B | | HLA-C | |
| A*24:02 | A*24:02 | B*37:01 | B*39:06 | C*06:02 | C*07:02 |

From among the sgRNAs identified in Experimental Example 1, two sgRNAs (A0101-ex3-g1 (where a target base sequence is set forth in SEQ ID NO: 45) and A0101-ex3-g2 (where a target base sequence is set forth in SEQ ID NO: 46)) which cleave only the A*01:01 allele and of which base sequences do not match the A*24:02 allele; two sgRNAs (B0702-ex2-g1 (where a target base sequence is set forth in SEQ ID NO: 47) and B0702-ex2-g2 (where a target base sequence is set forth in SEQ ID NO: 48)) which cleave only the B*07:02 allele and of which sequences do not match the B*37:01 allele were selected.

Then, each of the above sgRNAs was synthesized by an in vitro transcription reaction, and each was transfected into 604B1 iPS cells together with a purified Cas9 protein. The transfection was carried out using a commercially available kit (model "CRISPR-MAX," product number: CMAX00003, Thermo Fisher Scientific).

When the A*01:01 allele or the B*07:02 allele was cleaved by the introduction of sgRNA and a gene mutation occurred in the process of repairing it, the A*01:01 allele or the B*07:02 allele was knocked out, and thereby cells in which an HLA -A1 protein or an HLA-B7 protein was deficient were obtained.

Subsequently, each iPS cell was stimulated with 50 ng/mL of IFN-$\gamma$ for 48 hours to induce the expression of HLA protein. Subsequently, each iPS cell was stained with an anti-HLA-AI antibody (type "ab33883," abcam) or an anti-HLA-B7 antibody (type "MCA986," Bio-Rad), and a percentage of HLA protein-negative cells was analyzed by flow cytometry.

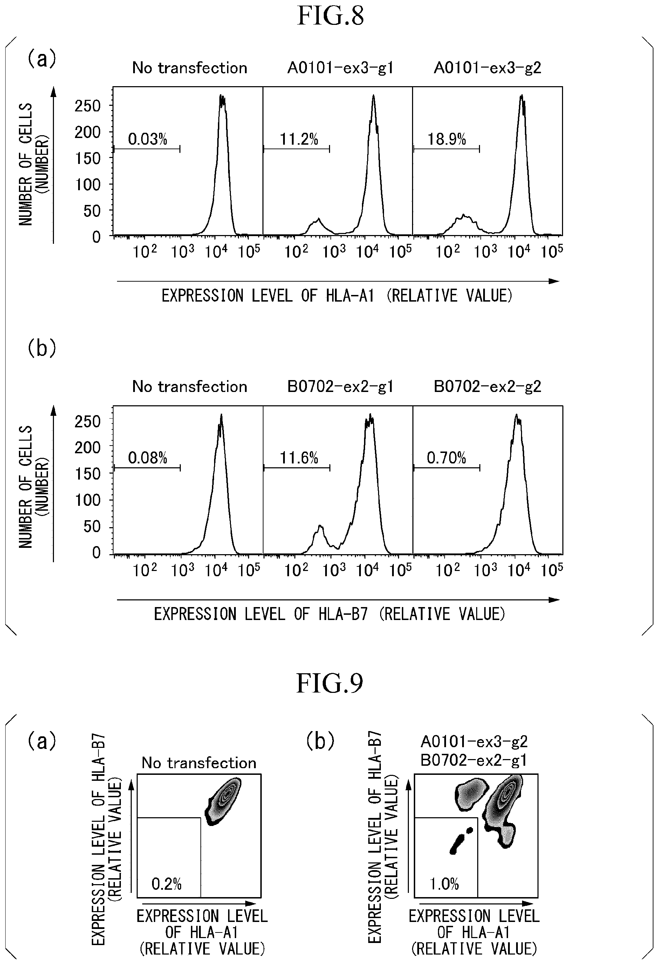

Fig. 8(a) and Fig. 8(b) are graphs showing results of flow cytometric analysis. Fig. 8(a) shows the results of measuring an expression level of the HLA-A1 protein in the iPS cells in which the A*01:01 allele was disrupted, and Fig. 8(b) shows the results of measuring an expression level of the HLA-B7 protein in the iPS cells in which the B*07:02 allele was disrupted. In Figs. 8(a) and 8(b), "No transfection" indicates the results of the iPS cells into which an sgRNA was not introduced.

As a result, it was revealed that 11.2% of cells were deficient in the HLA-A1 protein in a case where A0101-ex3-g1 (where a target base sequence is set forth in SEQ ID NO: 45) was used as sgRNA. In addition, it was revealed that 18.9% of the cells were deficient in the HLA-A1 protein in a case where A0101-ex3-g2 (where a target base sequence is set forth in SEQ ID NO: 46) was used as an sgRNA. Based on these results, it was revealed that A0101-ex3-g2 was an sgRNA targeting a higher knockout efficiency.

Furthermore, it was revealed that 11.6% of cells were deficient in the HLA-B7 protein in a case where B0702-ex2-g1 (where a target base sequence is set forth in SEQ ID NO: 47) was used as sgRNA. Furthermore, it was revealed that 0.7% of cells were deficient in the HLA-B7 protein in a case where B0702-ex2-g2 (where a target base sequence is set forth in SEQ ID NO: 48) was used as sgRNA. Based on these results, it was revealed that B0702-ex2-g1 was an sgRNA targeting a higher knockout efficiency.

Based on these results, A0101-ex3-g2 and B0702-ex2-g1 which had high knockout efficiency were selected, and both A*01:01 allele and B*07:02 allele were knocked out. Specifically, 604B1 iPS cells were transfected with the selected two kinds of sgRNA together with a purified Cas9 protein. The transfection was carried out using a commercially available kit (model "CRISPR-MAX," product number: CMAX00003, Thermo Fisher Scientific).

Subsequently, iPS cells were stimulated with 50 ng/mL of IFN-γ for 48 hours to induce the expression of HLA protein. Subsequently, iPS cells were stained with an anti-HLA-Al antibody (type "ab33883," abcam) and an anti-HLA-B7 antibody (type "MCA986," Bio-Rad), and analysis was performed by flow cytometry.

Fig. 9(a) and Fig. 9(b) are graphs showing results of flow cytometric analysis. In Fig. 9(a), "No transfection" indicates the analysis results of the iPS cells into which an sgRNA was not introduced. In addition, in Fig. 9(b), "A0101-ex3-g2" and "B0702-ex2-g1" indicate the analysis results of iPS cells into which these two sgRNAs were co-introduced and both the A*01:01 allele and the B*07:02 allele were knocked out.

In addition, single cell sorting was performed from a cell population in which both HLA-A1 protein and HLA-B7 protein were negative, and subclones of 15 strains were recovered. Subsequently, genomic DNA was extracted from each subclone. Then, a PCR reaction for amplifying a region containing a target site (exon 3) of A0101-ex3-g2, which is the sgRNA used and a region containing a target site (exon 2) of B0702-ex2-g1 was performed. Thereafter, a base sequence was analyzed by Sanger sequence.

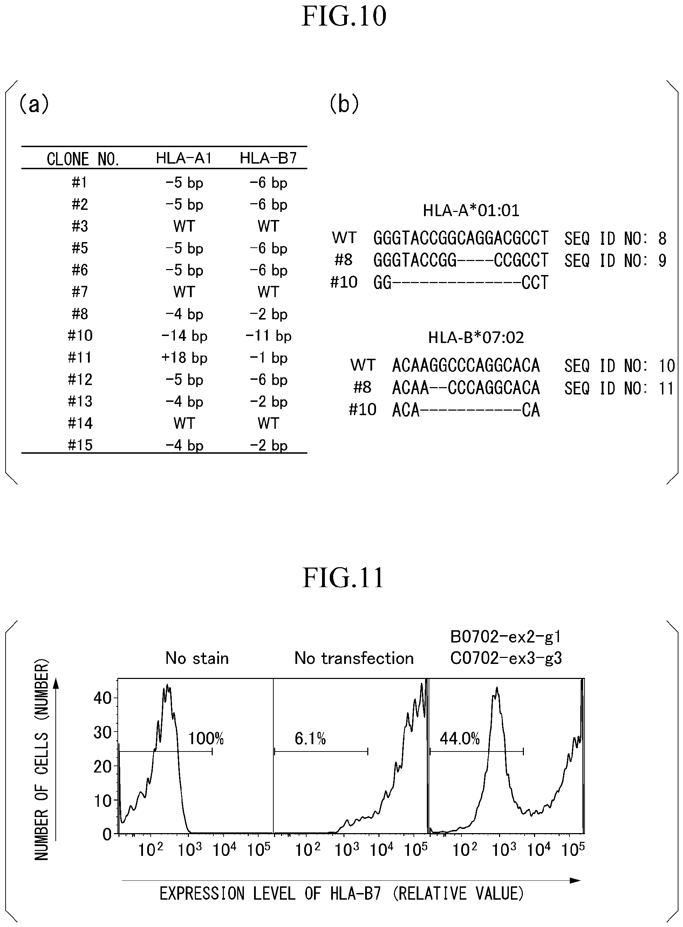

Fig. 10(a) is a table showing a base mutation pattern of each clone recovered. In Fig. 10(a), "WT" indicates that it was a wild-type base sequence, "-" indicates that it had a deletion mutation, and "+" indicates that it had an insertion mutation. For example, "-5 bp" indicates that it has a deletion of 5 bases, and "+18 bp" indicates that it has an insertion of 18 bases. In Fig. 10(b), base sequences of two strains (#8 and #10) are shown as a representative example.

As a result, it was revealed that some insertion deletion mutation (Indel mutation) occurred in both A*01:01 allele and B*07:02 allele in clones of 11 strains out of 15 strains. From the clones of 11 strains in which Indel mutation occurred, two strains in which frameshift mutation occurred (#8 and #10) were selected and used in the following experiments.

[Experimental Example 9]

(HLA-allele-specific knockout 3)

By disrupting the HLA allele of 585A1 iPS cells having the HLA haplotype shown in Table 10, low-antigenic cells were produced in which a rejection reaction was reduced in a case where the cells were allogeneically transplanted into a recipient B having a hypothetical HLA haplotype shown in Table 11. HLA haplotypes shown in Table 11 are HLA haplotypes of peripheral blood mononuclear cells (PBMC, type "2010113203," Wako).

As shown in Table 10 and Table 11, the 585A1 iPS cells had the B*07:02 allele and the C*07:02 allele. In addition, these HLA alleles are not present in the recipient B. Accordingly, in a case where the 585A1 iPS cells or cells differentiation-induced from the 585A1 iPS cells are transplanted into a recipient, T cells of the recipient B attack the cells and immune rejection occurs.

For this reason, in order to reduce a rejection reaction, the HLA alleles that are present in the donor cells but not present in the recipient B, that is, the B*07:02 allele and the C*07:02 allele, are disrupted such that cells do not express an HLA protein specific to donor cells.

[Table 10]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

[Table 11]

| HLA haplotype of virtual recipient B (PBMC "2010113203," Wako) | | | | | |
|---|---|---|---|---|---|
| HLA-A | | HLA-B | | HLA-C | |
| A*24:02 | A*24:02 | B*40:06 | B*52:01 | C*08:01 | C*12:02 |

As sgRNA, B0702-ex2-g1 (where a target base sequence is set forth in SEQ ID NO: 47) specific to the B*07:02 allele, and C0702-ex3-g3 (where a target base sequence is set forth in SEQ ID NO: 49) specific to the C*07:02 allele were used. In addition, both the B*07:02 allele and C*07:02 allele of the 585A1 iPS cells were knocked out in the same manner as in Experimental Example 7 except that a commercially available kit (trade name "4D-Nucleofector," model "V4XP-4032," Lonza) was used for transfection.

Subsequently, iPS cells were stimulated with 50 ng/mL of IFN-γ for 48 hours to induce the expression of HLA protein. Subsequently, iPS cells were stained with an anti-HLA-B7 antibody (type "MCA986," Bio-Rad), and analysis was performed by flow cytometry.

Fig. 11 is a graph showing results of flow cytometric analysis. In Fig. 11, "No stain" indicates the analysis results of iPS cells that were not stained with an antibody, "No transfection" indicates the analysis results of iPS cells into which sgRNA was not introduced, and "B0702-ex2-g1" and "C0702-ex3-g3" indicate the analysis results of iPS cells in which both B*07:02 allele and C*07:02 allele were knocked out by introducing these sgRNAs into the cells.

In addition, single cell sorting was performed from a cell population in which an HLA-B7 protein was negative, and subclones of 28 strains were recovered. Subsequently, genomic DNA was extracted from each subclone. Then, a PCR reaction for amplifying a region containing a target site (exon 2) of B0702-ex2-g1, which is the sgRNA used and a region containing a target site (exon 3) of C0702-ex3-g3 was performed. Thereafter, a base sequence was analyzed by Sanger sequence.

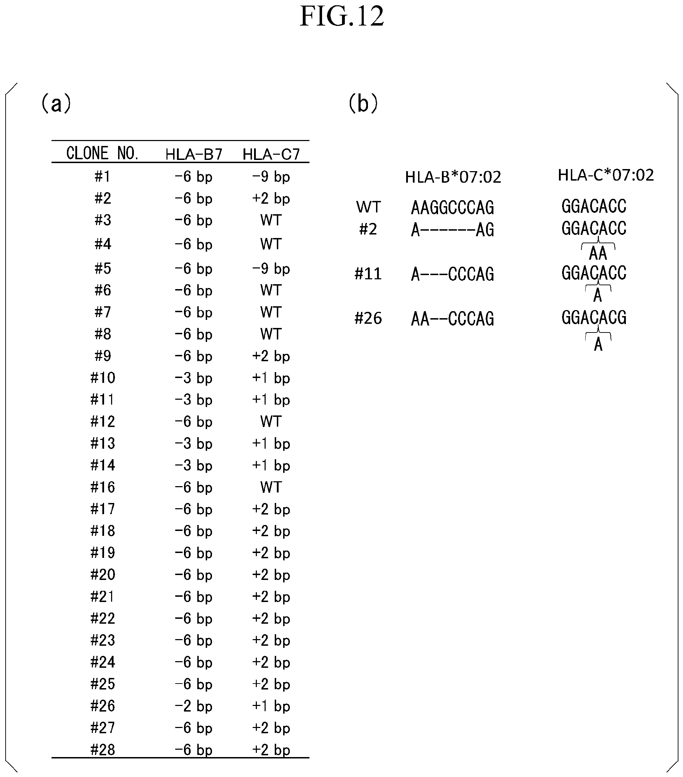

Fig. 12 is a table showing a base mutation pattern of each clone recovered. In Fig. 12(a), "WT" indicates that it was a wild-type base sequence, "-" indicates that it had a deletion mutation, and "+" indicates that it had an insertion mutation. In Fig. 12(b), base sequences of three strains (#2, #11, and #26) are shown as a representative example.

As a result, it was revealed that some insertion deletion mutation (Indel mutation) occurred in both B*07:02 allele and C*07:02 allele in multiple clones. Three strains (#2, #11, and #26) were selected from the clones in which the Indel mutation had occurred and used in the following experiment.

[Experimental Example 10]

(HLA-allele-specific knockout 4)

By disrupting the HLA allele of 585A1 iPS cells having the HLA haplotype shown in Table 12, low-antigenic cells were produced in which a rejection reaction was reduced in a case where the cells were allogeneically transplanted into a recipient C having a hypothetical HLA haplotype shown in Table 13. HLA haplotypes shown in Table 13 are HLA haplotypes of peripheral blood mononuclear cells (PBMC, type "LP_266," CTL).

As shown in Tables 12 and 13, the 585A1 iPS cells had the B*52:01 allele and the C*12:02 allele. In addition, these HLA alleles are not present in the recipient C. Accordingly, in a case where the 585A1 iPS cells or cells differentiation-induced from the 585A1 iPS cells are transplanted into the recipient C, T cells of the recipient C attack the cells and immune rejection occurs.

For this reason, in order to reduce a rejection reaction, the HLA alleles that are present in the donor cells but not present in the recipient C, that is, the B*52:01 allele and the C*12:02 allele, are disrupted such that cells do not express an HLA protein specific to donor cells.

[Table 12]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

[Table 13]

| HLA haplotype of virtual recipient C (PBMC "LP _266," CTL) | | | | | |
|---|---|---|---|---|---|
| HLA-A | | HLA-B | | HLA-C | |
| A*03:01 | A*24:02 | B*07:02 | B*40:02 | C*03:05 | C*07:02 |

The C*12:02 allele in the 585A1 iPS cells was knocked out in the same manner as in Experimental Example 9 except that C1202-ex3-g1 (where a target base sequence is set forth in SEQ ID NO: 50), which was specific to the C*12:02 allele, was used as sgRNA, and that cell sorting was not performed. Thereby, subclones of 17 strains were obtained. The cells were not sorted because the anti-HLA-C12 allele-specific antibody was not available.

Subsequently, genomic DNA was extracted from each subclone. Then, a PCR reaction for amplifying a region containing a target site (exon 3) of C1202-ex3-g1, which was sgRNA used, was performed. Thereafter, a base sequence was analyzed by Sanger sequence.

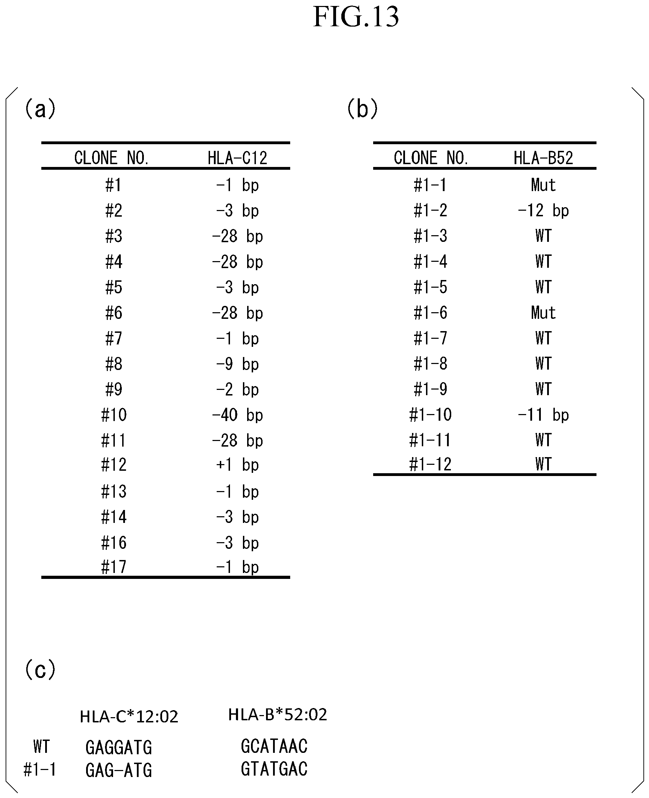

Fig. 13(a) is a table showing a base mutation pattern of each clone recovered. In Fig. 13(a), "-" indicates that it had a deletion mutation, and "+" indicates that it had an insertion mutation. As a result, it was revealed that some insertion deletion mutation (Indel mutation) occurred near the target site of sgRNA. From among the clones in which the Indel mutation had occurred, #1 cells having frameshift mutation were selected.

Then, B5201-ex3-g2 (where a target base sequence is set forth in SEQ ID NO: 51) specific to the B*52:01 allele was introduced, as sgRNA, into the #1 cells in which the C*12:02 allele was knocked out so that the B*52:01 allele was knocked out. Thereby, subclones of 12 strains (clones #1-1 to #1-12) were obtained. The cells were not sorted because the anti-HLA-B52 allele-specific antibody was not available.

Subsequently, genomic DNA was extracted from each subclone. Then, a PCR reaction for amplifying a region containing a target site (exon 3) of B5201-ex3-g2, which was sgRNA used, was performed. Thereafter, a base sequence was analyzed by Sanger sequence.

Fig. 13(b) is a table showing a base mutation pattern of each clone recovered. In Fig. 13(b), "Mut" indicates that it had a substitution mutation, "WT" indicates that it was a wild-type base sequence, and "-" had a deletion mutation. As a result, it was revealed that some mutation occurred near the target site of sgRNA. Fig. 13(c) shows a base sequence of a strain #1-1 as a representative example.

[Experimental Example 11]

(HLA-allele-specific knockout 5)

By disrupting the HLA allele of 604B1 iPS cells having the HLA haplotype shown in Table 14, low-antigenic cells were produced in which a rejection reaction was reduced in a case where the cells were allogeneically transplanted into a recipient C having a hypothetical HLA haplotype shown in Table 15. HLA haplotypes shown in Table 15 are HLA haplotypes of peripheral blood mononuclear cells (PBMC, type "LP _266," CTL).

As shown in Tables 14 and 15, 604B1 iPS cells had A*01:01 allele, B*37:01 allele, and C*06:02 allele. In addition, these HLA alleles are not present in the recipient C. Accordingly, in a case where the 604B1 iPS cells or cells differentiation-induced from the 604B1 iPS cells are transplanted into the recipient, T cells of the recipient attack the cells and immune rejection occurs.

For this reason, in order to reduce a rejection reaction, the HLA alleles that are present in the donor cells but not present in the recipient C, that is, the A*01:01 allele, the B*37:01 allele, and the C*06:02 allele, are disrupted such that cells do not express an HLA protein specific to donor cells.

[Table 14]

| HLA haplotype of 604B1 iPS cell | | | | | |
|---|---|---|---|---|---|
| HLA-A | | HLA-B | | HLA-C | |
| A*01:01 | A*24:02 | B*07:02 | B*37:01 | C*06:02 | C*07:02 |

[Table 15]

| HLA haplotype of virtual recipient C (PBMC "LP _266," CTL) | | | | | |
|---|---|---|---|---|---|
| HLA-A | | HLA-B | | HLA-C | |
| A*03:01 | A*24:02 | B*07:02 | B*40:02 | C*03:05 | C*07:02 |

A0101-ex3-g2 (where a target base sequence is set forth in SEQ ID NO: 46) which was specific to the *01:01 allele was used as sgRNA, and the A*01:01 allele of 604B1 iPS cells was knocked out in the same manner as in Experimental Example 9. Subsequently, C0602-ex3-g1 (where a target base sequence is set forth in SEQ ID NO: 52), which was an sgRNA and was specific to the C*06:02 allele, was introduced into the cells to knock out the C*06:02 allele, and subclones of nine strains were obtained.

Subsequently, genomic DNA was extracted from each subclone. Then, a PCR reaction for amplifying a region containing a target site (exon 3) of A0101-ex3-g2, which is the sgRNA used and a region containing a target site (exon 3) of C0602-ex3-gl was performed. Thereafter, a base sequence was analyzed by Sanger sequence.

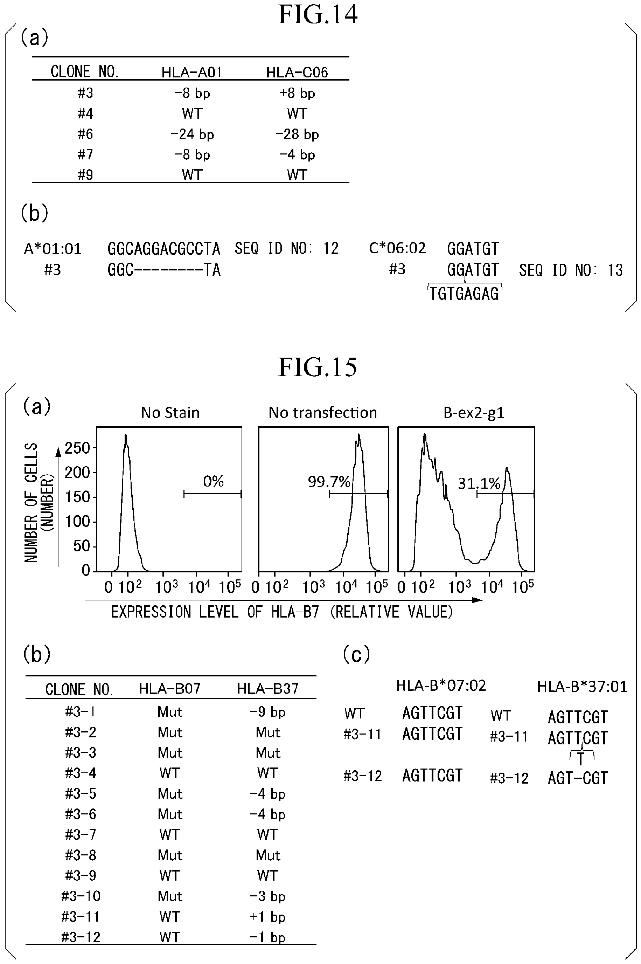

Fig. 14(a) is a table showing a base mutation pattern of each clone recovered. In Fig. 14(a), "WT" indicates that it was a wild-type base sequence, "-" indicates that it had a deletion mutation, and "+" indicates that it had an insertion mutation. As a result, a clone having some insertion deletion mutation (Indel mutation) near the target site of sgRNA was obtained. In Fig. 14(b), the base sequence is shown as a representative example of a clone #3 in which frameshift mutation had occurred among clones in which Indel mutation had occurred.

Subsequently, B-ex2-g1 (where a target base sequence is set forth in SEQ ID NO: 53) specific to the HLA-B allele was introduced, as sgRNA, into the clone #3 in which the A*01:01 allele and the C*06:02 allele were knocked out to test whether or not a clone in which only the B*37:01 allele was knocked out could be obtained. B-ex2-g1 is an sgRNA targeting both B*37:01 allele and B*07:02 allele.

Subsequently, iPS cells were stimulated with 50 ng/mL of IFN-γ for 48 hours to induce the expression of HLA protein. Subsequently, iPS cells were stained with an anti-HLA-B7 antibody (type "MCA986," Bio-Rad), and analysis was performed by flow cytometry.

Fig. 15(a) is a graph showing results of flow cytometric analysis. In Fig. 15(a), "No stain" indicates the analysis results of iPS cells that were not stained with an antibody, "No transfection" indicates the analysis results of iPS cells into which sgRNA was not introduced, and "B-ex2-g1" indicates the analysis results of iPS cells into which the sgRNA was introduced and in which the HLA-B allele was knocked out.

In addition, single cell sorting was performed from a cell population in which an HLA-B7 protein was positive, and subclones of 12 strains (clones #3-1 to #3-12) were recovered. Subsequently, genomic DNA was extracted from each subclone. Next, a PCR reaction was performed for respectively amplifying a target B*37:01 allele and a non-target B*07:02 allele of a region containing a target site (exon 2) of B-ex2-g1, which is the sgRNA used. Thereafter, a base sequence was analyzed by Sanger sequence.

Fig. 15(b) is a table showing a base mutation pattern of each clone recovered. In Fig. 13(b), "Mut" indicates that it had a substitution mutation, "WT" indicates that it was a wild-type base sequence, "-" had a deletion mutation, and "+" indicates that it had an insertion mutation. As a result, a clone having some insertion deletion mutation (Indel mutation) near the target site of sgRNA was obtained. In Fig. 15(c), base sequences of two strains (clones #3-11 and #3-12) are shown as representative examples among the clones in which the Indel mutation occurs only in the B*37:01 allele.

[Experimental Example 12]

(HLA-allele-specific knockout 6)

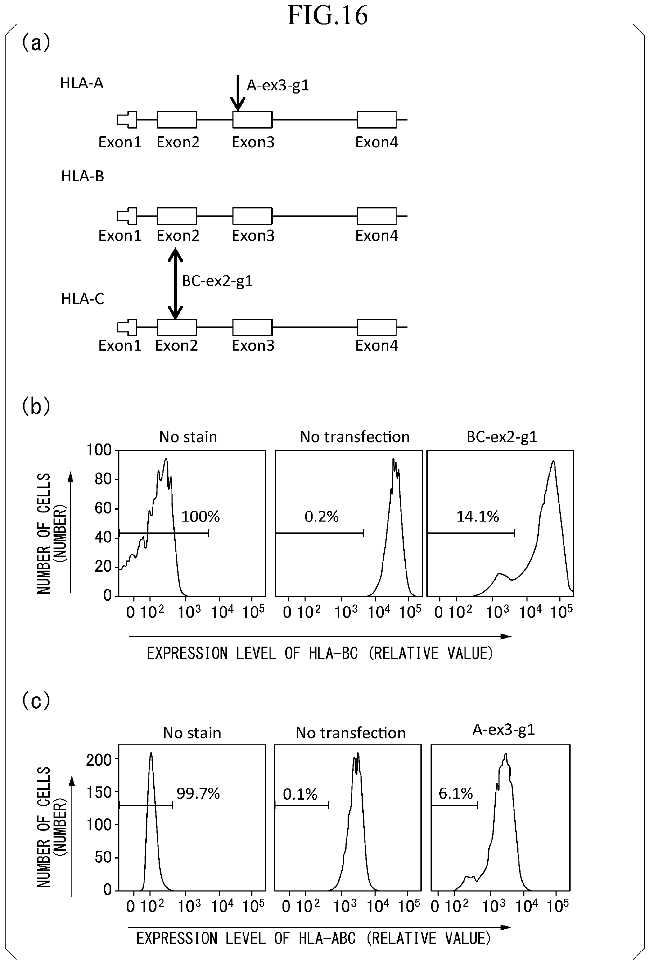

By disrupting an HLA allele of 604B1 iPS cells having an HLA haplotype shown in Table 16, cells in which all three genetic loci of an HLA-A allele, an HLA-B allele, and an HLA-C allele were knocked out were produced. Fig. 16(a) is a diagram showing a target site of an sgRNA used in the present experimental example.

[Table 16]

| HLA haplotype of 604B1 iPS cell | | | | | |
|---|---|---|---|---|---|
| HLA-A | | HLA-B | | HLA-C | |
| A*01:01 | A*24:02 | B*07:02 | B*37:01 | C*06:02 | C*07:02 |

The B*07:02 allele, B*37:01 allele, C*06:02 allele, and C*07:02 allele of 604B1 iPS cells were knocked out in the same manner as in Experimental Example 9 using, as sgRNA, BC-ex2-g1 (where a target base sequence is set forth in SEQ ID NO: 54) capable of targeting both HLA-B allele and HLA-C allele.

Then, iPS cells were stimulated with 50 ng/mL IFN-γ for 48 hours and subjected to flow cytometric analysis to examine the expression of HLA-B protein and HLA-C protein. Fig. 16(b) is a graph showing the results of flow cytometry. In Fig. 16(b), "No stain" indicates the analysis results of iPS cells that were not stained with an antibody, "No transfection" indicates the analysis results of iPS cells into which sgRNA was not introduced, and "BC-ex2-g1" indicates the analysis results of iPS cells into which the sgRNA was introduced and in which both the HLA-B allele and the HLA-C allele were knocked out.

Subsequently, a cell population in which both the HLA-B protein and the HLA-C protein was negative was sorted and recovered. Subsequently, A-ex3-g1 (where a target base sequence is set forth in SEQ ID NO: 55) capable of targeting both alleles of an HLA-A gene was introduced into the recovered cell population, and the A*01:01 allele and the A*24:02 allele were knocked out.

Then, iPS cells were stimulated with 50 ng/mL IFN-γ for 48 hours and subjected to flow cytometric analysis to examine the expression of HLA-A protein, HLA-B protein, and HLA-C protein. Fig. 16(c) is a graph showing the results of flow cytometry. In Fig. 16(c), "No stain" indicates the analysis results of iPS cells that were not stained with an antibody, "No transfection" indicates the analysis results of iPS cells into which sgRNA was not introduced, and "A-ex3-g1" indicates the analysis results of iPS cells into which the sgRNA was introduced and in which the HLA-A allele was knocked out.

Subsequently, single cell sorting of a cell population in which HLA-A protein, HLA-B protein, and HLA-C protein were all negative was performed to recover subclones of 8 strains. Subsequently, genomic DNA was extracted from each subclone. Then, a PCR reaction for respectively amplifying a region containing a target site which was sgRNA used, was performed. Thereafter, a base sequence was analyzed by Sanger sequence.

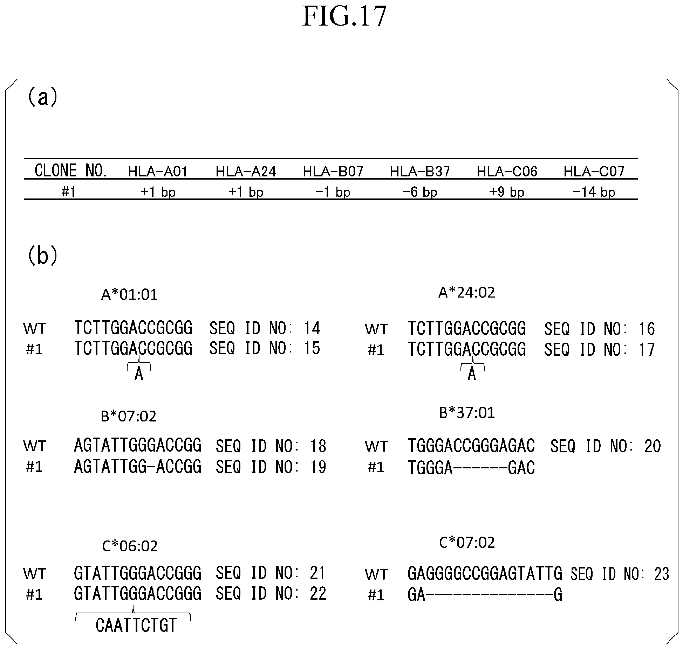

Fig. 17(a) is a table showing a base mutation pattern of one of the recovered clones (clone #1). In Fig. 17(a), "-" indicates that it had a deletion mutation, and "+" indicates that it had an insertion mutation. As a result, it was confirmed that a clone could be obtained, in which an insertion deletion mutation (Indel mutation) had occurred in all of the A*01:01 allele, A*24:02 allele, B*07:02 allele, B*37:01 allele, C*06:02 allele, and C*07:02 allele. Fig. 17(b) shows a base sequence of each HLA allele of the clone #1. In Fig. 17(b), "WT" indicates a wild-type base sequence.

[Experimental Example 13]

(HLA-allele-specific knockout 7)

By disrupting an HLA allele of 585A1 iPS cells having an HLA haplotype shown in Table 17, cells in which the HLA-A allele and the HLA-B allele were knocked out and only one of the HLA-C allele was knocked out were produced.

[Table 17]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

The A*24:02 allele, B*07:02 allele, and B*52:01 allele of 585A1 iPS cells were knocked out in the same manner as in Experimental Example 9 using, as sgRNA, A-ex3-g1 capable of targeting the HLA-A allele (where a target base sequence is set forth in SEQ ID NO: 55) and B-ex2-g1 capable of targeting the HLA-B allele (where a target base sequence is set forth in SEQ ID NO: 53).

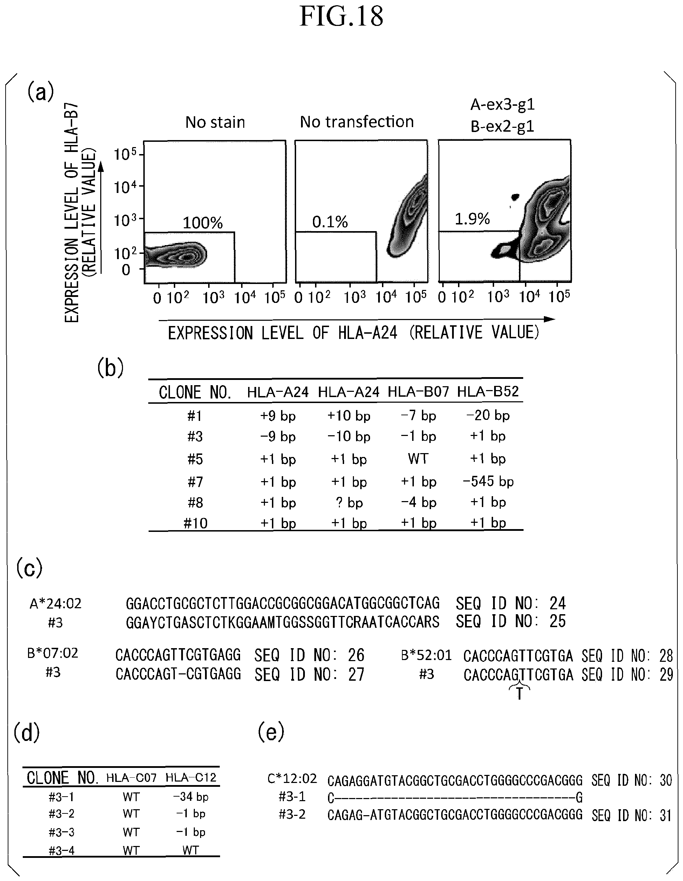

Then, iPS cells were stimulated with 50 ng/mL IFN-γ for 48 hours and subjected to flow cytometric analysis to examine the expression of HLA-A24 antigen and HLA-B7 antigen. Fig. 18(a) is a graph showing results of flow cytometry.

Subsequently, single cell sorting was performed on a cell population in which both HLA-A24 antigen and HLA-B7 antigen were negative, and subclones of 10 strains were recovered. Subsequently, genomic DNA was extracted from each subclone. Then, a PCR reaction for respectively amplifying a region containing a target site which was sgRNA used, was performed. Thereafter, a base sequence was analyzed by Sanger sequence.

Fig. 18(b) is a table showing a base mutation pattern of each clone recovered. In Fig. 18(b), "WT" indicates that it was a wild-type base sequence, "-" had a deletion mutation, "+" indicates that it had an insertion mutation, and "?" indicates that it could not be determined. As a result, a clone #3 was selected in which it was revealed that insertion deletion mutation (Indel mutations) had occurred in all of the A*24:02 allele, B*07:02 allele, and B*52:01 allele. Fig. 18(c) shows a base sequence of the clone #3.

Subsequently, C1202-ex3-g1 (where a target base sequence is set forth in SEQ ID NO: 50) specific to the C*12:02 allele was introduced into the clone #3, and the C*12:02 allele was knocked out. Subsequently, subclones of four strains (clones #3-1 to #3-4) were recovered. Subsequently, genomic DNA was extracted from each subclone. Subsequently, a PCR reaction for respectively amplifying the C*07:02 allele and the C*12:02 allele was performed. Thereafter, the base sequence was analyzed by Sanger sequence.

Fig. 18(d) is a table showing a base mutation pattern of each clone recovered. In Fig. 18(d), "WT" indicates that it was a wild-type base sequence, and "-" indicates that it had a deletion mutation. As a result, it was confirmed that the C*07:02 allele remained and clones of three strains (clones #3-1, #3-2, and #3-3) in which an insertion deletion mutation (Indel mutation) had occurred in the C*12:02 allele were obtained. Fig. 18(e) shows a base sequence of a clone #3-1 as a representative example.

[Experimental Example 14]

(HLA-allele-specific knockout 8)

By disrupting an HLA allele of 604B1 iPS cells having an HLA haplotype shown in Table 18, cells in which the HLA-A allele and HLA-B allele were knocked out were produced.

[Table 18]

| HLA haplotype of 604B1 iPS cell | | | | | |
|---|---|---|---|---|---|
| HLA-A | | HLA-B | | HLA-C | |
| A*01:01 | A*24:02 | B*07:02 | B*37:01 | C*06:02 | C*07:02 |

The A*01:01 allele, A*24:02 allele, B*07:02 allele, and B*37:01 allele of 604B1 iPS cells were knocked out in the same manner as in Experimental Example 9 using, as sgRNA, A-ex3-g1 capable of targeting the HLA-A allele (where a target base sequence is set forth in SEQ ID NO: 55) and B-ex2-g1 capable of targeting the HLA-B allele (where a target base sequence is set forth in SEQ ID NO: 53).

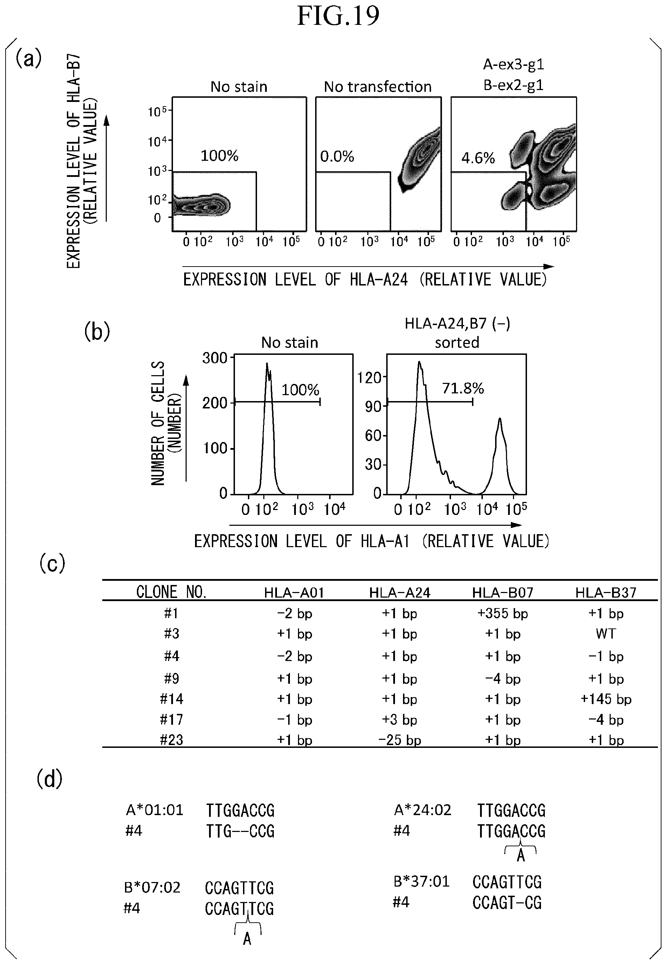

Then, iPS cells were stimulated with 50 ng/mL IFN-$\gamma$ for 48 hours and subjected to flow cytometric analysis to examine the expression of HLA-A24 antigen and HLA-B7 antigen. Fig. 19(a) is a graph showing results of flow cytometry. In Fig. 19(a), "No stain" indicates the analysis results of iPS cells that were not stained with an antibody, "No transfection" indicates the analysis results of iPS cells into which sgRNA was not introduced, and "A-ex2-g1" and "B-ex2-g1" indicate the analysis results of iPS cells into which these sgRNAs were introduced and in which both the HLA-A allele and the HLA-B allele were knocked out.

Subsequently, a cell population in which both HLA-A24 antigen and HLA-B7 antigen were negative was sorted and recovered, and furthermore, the expression of HLA-A1 antigen was examined. Fig. 19(b) is a graph showing the results of flow cytometry. In Fig. 19(b), "No stain" indicates the analysis results of the iPS cells not stained with an antibody, and "HLA-A24, B7 (-) sorted" indicates the analysis results of a cell population in which both HLA-A24 antigen and HLA-B7 antigen were negative.

Subsequently, a cell population in which HLA-A1 antigen was negative was sorted and recovered, and subclones of 23 strains were recovered. Subsequently, genomic DNA was extracted from each subclone. Then, a PCR reaction for respectively amplifying a region containing a target site which was sgRNA used, was performed. Thereafter, a base sequence of seven strains was analyzed by Sanger sequence.

Fig. 19(c) is a table showing a base mutation pattern of each clone recovered. In Fig. 19(c), "WT" indicates that it was a wild-type base sequence, "-" indicates that it had a deletion mutation, and "+" indicates that it had an insertion mutation. As a result, it became clear that clones of six strains could be obtained in which all of the A*01:01 allele, A*24:02 allele, B*07:02 allele, and B*37:01 allele had an insertion deletion mutation (Indel mutation). A base sequence of a clone #4 is shown in Fig. 19(d) as a representative example.

[Experimental Example 15]

(HLA-allele-specific knockout 9)

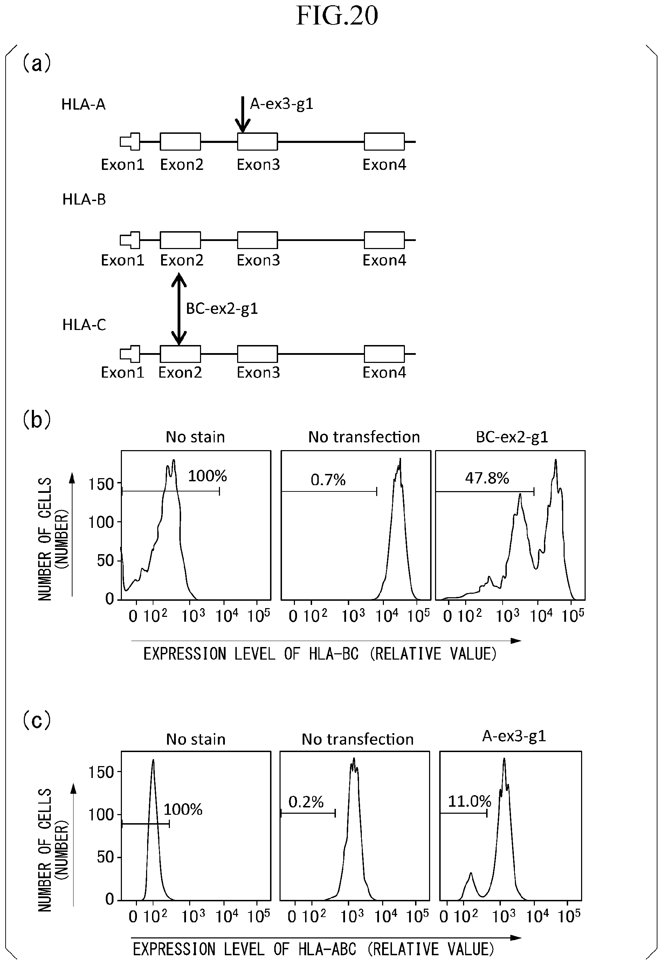

By disrupting an HLA allele of 1383D2 iPS cells having an HLA haplotype shown in Table 19, cells in which all three genetic loci of an HLA-A allele, an HLA-B allele, and an HLA-C allele were knocked out were produced. Fig. 20(a) is a diagram showing a target site of an sgRNA used in the present experimental example.

[Table 19]

| HLA haplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

The B*15:02 allele, B*51:01 allele, C*08:01 allele, and C*14:02 allele of 1383D2 iPS cells were knocked out in the same manner as in Experimental Example 9 using, as sgRNA, BC-ex2-g1 (where a target base sequence is set forth in SEQ ID NO: 54) capable of targeting both HLA-B allele and HLA-C allele.

Then, iPS cells were stimulated with 50 ng/mL IFN-γ for 48 hours and subjected to flow cytometric analysis to examine the expression of HLA-B protein and HLA-C protein. Fig. 20(b) is a graph showing the results of flow cytometry. In Fig. 20(b), "No stain" indicates the analysis results of iPS cells that were not stained with an antibody, "No transfection" indicates the analysis results of iPS cells into which sgRNA was not introduced, and "BC-ex2-g1" indicates the analysis results of iPS cells into which the sgRNA was introduced and in which both the HLA-B allele and the HLA-C allele were knocked out.

Subsequently, a cell population in which both the HLA-B protein and the HLA-C protein was negative was sorted and recovered. Subsequently, A-ex3-g1 (where a target base sequence is set forth in SEQ ID NO: 55) capable of targeting both alleles of an HLA-A gene was introduced into the recovered cell population, and the A*02:07 allele and the A*32:01 allele were knocked out.

Then, iPS cells were stimulated with 50 ng/mL IFN-γ for 48 hours and subjected to flow cytometric analysis to examine the expression of HLA-A protein, HLA-B protein, and HLA-C protein.

Fig. 20(c) is a graph showing the results of flow cytometry. In Fig. 20(c), "No stain" indicates the analysis results of iPS cells that were not stained with an antibody, "No transfection" indicates the analysis results of iPS cells into which sgRNA was not introduced, and "A-ex3-g1" indicates the analysis results of iPS cells into which the sgRNA was introduced and in which the HLA-A allele was knocked out. As a result, the presence of a cell population in which all of HLA-A protein, HLA-B protein, and HLA-C protein were negative was confirmed.

[Experimental Example 16]

(Modification of HLA allele)

An examination was conducted to modify the A*02:07 allele of 1383D2 iPS cells having an HLA haplotype shown in Table 20 into the A*01:01 allele by genome editing.

[Table 20]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

First, a double-regulated Cas9 expression vector and an sgRNA expression vector, both of which are piggyBac transposon vectors, were introduced into 1383D2 iPS cells. Drug selection was performed for 5 days using Puromycin (Cat. No. 29455-54, NACALAI TESQUE, INC.) at a final concentration of 1 to 15 μg/mL or hygromycin B (Cat. No. 10687-010, Thermo Fisher Scientific) at a final concentration of 100 to 200 μg/mL. Thereby, a cell population into which the chromosome was stably introduced was obtained.

The double-regulated Cas9 expression vector is a Cas9 expression vector capable of controlling both expression induction and intracellular localization (Ishida et al., Site-specific randomization of the endogenous genome by a regulatable CRISPR-Cas9 piggyBac system in human cells, Sci. Rep., 8: 310, 2018). In the present experimental example, a double-regulated Cas9 expression vector capable of inducing expression of a fusion protein of Cas9 protein and glucocorticoid receptor (GR) (hereinafter referred to as a "Cas9-GR protein") by adding doxycycline (Dox) to a medium of cells, and capable of translocating Cas9-GR into the nucleus by adding dexamethasone (Dex) to the medium of cells, was used. In addition, as the sgRNA, A0207-ex3-g1 (where a target base sequence is set forth in SEQ ID NO: 3) was used.

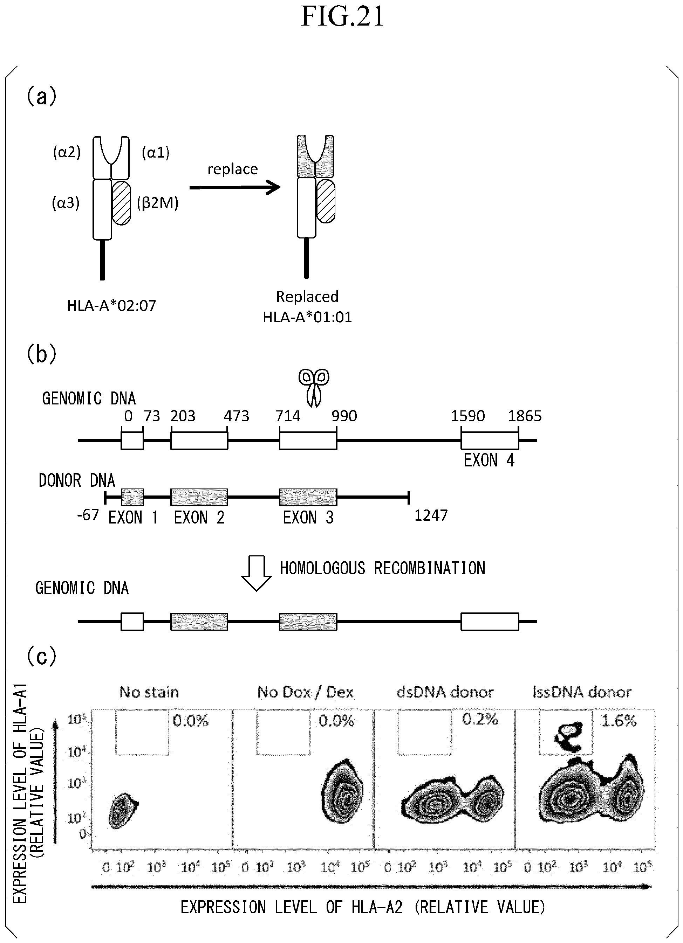

Fig. 21(a) and Fig. 21(b) are schematic diagrams illustrating homologous recombination in the present experimental example. Then, in order to induce homologous recombination, single-stranded DNA (where a target base sequence is set forth in SEQ ID NO: 56) encoding a region containing exons 1 to 3 of the A*01:01 allele was produced.

Specifically, first, using genomic DNA of 604B1 iPS cells as a template, a portion from 67 bases upstream of exon 1 to 1247 bases downstream of exon 3 of the A*01:01 allele was amplified by PCR using a primer shown in Table 21, and integrated into a pENTR plasmid to produce a pENTR vector.

[Table 21]

| Primer name | Base sequence | SEQ ID NO. |
|---|---|---|
| 604B1-HLA-A-be-ex1+15bp-fwd | ccaattcagtcgacgGTCGCGGTCGCTGTTCTAA | 57 |
| 604B1-HLA-A-in3+15bp-rev | gtctagatatctcgaTCATCAGTATTCGAGGGATCGTCT | 58 |

Subsequently, this plasmid DNA was treated with nicking endonuclease Nb. BsrDI enzyme (Model number "R0648S," New England Biolabs), agarose electrophoresis was performed under formamide denaturing conditions, the corresponding DNA band was cut out and purified, and thereby a desired single-stranded DNA donor (lssDNA donor, SEQ ID NO: 56) was prepared.

Subsequently, a PENTR vector (dsDNA donor) in a double-stranded DNA state and prepared single-stranded DNA (lssDNA donor, SEQ ID NO: 56) were respectively introduced into 1383D2 iPS cells, Dox and Dex were added at final concentrations of 2 $\mu$M and 1 $\mu$M, respectively, and homologous recombination was induced by genome editing.

Then, respective iPS cells were stimulated with 50 ng/mL IFN-$\gamma$ for 48 hours and subjected to flow cytometric analysis to examine the expression of HLA-A2 antigen and HLA-A1 antigen.

Fig. 21(c) is a graph showing the results of flow cytometry. In Fig. 20(c), "No stain" indicates the analysis results of iPS cells that were not stained with an antibody, "No Dox/Dex" indicates the analysis results of iPS cells that did not induce Cas9 expression, "dsDNA donor" indicates the analysis results of iPS cells in which a double-stranded DNA donor was introduced to induce homologous recombination, and "lssDNA donor" indicates the analysis results of iPS cells in which a single-stranded DNA donor was introduced to induce homologous recombination.

As a result, a ratio of negative A2 antigen and positive A1 antigen was 0.2% in a case where double-stranded DNA was used as a donor, and was 1.6% in a case where single-stranded DNA was used as a donor.

Subsequently, a cell population in which the A2 antigen became negative and the A1 antigen became positive by homologous recombination using a single-stranded DNA donor was sorted by flow cytometry, and a base sequence of the HLA-A genetic locus was analyzed for the cell population and subclones after single cell sorting.

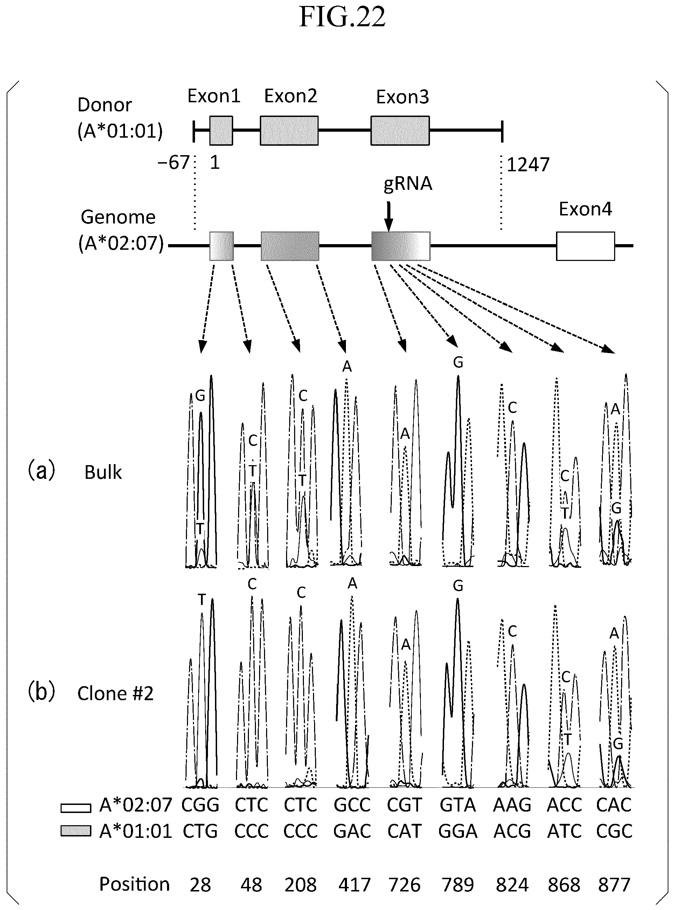

Fig. 22(a) is a diagram showing the results of analyzing a base sequence of the cell population as it is. In Fig. 22(a), "Bulk" indicates the results obtained by analyzing the cell population as it is. As a result, it was revealed that cells in which part of the A*02:07 genetic locus (particularly a 5' side of exon 2 and exon 3) was modified to an A*01:01 gene were present in combination.

In addition, Fig. 22(b) is a diagram showing the results of analyzing a base sequence of a typical single cell-sorted subclone (clone #2). As a result, it was revealed that part of the A*02:07 genetic locus was modified to the A*01:01 gene.

[Experimental Example 17]

(Production 1 of B2M knockout iPS cell)

1383D2 iPS cells were transfected with a plasmid vector expressing Cas9 and B2M-specific sgRNA. As sgRNA, B2M-g1 (where a target base sequence is set forth in SEQ ID NO: 59) and B2M-g2 (where a target base sequence is set forth in SEQ ID NO: 60) were used.

Subsequently, cells stimulated with 50 ng/mL IFN-$\gamma$ for 48 hours were stained with an anti-HLA-ABC antibody, and flow cytometric analysis was performed.

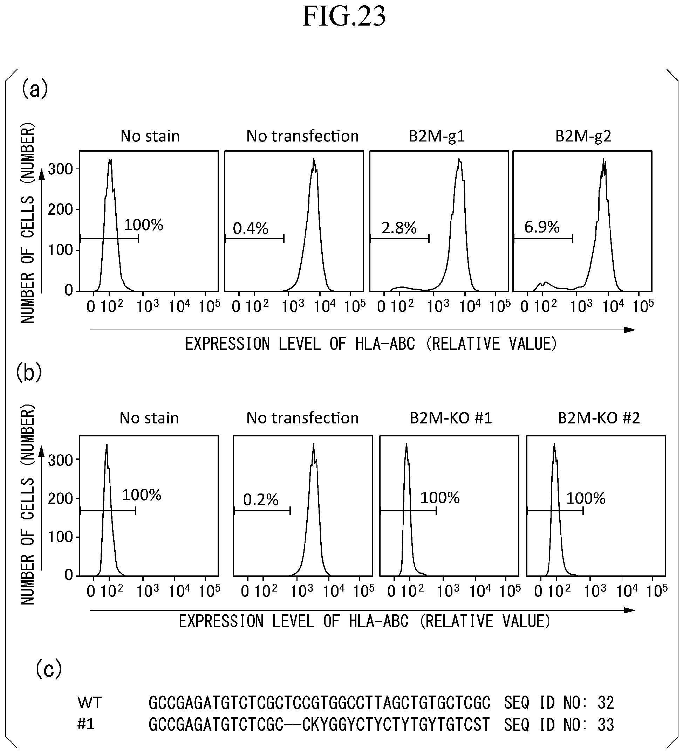

Fig. 23(a) is a graph showing results of flow cytometry. In Fig. 23(a), "No stain" indicates the analysis results of iPS cells that were not stained with an antibody, "No transfection" indicates the analysis results of iPS cells into which sgRNA was not introduced, and "B2M-g1" and "B2M-g2" indicate the analysis results of iPS cells into which the sgRNA was introduced and in which the B2M allele was knocked out.

As a result, a proportion of the cell population in which all of HLA-A protein, HLA-B protein, and HLA-C protein were negative was 2.8% in a case where B2M-g1 (where a target base sequence is set forth in SEQ ID NO: 59) was used as sgRNA. Furthermore, a proportion thereof was 6.9% in a case where B2M-g2 (where a target base sequence is set forth in SEQ ID NO: 60) was used as sgRNA.

Subsequently, single cell sorting was performed from a cell population in which HLA-A, HLA-B, and HLA-C were all negative, which were obtained by the introduction of B2M-g2 sgRNA. Thereby, subclones of two strains of clones #1 and #2 were obtained.

The obtained subclones of two strains were stimulated again with IFN-$\gamma$ for 48 hours, and flow cytometric analysis was performed. Fig. 23(b) is a graph showing the results of flow cytometry. In Fig. 23(b), "B2M-KO #1" indicates the results of the clone #1, and "B2M-KO #2" indicates the results of the clone #2. As a result, it was confirmed that HLA-A, HLA-B, and HLA-C were all negative in all the clones.

Subsequently, the B2M gene of the clone #1 was amplified by PCR to check a base sequence, and it was confirmed that an insertion deletion mutation (Indel mutation) had been introduced. Fig. 23(c) shows a base sequence of the B2M allele of the clone #1. In Fig. 23(c), "WT" indicates a wild-type base sequence.

[Experimental Example 18]

(Production 2 of B2M knockout iPS cell)

604B1 iPS cells were subjected to the same operation as in Experimental Example 17 to knock out the B2M allele. B2M-g2 (where a target base sequence is set forth in SEQ ID NO: 60) was used as sgRNA.

Subsequently, cells stimulated with 50 ng/mL IFN-γ for 48 hours were stained with an anti-HLA-ABC antibody, and flow cytometric analysis was performed.

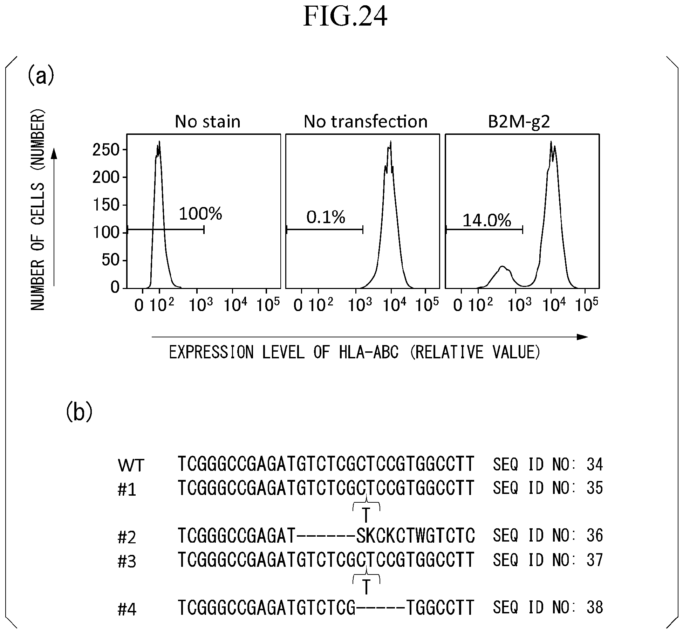

Fig. 24(a) is a graph showing results of flow cytometry. In Fig. 24(a), "No stain" indicates the analysis results of iPS cells that were not stained with an antibody, "No transfection" indicates the analysis results of iPS cells into which sgRNA was not introduced, and "B2M-g2" indicates that these sgRNAs were introduced to knock out the B2M allele. As a result, it became clear that a cell population in which all of HLA-A protein, HLA-B protein, and HLA-C protein were negative could be obtained.

Subsequently, single cell sorting was performed from a cell population in which HLA-A, HLA-B, and HLA-C were all negative. Thereby, subclones of four strains of clones #1, #2, #3, and #4 were obtained.

Then, the B2M gene of these subclones was amplified by PCR to check a base sequence. Fig. 24(b) is a diagram showing results of analysis of a base sequence. As a result, it was confirmed that an insertion deletion mutation (Indel mutation) was introduced into all the subclones.

[Experimental Example 19]

(Test 1 of T cell activation)

(1) 604B1 iPS cells having an HLA haplotype shown in Table 22, (2) a clone #8 which was produced in Experimental Example 8 and in which the A*01:01 and B*07:02 alleles of the 604B1 iPS cells had been knocked out, (3) same clone #10, (4) 1383D2 iPS cells having an HLA haplotype in Table 23, and (5) 604B1 iPS cells (clone #1) which were produced in Experimental Example 18 and in which the B2M allele had been knocked out were respectively differentiation-induced into blood-cell-like cells derived from embryoid body (EB).

[Table 22]

| HLA haplotype of 604B1 iPS cell | | | | | |
|---|---|---|---|---|---|
| HLA-A | | HLA-B | | HLA-C | |
| A*01:01 | A*24:02 | B*07:02 | B*37:01 | C*06:02 | C*07:02 |

[Table 23]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

Subsequently, CD8-positive T cells recovered by sorting from peripheral blood mononuclear cells having an HLA haplotype shown in Table 24 (PBMC, type "LP_194," CTL, corresponding to the above-mentioned recipient A) were stained with CFSE (Cayman Chemical Co., Ltd.). The cells were co-cultured with EB-derived blood-cell-like cells differentiated from the respective cells of (1) to (5) in the presence of IL-2 and IFN-γ for 8 days.

[Table 24]

| HLA haplotype of PBMC (LP_194, CTL) | | | | | |
|---|---|---|---|---|---|
| HLA-A | | HLA-B | | HLA-C | |
| A*24:02 | A*24:02 | B*37:01 | B*39:06 | C*06:02 | C*07:02 |

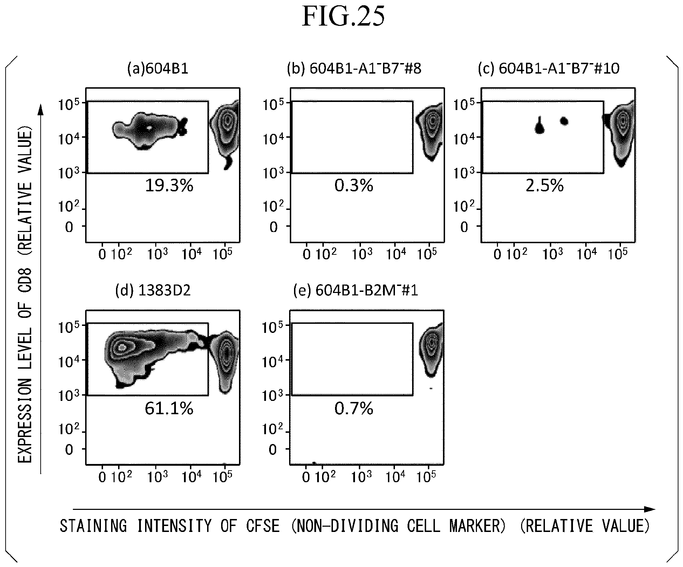

After co-culture, flow cytometric analysis was performed to examine fluorescence of CFSE. Figs. 25(a) to 25(e) are graphs showing results of flow cytometry. Figs. 25(a) to 25(e) show the results of CD8-positive T cells co-cultured with EB-derived blood-cell-like cells differentiated from the respective cells of (1) to (5). As a result, it was confirmed that only a T cell population that specifically recognized an HLA antigen of each of the iPS cells proliferated, and CFSE became negative.

[Experimental Example 20]

(Test 2 of T cell activation)

(1) 585A1 iPS cells having an HLA haplotype shown in Table 25, (2) a clone #2 which was produced in Experimental Example 9 and in which the B*07:02 and C*07:02 alleles of the 585A1 iPS cells had been knocked out, (3) same clone #11, (4) same clone #26, (5) 1383D2 iPS cells having an HLA haplotype in Table 26, and (6) 604B1 iPS cells (clone # 1) which were produced in Experimental Example 18 and in which the B2M had been knocked out were respectively differentiation-induced into EB-derived blood-cell-like cells.

[Table 25]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

[Table 26]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

Subsequently, CD8-positive T cells recovered by sorting from peripheral blood mononuclear cells having an HLA haplotype shown in Table 27 (PBMC, type "2010113203," Wako, corresponding to the above-mentioned recipient B) were stained with CFSE (Cayman Chemical Co., Ltd.). The cells were co-cultured with EB-derived blood-cell-like cells differentiated from the respective cells of (1) to (6) in the presence of IL-2 and IFN-γ for 8 days.

[Table 27]

| HLA haplotype of PBMC ("2010113203," Wako) | | | | | |
|---|---|---|---|---|---|
| HLA-A | | HLA-B | | HLA-C | |
| A*24:02 | A*24:02 | B*40:06 | B*52:01 | C*08:01 | C*12:02 |

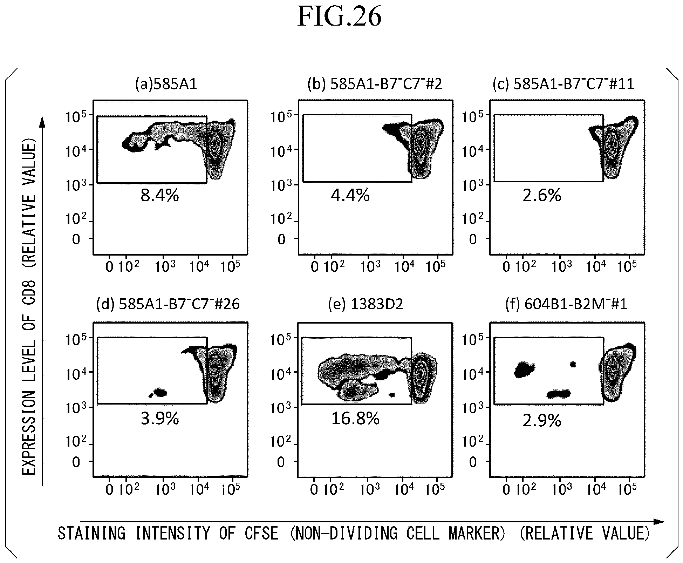

After co-culture, flow cytometric analysis was performed to examine fluorescence of CFSE. Figs. 26(a) to 26(f) are graphs showing results of flow cytometry. Figs. 26(a) to 26(f) show the results of CD8-positive T cells co-cultured with EB-derived blood-cell-like cells differentiated from the respective cells of (1) to (6). As a result, it was confirmed that a T cell population that specifically recognized an HLA antigen of each of the iPS cells proliferated, and CFSE became negative.

[Experimental Example 21]

(Test 3 of T cell activation)

(1) 604B1 iPS cells having an HLA haplotype shown in Table 28, (2) a clone #3-11 which was produced in Experimental Example 11 and in which the A*01:01, B*37:01, and C*06:02 alleles of the 604B1 iPS cells had been knocked out, (3) same clone #3-12, (4) 1383D2 iPS cells having an HLA haplotype in Table 29, and (5) 604B1 iPS cells (clone # 1) which were produced in Experimental Example 18 and in which the B2M had been knocked out were respectively differentiation-induced into EB-derived blood-cell-like cells.

[Table 28]

| HLA haplotype of 604B1 iPS cell | | | | | |
|---|---|---|---|---|---|
| HLA-A | | HLA-B | | HLA-C | |
| A*01:01 | A*24:02 | B*07:02 | B*37:01 | C*06:02 | C*07:02 |

[Table 29]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

Subsequently, CD8-positive T cells recovered by sorting from peripheral blood mononuclear cells having an HLA haplotype shown in Table 30 (PBMC, type "LP_266," CTL, corresponding to the above-mentioned recipient C) were stained with CFSE (Cayman Chemical Co., Ltd.). The cells were co-cultured with EB-derived blood-cell-like cells differentiated from the respective cells of (1) to (5) in the presence of IL-2 and IFN-γ for 8 days.

[Table 30]

| HLA haplotype of PBMC (LP 266, CTL) | | | | | |
|---|---|---|---|---|---|
| HLA-A | | HLA-B | | HLA-C | |
| A*03:01 | A*24:02 | B*07:02 | B*40:02 | C*03:05 | C*07:02 |

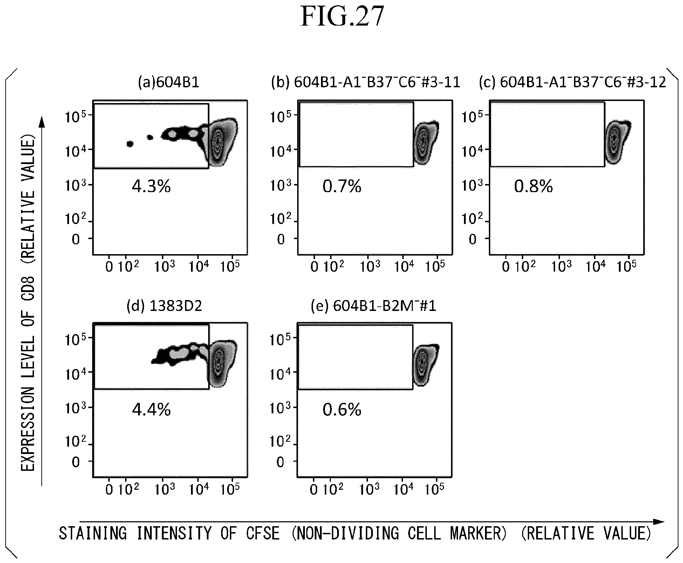

After co-culture, flow cytometric analysis was performed to examine fluorescence of CFSE. Figs. 27(a) to 27(e) are graphs showing results of flow cytometry. Figs. 27(a) to 27(e) show the results of CD8-positive T cells co-cultured with EB-derived blood-cell-like cells differentiated from the respective cells of (1) to (5). As a result, it was confirmed that a T cell population that specifically recognized an HLA antigen of each of the iPS cells proliferated, and CFSE became negative.

[Experimental Example 22]

(Preparation of HLA-antigen-reactive CD8-positive T cells)

CD8-positive T cells reactive with an HLA antigen of 604B1 iPS cells were prepared. Peripheral blood mononuclear cell (PBMC)-derived T cells were present in combination with T cells that recognized various antigens. Among these T cells, CD8-positive T cells reactive with the HLA antigen of 604B1 iPS cells were stimulated to proliferate and were recovered.

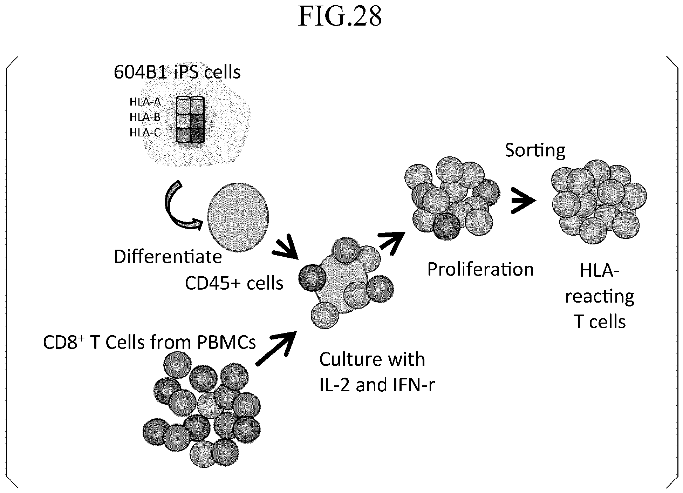

Fig. 28 is a schematic diagram showing a procedure of the present experimental example. As shown in Fig. 28, first, 604B1 iPS cells were differentiated into EB-derived blood-cell-like cells. The EB-derived blood-cell-like cells include CD45-positive leukocytes. Subsequently, CD8-positive T cells contained in PBMC were stained with CFSE (Cayman Chemical Co.) and co-cultured with EB-derived blood-cell-like cells differentiated from 604B1 iPS cells in the presence of IL-2 and IFN-γ for 8 days.

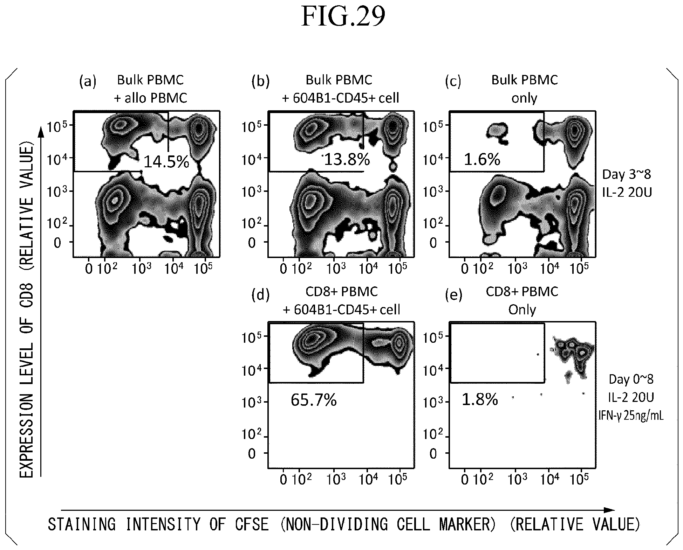

Subsequently, after co-culture, flow cytometric analysis was performed to examine fluorescence of CFSE. Figs. 29(a) to 29(e) are graphs showing results of flow cytometry.

Fig. 29(a) shows the results of co-culturing a PBMC cell population with a PBMC cell population of allogeneic cells (allo), Fig. 29(b) shows the results of co-culturing the PBMC cell population with EB-derived blood-cell-like cells differentiated from 604B1 iPS cells, Fig. 29(c) shows the results of culturing only the PBMC cell population, Fig. 29(d) shows the results of co-culturing CD8-positive T cells recovered from the PBMC cell population by sorting with EB-derived blood-cell-like cells differentiated from 604B1 iPS cells, and Fig. 29(e) shows the results of culturing only CD8-positive T cells recovered from the PBMC cell population by sorting. As a result, it was confirmed that a T cell population that specifically recognized an incompatible HLA antigen proliferated, and CFSE became negative.

Subsequently, CD8-positive T cells that became CFSE-negative as shown in Fig. 29(d) were sorted and recovered. Thereby, T cells reactive with the HLA antigen of 604B1 iPS cells were obtained.

[Experimental Example 23]

(Test 1 of T cell cytotoxicity)

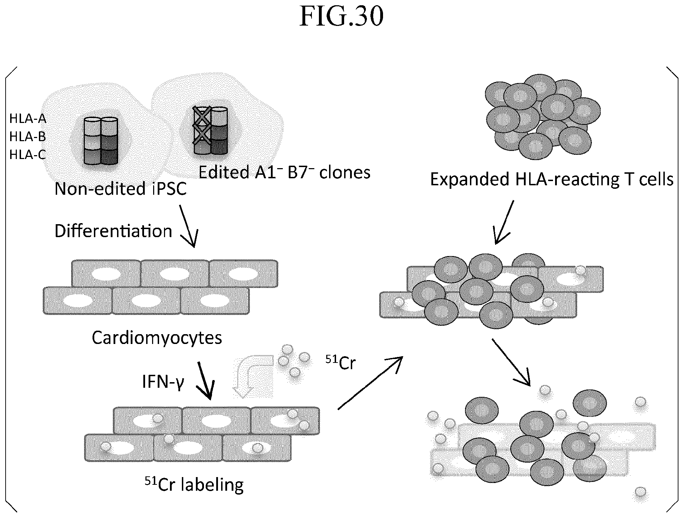

Fig. 30 is a schematic diagram showing a procedure of the present experimental example. First, (1) 604B1 iPS cells having an HLA haplotype shown in Table 31, (2) a clone #8 which was produced in Experimental Example 8 and in which the A*01:01 and B*07:02 alleles of the 604B1 iPS cells had been knocked out, (3) same clone #10, and (4) 1383D2 iPS cells (clone #1) which were produced in Experimental Example 17 and in which the B2M had been knocked out, were respectively differentiation-induced into cardiomyocytes.

[Table 31]

| HLA haplotype of 604B1 iPS cell | | | | | |
| --- | --- | --- | --- | --- | --- |
| HLA-A | | HLA-B | | HLA-C | |
| A*01:01 | A*24:02 | B*07:02 | B*37:01 | C*06:02 | C*07:02 |

Subsequently, each cardiomyocyte was stimulated with IFN-γ to induce the expression of HLA protein. Subsequently, in order to perform a $^{51}$Cr release assay to be described later, $^{51}$Cr (chromium) was added to a medium and incorporated into each cardiomyocyte.

Subsequently, CD8-positive T cells reactive with the HLA antigen of 604B1 iPS cells prepared in Experimental Example 22 were added to the medium of each cardiomyocyte at various ratios to examine whether each cardiomyocyte was attacked. That is, the CD8 T cells reactive with the HLA antigen prepared in Experimental Example 22 were used as effector cells, and the above-described cardiomyocytes derived from iPS cells were used as target cells. A ratio of the number of CD8-positive T cells to be added was 0.625, 1.25, 2.5, 5, and 10 with respect to cardiomyocytes 1.

When cardiomyocytes are attacked by CD8-positive cells, the cells are damaged and $^{51}$Cr is released from the inside of the cells. Accordingly, by measuring $^{51}$Cr released into the medium, it is possible to determine whether or not each cardiomyocyte is attacked. This is called $^{51}$Cr release assay. An amount of $^{51}$Cr contained in the supernatant of the cardiomyocytes that had not been treated after labeling with $^{51}$Cr was defined as 0% Specific Lysis. A value obtained by subtracting an amount of $^{51}$Cr released in the supernatant when $^{51}$Cr-labeled cardiomyocytes were cultured in a normal medium from an amount of $^{51}$Cr released in the supernatant when $^{51}$Cr-labeled cardiomyocytes were treated by a surfactant (Triton-X) and killed was defined as 100% Specific Lysis.

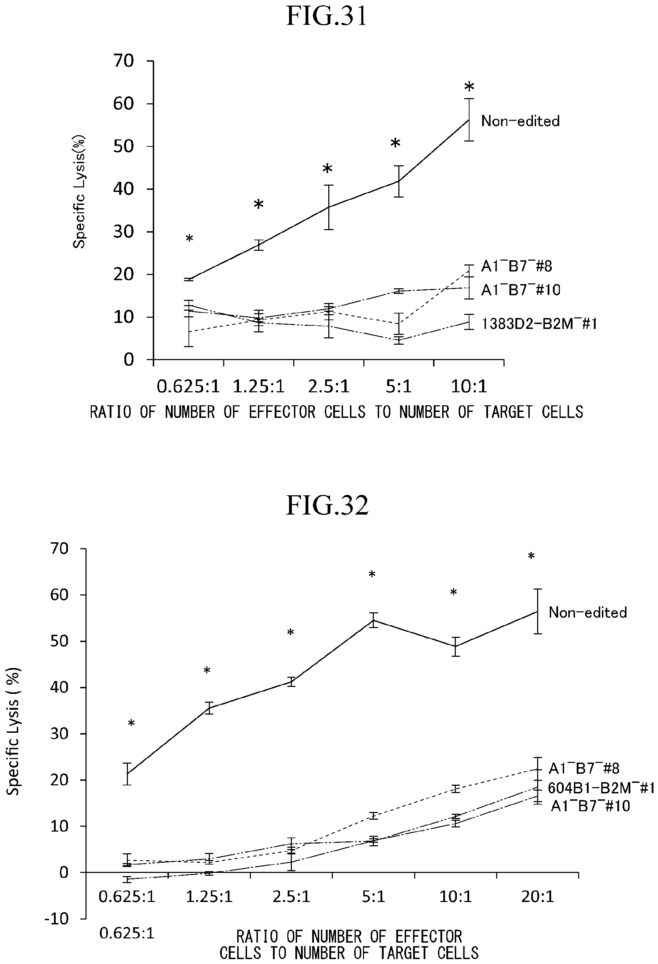

Fig. 31 is a graph showing a ratio of each cardiomyocyte damaged and lysed. In Fig. 31, the symbol "*" indicates that there is a significant difference at P < 0.05, "Non-edited" indicates the results of cardiomyocytes differentiation-induced the 604B1 iPS cells of (1), "A1⁻B7⁻#8" indicates the results of the cardiomyocytes differentiation-induced the clone #8 of (2), "A1⁻B7⁻#10" indicates the results of the cardiomyocytes differentiation-induced the clone #10 of (3), and "1383D2-B2M⁻#1" indicates the results of cardiomyocytes differentiation-induced the 1383D2 iPS cells (clone #1) of (4) in which B2M had been knocked out.

As a result, it was revealed that the cardiomyocytes differentiation-induced from the 604B1 iPS cells had a higher degree of injury when a proportion of CD8-positive T cells as effectors was increased.

In contrast, it was clarified that the cardiomyocytes differentiation-induced from the 1383D2 iPS cells in which B2M had been knocked out were not damaged even when a proportion of CD8-positive T cells as effectors was increased. Similarly, it was clarified that the cardiomyocytes differentiation-induced from the clones #8 and #10 in which the A*01:01 and B*07:02 alleles of 604B1 iPS cells were knocked out were not damaged even when a proportion of CD8-positive T cells as effectors was increased.

These results show that it is possible to reduce a rejection reaction in a case where cells were differentiation-induced and allogeneically transplanted by disrupting the HLA allele of iPS cells.

In addition, the fact that the respective iPS cells of (2) to (4) were able to be differentiation-induced into cardiomyocytes indicates that expression of HLA protein could be maintained by the IFN-γ treatment while maintaining pluripotency of pluripotent stem cells.

[Experimental Example 24]

(Test 2 of T cell cytotoxicity)

The iPS cells of (1) to (4) similar to those of Experimental Example 23 were differentiation-induced into EB-derived blood-cell-like cells, and $^{51}$Cr release assay was performed.

First, (1) 604B1 iPS cells, (2) a clone #8 which was produced in Experimental Example 7 and in which the A*01:01 and B*07:02 alleles of the 604B1 iPS cells had been knocked out, (3) same clone #10, and (4) 604B1 iPS cells (clone #1) which were produced in Experimental Example 18 and in which the B2M had been knocked out were respectively differentiation-induced into EB-derived blood-cell-like cells. The EB-derived blood-cell-like cells include blood cells.

Subsequently, CD8-positive T cells reactive with the HLA antigen of 604B1 iPS cells prepared in Experimental Example 22 were added to the medium of each EB-derived blood-cell-like cell at various ratios to examine whether each EB-derived blood-cell-like cell was attacked. That is, the CD8 T cells reactive with the HLA antigen prepared in Experimental Example 21 were used as effector cells, and the above-described EB-derived blood-cell-like cells were used as target cells. A ratio of the number of CD8-positive T cells to be added was 0.625, 1.25, 2.5, 5, 10, and 20 with respect to EB-derived blood-cell-like cells 1.

Fig. 32 is a graph showing a ratio of each EB-derived blood-cell-like cell damaged and lysed. In Fig. 32, the symbol "*" indicates that there is a significant difference at P < 0.05, "Non-edited" indicates the results of EB-derived blood-cell-like cells differentiation-induced from the 604B1 iPS cells of (1), "A1⁻B7⁻#8" indicates the results of the EB-derived blood-cell-like cells differentiation-induced from the clone #8 of (2), "A1⁻B7⁻#10" indicates the results of the EB-derived blood-cell-like cells differentiation-induced from the clone #10 of (3), and "604B1-B2M⁻#1" indicates the results of EB-derived blood-cell-like cells differentiation-induced from the 604B1 iPS cells (clone #1) of (4) in which B2M had been knocked out.

As a result, it was revealed that the EB-derived blood-cell-like cells differentiation-induced from the 604B1 iPS cells had a higher degree of injury when a proportion of CD8-positive T cells as effectors was increased.

In contrast, it was clarified that the EB-derived blood-cell-like cells differentiation-induced from the 604B1 iPS cells in which B2M had been knocked out were not damaged even when a proportion of CD8-positive T cells as effectors was increased. Similarly, it was clarified that the EB-derived blood-cell-like cells differentiation-induced from the clones #8 and #10 in which the A*01:01 and B*07:02 alleles of 604B1 iPS cells were knocked out were not damaged even when a proportion of CD8-positive T cells as effectors was increased.

These results show that it is possible to reduce a rejection reaction in a case where cells were differentiation-induced and allogeneically transplanted by disrupting the HLA allele of iPS cells.

In addition, the fact that the respective iPS cells of (2) to (4) were able to be differentiation-induced into EB-derived blood-cell-like cells indicates that expression of HLA protein could be maintained by the IFN-γ treatment while maintaining pluripotency of pluripotent stem cells.

[Experimental Example 25]

(Test 3 of T cell cytotoxicity)

The same examination as in Experimental Example 24 was performed except that iPS cells different from those in Experimental Example 24 were used. First, (1) 585A1 iPS cells having an HLA haplotype shown in Table 32, (2) a clone #2 which was produced in Experimental Example 9 and in which the B*07:02 and C*07:02 alleles of the 585A1 iPS cells had been knocked out, (3) same clone #11, (4) same clone #26, and (5) 604B1 iPS cells (clone # 1) which were produced in Experimental Example 18 and in which the B2M had been knocked out were respectively differentiation-induced into EB-derived blood-cell-like cells. The EB-derived blood-cell-like cells include blood cells.

[Table 32]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

Subsequently, CD8-positive T cells as effectors derived from peripheral blood mononuclear cells having HLA haplotypes shown in Table 33 (corresponding to PBMC, type "2010113203," Wako, the above-mentioned recipient B) were added to a medium of each EB-derived blood-cell-like cell at various ratios to examine whether or not each EB-derived blood-cell-like cell was attacked. As the effector cells, the CD8-positive T cells in CFB-negative PBMC shown in Fig. 26(a) of Experimental Example 20 were sorted and used. In addition, target cells were the above-mentioned EB-derived blood-cell-like cells. A ratio of the number of CD8-positive T cells to be added as effectors was 0.625, 1.25, 2.5, 5, 10, and 20 with respect to EB-derived blood-cell-like cells 1.

[Table 33]

| HLA haplotype of PBMC ("2010113203," Wako) | | | | | |
|---|---|---|---|---|---|
| HLA-A | | HLA-B | | HLA-C | |
| A*24:02 | A*24:02 | B*40:06 | B*52:01 | C*08:01 | C*12:02 |

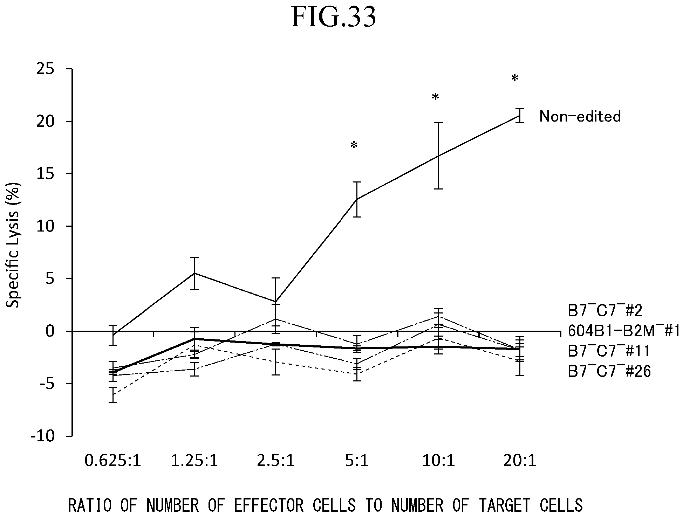

Fig. 33 is a graph showing a ratio of each EB-derived blood-cell-like cell damaged and lysed. In Fig. 33, the symbol "*" indicates that there is a significant difference at P < 0.05, "Non-edited" indicates the results of EB-derived blood-cell-like cells differentiation-induced from the 585A1 iPS cells of (1), "B7⁻C7⁻#2" indicates the results of the EB-derived blood-cell-like cells differentiation-induced from the clone #2 of (2), "B7⁻C7⁻#11" indicates the results of the EB-derived blood-cell-like cells differentiation-induced from the clone #11 of (3), "B7⁻C7⁻#26" indicates the results of the EB-derived blood-cell-like cells differentiation-induced from the clone #26 of (4), and "604B1-B2M⁻#1" indicates the results of EB-derived blood-cell-like cells differentiation-induced from the 604B1 iPS cells (clone #1) of (5) in which B2M had been knocked out.

As a result, it was revealed that the EB-derived blood-cell-like cells differentiation-induced from the 585A1 iPS cells had a higher degree of injury when a proportion of CD8-positive T cells as effectors was increased.

In contrast, it was clarified that the EB-derived blood-cell-like cells differentiation-induced from the 604B1 iPS cells in which B2M had been knocked out were not damaged even when a proportion of CD8-positive T cells as effectors was increased. Similarly, it was clarified that the EB-derived blood-cell-like cells differentiation-induced from the clones #2, #11, and #26 in which the B*07:02 and C*07:02 alleles of 585A1 iPS cells were knocked out were not damaged even when a proportion of CD8-positive T cells as effectors was increased.

These results show that it is possible to reduce a rejection reaction in a case where cells were differentiation-induced and allogeneically transplanted by disrupting the HLA allele of iPS cells.

In addition, the fact that the respective iPS cells of (2) to (5) were able to be differentiation-induced into EB-derived blood-cell-like cells indicates that expression of HLA protein could be maintained by the IFN-γ treatment while maintaining pluripotency of pluripotent stem cells.

[Experimental Example 26]

(Test 4 of T cell cytotoxicity)

The 604B1 iPS cells having HLA haplotypes shown in Table 34 and the 604B 1 iPS cells (clone #1) which were produced in Experimental Example 18 and in which B2M had been knocked out were mixed with cells obtained by labeling, with CFSE, peripheral blood mononuclear cells having an HLA haplotype shown in Table 35 (PBMC, type "LP_266," CTL, corresponding to the above-mentioned recipient C). These cells were co-cultured for 7 days.

[Table 34]

| HLA haplotype of 604B1 iPS cell | | | | | |
|---|---|---|---|---|---|
| HLA-A | | HLA-B | | HLA-C | |
| A*01:01 | A*24:02 | B*07:02 | B*37:01 | C*06:02 | C*07:02 |

[Table 35]

| HLA haplotype of PBMC (LP 266, CTL) | | | | | |
|---|---|---|---|---|---|
| HLA-A | | HLA-B | | HLA-C | |
| A*03:01 | A*24:02 | B*07:02 | B*40:02 | C*03:05 | C*07:02 |

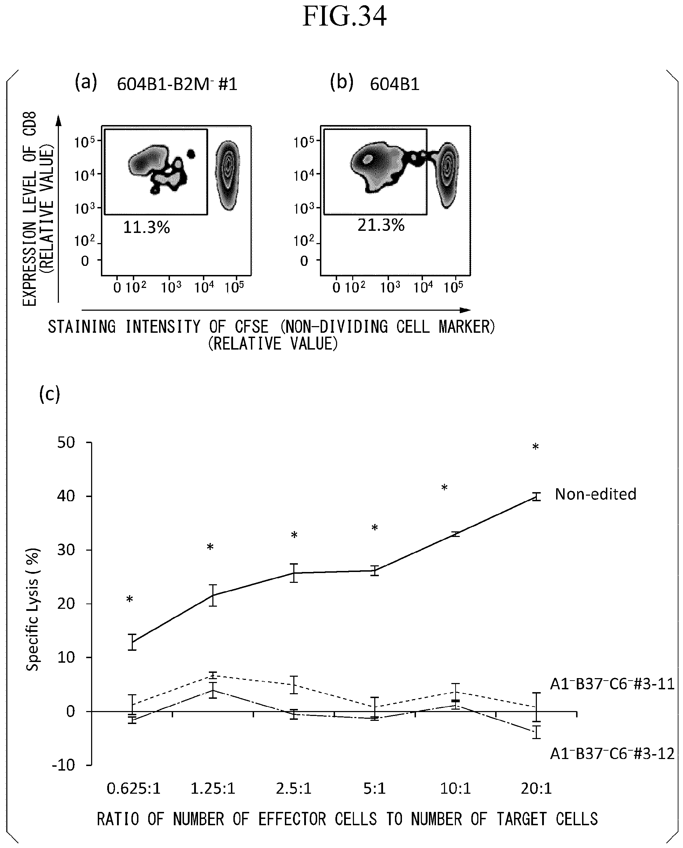

After co-culture, flow cytometric analysis was performed to examine fluorescence of CFSE. Fig. 34(a) and 34(b) are graphs showing results of flow cytometry. In Figs. 34(a) and 34(b), "604B1-B2M⁻#1" indicates the results of PBMC co-cultured with the 604B1 iPS cells (clone # 1) in which B2M had been knocked out, and "604B1" indicates the results of PBMC co-cultured with the 604B1 iPS cells.

As a result, as shown in Fig. 34(a), 11.3% of CD8-positive and CSFE-negative T cells were contained in the PBMC co-cultured with the 604B1 iPS cells (clone #1) in which B2M had been knocked out. Furthermore, as shown in Fig. 34(b), 21.3% of CD8-positive and CSFE-negative T cells were contained in the PBMC co-cultured with the 604B1 iPS cells. Subsequently, from the PBMC co-cultured with the 604B1 iPS cells shown in Fig. 34(b), CD8-positive and CSFE-negative cells were sorted and recovered as effector CD8-positive T cells.

Subsequently, (1) 604B1 iPS cells; (2) clone #3-11 which was produced in Experimental Example 11 and in which A*01:01 allele, B*37:01 allele, and C*06:02 allele of the 604B1 iPS cells had been knocked out; and (3) the same clone # 3-12 were respectively differentiation-induced into EB-derived blood-cell-like cells. The EB-derived blood-cell-like cells include blood cells.

Subsequently, the recovered effector CD8-positive T cells were added to the medium of each EB-derived blood-cell-like cell at various ratios to examine whether each EB-derived blood-cell-like cell was attacked. That is, target cells were EB-derived blood-cell-like cells that had been differentiation-induced from the cells of (1) to (3). A ratio of the number of CD8-positive T cells to be added as effectors was 0.625, 1.25, 2.5, 5, 10, and 20 with respect to EB-derived blood-cell-like cells 1.

Fig. 34(c) is a graph showing a ratio of each EB-derived blood-cell-like cell damaged and lysed. In Fig. 34(c), the symbol "*" indicates that there is a significant difference at P < 0.05, "Non-edited" indicates the results of EB-derived blood-cell-like cells differentiation-induced from the 604B1 iPS cells of (1), "A1⁻B37⁻C6⁻#3-11" indicates the results of the EB-derived blood-cell-like cells differentiation-induced from the clone #3-11 of (2), and "A1⁻B37⁻C6⁻#3-12" indicates the results of the EB-derived blood-cell-like cells differentiation-induced from the clone #3-12 of (3).

As a result, it was revealed that the EB-derived blood-cell-like cells differentiation-induced from the 604B1 iPS cells had a higher degree of injury when a proportion of CD8-positive T cells as effectors was increased.

In contrast, it was clarified that the EB-derived blood-cell-like cells differentiation-induced from the clones #3-11 and #3-12 in which the A*01:01 allele, B*37:01 allele, and C*06:02 allele of 604B1 iPS cells were knocked out were not damaged even when a proportion of CD8-positive T cells as effectors was increased.

These results show that it is possible to reduce a rejection reaction in a case where cells were differentiation-induced and allogeneically transplanted by disrupting the HLA allele of iPS cells.

In addition, the fact that the respective iPS cells of (2) and (3) were able to be differentiation-induced into EB-derived blood-cell-like cells indicates that expression of HLA protein could be maintained by the IFN-γ treatment while maintaining pluripotency of pluripotent stem cells.

[Experimental Example 27]

(Reactivity test 1 of NK cells)

(1) 604B1 iPS cells having an HLA haplotype shown in Table 36, (2) a clone #8 which was produced in Experimental Example 8 and in which the A*01:01 and B*07:02 alleles of the 604B1 iPS cells had been knocked out, (3) same clone #10, (4) 1383D2 iPS cells having an HLA haplotype in Table 37, and (5) 604B1 iPS cells (clone # 1) which were produced in Experimental Example 18 and in which the B2M had been knocked out were respectively differentiated into EB-derived blood-cell-like cells. The EB-derived blood-cell-like cells include CD45-positive leukocytes.

[Table 36]

| HLA haplotype of 604B1 iPS cell | | | | | |
|---|---|---|---|---|---|
| HLA-A | | HLA-B | | HLA-C | |
| A*01:01 | A*24:02 | B*07:02 | B*37:01 | C*06:02 | C*07:02 |

[Table 37]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

In addition, CD56-positive cells (NK cells) were recovered from PBMC having an HLA haplotype shown in Table 38 (type "LP_194," CTL, corresponding to the above-mentioned recipient A) by sorting. Subsequently, the recovered NK cells were co-cultured with each of differentiated CD45-positive leukocytes in the presence of the anti-CD107a antibody. As a result, when the NK cells were activated, they became CD107a-positive. After co-culture, flow cytometric analysis was performed to measure a percentage of activated NK cells.

[Table 38]

| HLA haplotype of PBMC (LP_194, CTL) | | | | | |
|---|---|---|---|---|---|
| HLA-A | | HLA-B | | HLA-C | |
| A*24:02 | A*24:02 | B*37:01 | B*39:06 | C*06:02 | C*07:02 |

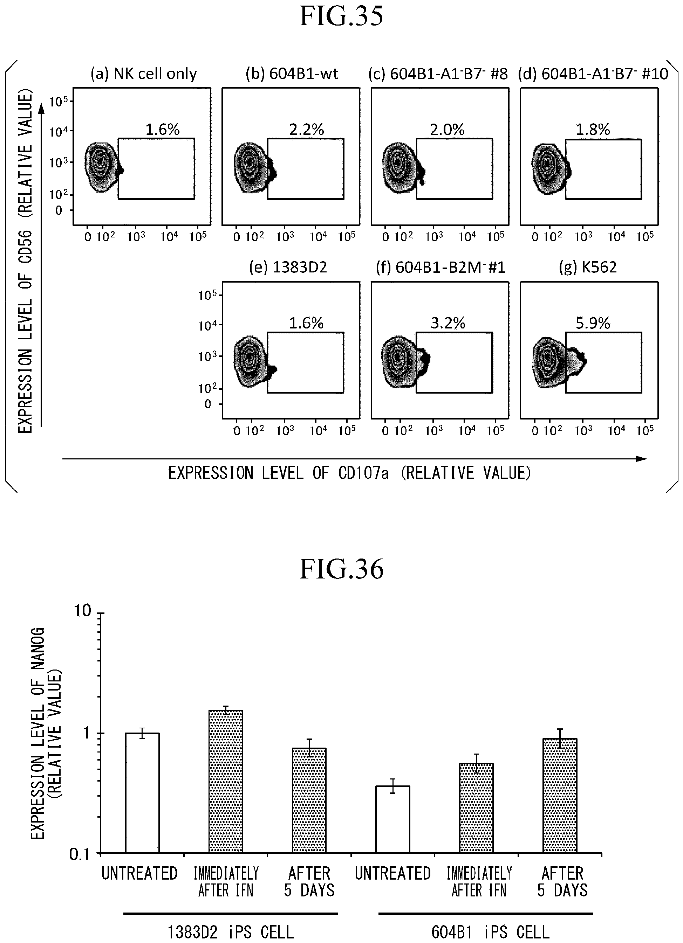

Figs. 35(a) to 35(g) are graphs showing results of flow cytometric analysis. Fig. 35 (a) shows the results of culturing only NK cells and analyzing them. Fig. 35(b) shows the results of analysis of NK cells co-cultured with EB-derived blood-cell-like cells differentiation-induced from 604B1 iPS cells. Fig. 35(c) shows the results of analysis of NK cells co-cultured with EB-derived blood-cell-like cells differentiation-induced from the clone #8 in which the A*01:01 and B*07:02 alleles of 604B1 iPS cells were knocked out. Fig. 35(d) shows the results of analysis of NK cells co-cultured with EB-derived blood-cell-like cells differentiation-induced from the clone #10. Fig. 35(e) shows the results of analysis of NK cells co-cultured with EB-derived blood-cell-like cells differentiation-induced from 1383D2 iPS cells. Fig. 35(f) shows the results of analysis of NK cells co-cultured with EB-derived blood-cell-like cells differentiation-induced from 604B1 iPS cells in which B2M had been knocked out. Fig. 35(g) shows the results of analysis of NK cells co-cultured with K562 cells known not to express HLA antigen, as a positive control.

As a result, it was revealed that 5.9% of NK cells co-cultured with K562 cells were activated. In addition, it was revealed that 3.2% of NK cells co-cultured with EB-derived blood-cell-like cells differentiation-induced from 604B1 iPS cells in which B2M had been knocked out and which did not express HLA antigen were activated.

In contrast, in the clones #8 and #10 in which the A*01:01 and B*07:02 alleles of 604B1 iPS cells had been knocked out, expression of the other A*24:02 and B*37:01 alleles remained. Based on this result, it was revealed that activation of NK cells co-cultured with EB-derived blood-cell-like cells differentiation-induced therefrom was inhibited.

That is, it was revealed that cells in which B2M had been knocked out were significantly attacked by NK cells, whereas cells in which some HLA alleles remained were able to avoid attack by NK cells.

[Experimental Example 28]

(Confirmation test 1 of pluripotency)

For 1383D2 iPS cells and 604B1 iPS cells, (1) cells in an untreated state, (2) cells immediately after being treated with 50 ng/mL IFN-γ for 48 hours, and (3) cells 5 days after being treated with 50 ng/mL IFN-γ for 48 hours were respectively prepared, and mRNA was extracted. Then, after synthesizing cDNA using Reverse Tra Ase qPCR RT Master Mix (Toyobo), quantitative PCR was performed using a primer for amplifying human NANOG cDNA specifically expressed in pluripotent stem cells, and as a control of a total amount of mRNA, a primer for amplifying human ACTB cDNA.

The primer used for amplifying human NANOG cDNA and the primer used for amplifying human ACTB cDNA are shown in Table 39.

[Table 39]

| Primer name | Base sequence | SEQ ID NO. |
|---|---|---|
| hNANOG(+483 5)-fwd | CTTCACTTCCCAGGTGCAA | 66 |
| hNANOG(+483 5)-rev | AGGCTAGCCAACATGAGGAA | 67 |
| hACTB-Fwd | CCAACCGCGAGAAGATGA | 68 |
| hACTB-Rev | CCAGAGGCGTACAGGGATAG | 69 |

Fig. 36 is a graph showing the results of quantitative PCR in which an expression level of the NANOG gene was measured. The expression level of the NANOG gene was shown as a relative value with an expression level of the NANOG gene relative to the ACTB gene in "untreated" 1383D2 cells being 1.

In Fig. 36, the term "untreated" indicates the results of iPS cells that were not treated with IFN-γ and cultured under normal undifferentiated condition, the term "immediately after IFN" indicates immediately after being treated with IFN-γ for 48 hours, and the term "5 days after" indicates the results of cells 5 days after being treated with IFN-γ for 48 hours.

As a result, it was revealed that an expression level of NANOG, which has been widely used as a pluripotency marker, did not decrease even when iPS cells were treated with IFN-γ for 48 hours. This result indicates that pluripotency is maintained even when iPS cells are treated with IFN-γ.

[Experimental Example 29]

(Confirmation test 2 of pluripotency)

(1) 604B1 iPS cells, (2) a clone #3-11 which was produced in Experimental Example 11 and in which the A*01:01, B*37:01, and C*06:02 alleles of the 604B1 iPS cells had been disrupted, (3) same clone #3-12, (4) clone #1 in which a B2M gene of 604B1 iPS cells produced in Experimental Example 18 was had been disrupted, (5) 1383D2 iPS cells, (6) clone # 1 in which a B2M gene of 1383D2 iPS cells produced in Experimental Example 17 had been disrupted were respectively cultured under undifferentiated conditions, and morphology of the respective cells was observed with a phase contrast microscope.

The culture under undifferentiated conditions was carried out using an AK03N medium (StemFit, Ajinomo) as a medium and using a type "iMatrix-511" (Nippi) as a cell matrix.

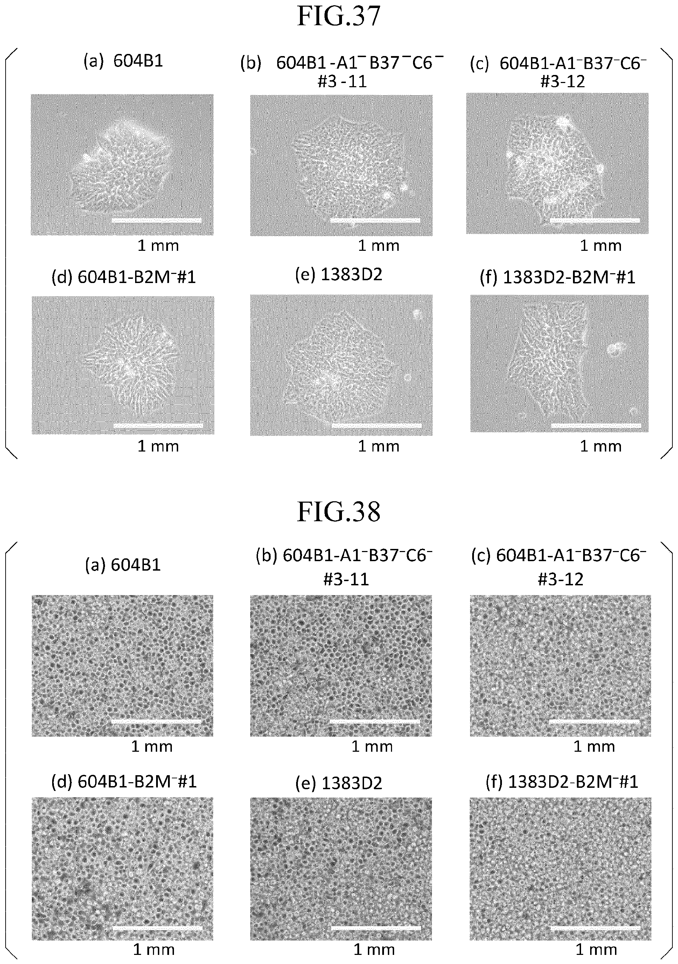

Figs. 37(a) to 37(f) are photographs of the cells of (1) to (5) respectively captured with a phase contrast microscope. A scale bar is 1 mm.

As a result, it was revealed that the HLA allele and the B2M allele were disrupted, and the IFN-γ-treated iPS cells also formed flat colonies as characteristics of undifferentiated pluripotent stem cells and proliferated. This result indicates that pluripotency is maintained even when iPS cells are treated with IFN-γ. This result also indicates that pluripotency is maintained even when the HLA allele or B2M allele of iPS cells has been disrupted.

[Experimental Example 30]

(Confirmation test 3 of pluripotency)

(1) 604B1 iPS cells, (2) a clone #3-11 which was produced in Experimental Example 11 and in which the A*01:01, B*37:01, and C*06:02 alleles of the 604B1 iPS cells had been disrupted, (3) same clone #3-12, (4) clone #1 in which a B2M gene of 604B1 iPS cells produced in Experimental Example 18 was had been disrupted, (5) 1383D2 iPS cells, (6) clone # 1 in which a B2M gene of 1383D2 iPS cells produced in Experimental Example 17 had been disrupted were respectively cultured under conditions for inducing blood cell differentiation, and morphology of EB-derived blood-cell-like cells was observed with a phase contrast microscope.

Differentiation-induction of blood cell of iPS cells was performed as follows. First, undifferentiated iPS cells were seeded on an ultra-low adhesion culture plate (Corning), and EB (embryoid body) was formed for 2 days in an AK03N medium containing Y-27633. Subsequently, the cells were cultured for 3 days in a StemPro-34 SFM medium (Thermo Fisher Scientific) containing human bFGF (Oriental Yeast Co., Ltd.), human VEGF (R & D Systems), Insulin-Transferrin-Selenium (ITS) (Thermo Fisher Scientific Co.), GlutaMax-I (Thermo Fisher Scientific), L-ascorbic acid 2-phosphate sesquimagnesium salt hydrate (Sigma), 1-Thioglycerol (MTG, NACALAI TESQUE, INC.), and human BMP4 (R & D Systems). Thereafter, human BMP4 was removed, human SCF was added, and the cells were cultured for 2 days. Subsequently, human Flt3-Ligand (Peprotech) and human TPO (Peprotech) were added and cultured. As a result, EB-derived blood-cell-like cells were generated.

Figs. 38(a) to 38(f) are photographs of EB-derived blood-cell-like cells of (1) to (5) on the 22nd day of induction of differentiation, which were captured with a phase contrast microscope. A scale bar is 1 mm.

As a result, it was revealed that the HLA allele and the B2M allele were disrupted, and the iPS cells treated with IFN-γ could also be able to be differentiation-induced into blood cells. This result indicates that pluripotency is maintained even when iPS cells are treated with IFN-γ. This result also indicates that pluripotency is maintained even when the HLA allele or B2M allele of iPS cells has been disrupted.

[Experimental Example 31]

(Test 5 of T cell cytotoxicity)

(1) Cells (clone # 1) which were produced in Experimental Example 17 and in which the B2M allele of the 1383D2 iPS cells having HLA haplotypes shown in Table 40 had been knocked out, and (2) 585A1 iPS cells having HLA haplotypes shown in Table 41, were stained with CFSE (Cayman Chemical Company), and were co-cultured for 7 days with peripheral blood mononuclear cells having HLA haplotypes shown in Table 42 (PBMC, type "LP 275," CTL).

[Table 40]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

[Table 41]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

[Table 42]

| HLA haplotype of PBMC (LP 275, CTL) | | | | | |
|---|---|---|---|---|---|
| HLA-A | | HLA-B | | HLA-C | |
| A*02:06 | A*33:03 | B*40:01 | B*58:01 | C*03:02 | C*07:02 |

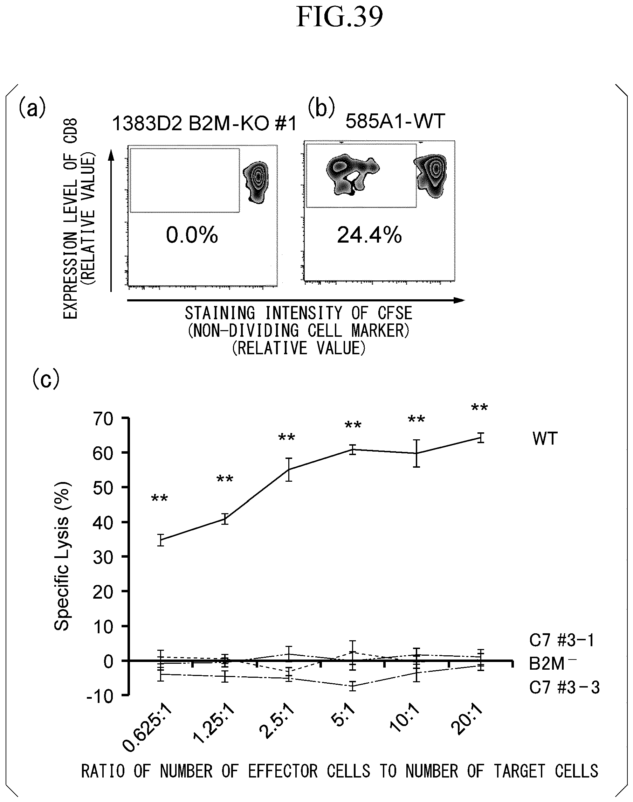

After co-culture, flow cytometric analysis was performed to examine fluorescence of CFSE. Fig. 39(a) and 39(b) are graphs showing results of flow cytometry. In Figs. 39(a) and 39(b), "1383D2 B2M-KO #1" indicates the results of PBMC co-cultured with the 1383D2 iPS cells (clone # 1) in which B2M had been knocked out, and "585A1-WT" indicates the results of PBMC co-cultured with the 585A1 iPS cells.

As a result, as shown in Fig. 39(a), no CD8-positive and CSFE-negative T cells were present in the PBMC co-cultured with the 1383D2 iPS cells (clone #1) in which B2M had been knocked out. In addition, as shown in Fig. 39(b), the PBMC co-cultured with the 585A1 iPS cells contained 24.4% of CD8-positive T cells that had been activated and became CSFE-negative. Subsequently, from the PBMC co-cultured with the 585A1 iPS cells shown in Fig. 39(b), CD8-positive and CSFE-negative cells were sorted and recovered as effector CD8-positive T cells.

Subsequently, (1) 585A1 iPS cells, (2) clone #3-1 which was produced in Experimental Example 13 and in which A*24:02 allele, B*07:02 allele, B*52:01 allele, and C*12:02 allele of 585A1 iPS cells had been knocked out, (3) the same clone # 3-3, and (4) cells (not subcloned bulk cells) produced in Experimental Example 46 to be described later and in which the B2M allele of 585A1 iPS cells had been knocked out, were respectively differentiation-induced into EB-derived blood-cell-like cells. The EB-derived blood-cell-like cells include blood cells.

Subsequently, the recovered effector CD8-positive T cells were added to the medium of each EB-derived blood-cell-like cell at various ratios to examine whether each EB-derived blood-cell-like cell was attacked. That is, target cells were EB-derived blood-cell-like cells that had been differentiation-induced from the cells of (1) to (4). A ratio of the number of CD8-positive T cells to be added as effectors was 0.625, 1.25, 2.5, 5, 10, and 20 with respect to EB-derived blood-cell-like cells 1.

Fig. 39(c) is a graph showing a ratio of each EB-derived blood-cell-like cell damaged and lysed. In Fig. 39(c), the symbol "**" indicates that there is a significant difference at $P < 0.01$, "WT" indicates the results of EB-derived blood-cell-like cells differentiation-induced from the 585A1 iPS cells of (1), "C7#3-1" indicates the results of the EB-derived blood-cell-like cells differentiation-induced from the clone #3-1 of (2), "C7#3-3" indicates the results of the EB-derived blood-cell-like cells differentiation-induced from the clone #3-3 of (3), and "B2M⁻" indicates the results of the EB-derived blood-cell-like cells differentiation-induced from cells in which the B2M allele of the 585A1 iPS cells of (4) had been knocked out.

As a result, it was revealed that the EB-derived blood-cell-like cells differentiation-induced from the 585A1 iPS cells had a higher degree of injury when a proportion of CD8-positive T cells as effectors was increased.

In contrast, it was clarified that EB-derived blood-cell-like cells differentiation-induced from the clones #3-1 and #3-3 in which the A*24:02 allele, B*07:02 allele, B*52:01 allele, and C*12:02 allele of the 585A1 iPS cells were knocked out; and the EB-derived blood-cell-like cells differentiation-induced from cells in which the B2M allele of the 585A1 iPS cells were knocked out, were not damaged even when a proportion of CD8-positive T cells as effectors was increased.

These results further support that it is possible to reduce a rejection reaction in a case where cells were differentiation-induced and allogeneically transplanted by disrupting the HLA allele of iPS cells.

[Experimental Example 32]

(Test 6 of T cell cytotoxicity)

(1) Cells (clone # 1) which were produced in Experimental Example 17 and in which the B2M allele of the 1383D2 iPS cells having HLA haplotypes shown in Table 43 had been knocked out, and (2) 585A1 iPS cells having HLA haplotypes shown in Table 44, were co-cultured for 7 days with cells obtained by staining, with CFSE (Cayman Chemical Company), peripheral blood mononuclear cells having HLA haplotypes shown in Table 45 (PBMC, type "LP_329," CTL).

[Table 43]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

[Table 44]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

[Table 45]

| HLA haplotype of PBMC (LP_329, CTL) | | | | | |
|---|---|---|---|---|---|
| HLA-A | | HLA-B | | HLA-C | |
| A*29:02 | A*68:01 | B*39:06 | B*40:02 | C*03:05 | C*07:02 |

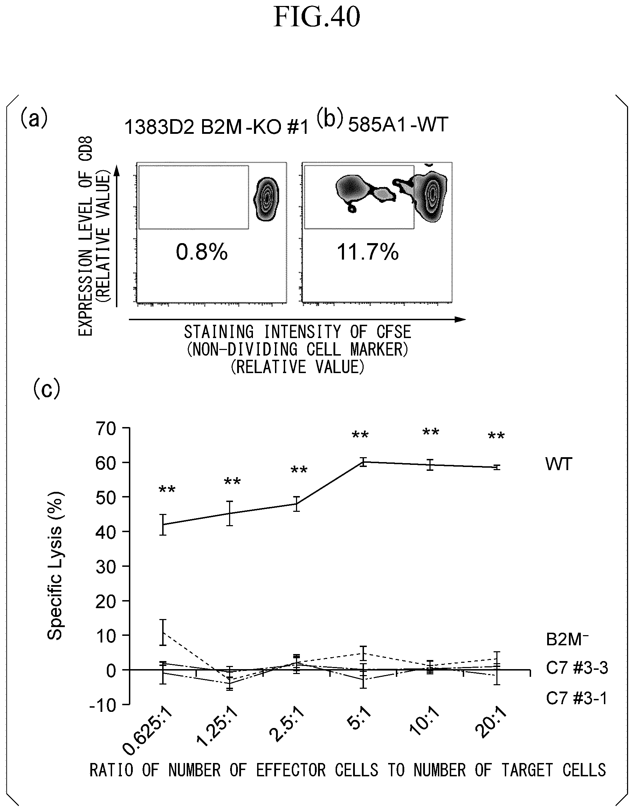

After co-culture, flow cytometric analysis was performed to examine fluorescence of CFSE. Fig. 40(a) and 40(b) are graphs showing results of flow cytometry. In Figs. 40(a) and 40(b), "1383D2 B2M-KO #1" indicates the results of PBMC co-cultured with the 1383D2 iPS cells (clone # 1) in which B2M had been knocked out, and "585A1-WT" indicates the results of PBMC co-cultured with the 585A1 iPS cells.

As a result, as shown in Fig. 40(a), no CD8-positive and CSFE-negative T cells were present in the PBMC co-cultured with the 1383D2 iPS cells (clone #1) in which B2M had been knocked out. In addition, as shown in Fig. 40(b), the PBMC co-cultured with the 585A1 iPS cells contained 11.7% of CD8-positive T cells that had been activated and became CSFE-negative. Subsequently, from the PBMC co-cultured with the 585A1 iPS cells shown in Fig. 40(b), CD8-positive and CSFE-negative cells were sorted and recovered as effector CD8-positive T cells.

Subsequently, (1) 585A1 iPS cells, (2) clone #3-1 which was produced in Experimental Example 13 and in which A*24:02 allele, B*07:02 allele, B*52:01 allele, and C*12:02 allele of 585A1 iPS cells had been knocked out, (3) the same clone # 3-3, and (4) cells (not subcloned bulk cells) produced in Experimental Example 46 to be described later and in which the B2M allele of 585A1 iPS cells had been knocked out, were respectively differentiation-induced into EB-derived blood-cell-like cells. The EB-derived blood-cell-like cells include blood cells.

Subsequently, the recovered effector CD8-positive T cells were added to the medium of each EB-derived blood-cell-like cell at various ratios to examine whether each EB-derived blood-cell-like cell was attacked. That is, target cells were EB-derived blood-cell-like cells that had been differentiation-induced from the cells of (1) to (4). A ratio of the number of CD8-positive T cells to be added as effectors was 0.625, 1.25, 2.5, 5, 10, and 20 with respect to EB-derived blood-cell-like cells 1.

Fig. 40(c) is a graph showing a ratio of each EB-derived blood-cell-like cell damaged and lysed. In Fig. 40(c), the symbol "*" indicates that there is a significant difference at P < 0.01, "WT" indicates the results of EB-derived blood-cell-like cells differentiation-induced from the 585A1 iPS cells of (1), "C7#3-1" indicates the results of the EB-derived blood-cell-like cells differentiation-induced from the clone #3-1 of (2), "C7#3-3" indicates the results of the EB-derived blood-cell-like cells differentiation-induced from the clone #3-3 of (3), and "B2M⁻" indicates the results of the EB-derived blood-cell-like cells differentiation-induced from cells in which the B2M allele of the 585A1 iPS cells of (4) had been knocked out.

As a result, it was revealed that the EB-derived blood-cell-like cells differentiation-induced from the 585A1 iPS cells had a higher degree of injury when a proportion of CD8-positive T cells as effectors was increased.

In contrast, it was clarified that EB-derived blood-cell-like cells differentiation-induced from the clones #3-1 and #3-3 in which the A*24:02 allele, B*07:02 allele, B*52:01 allele, and C*12:02 allele of the 585A1 iPS cells were knocked out; and the EB-derived blood-cell-like cells differentiation-induced from cells in which the B2M allele of the 1383D2 iPS cells were knocked out, were not damaged even when a proportion of CD8-positive T cells as effectors was increased.

These results further support that it is possible to reduce a rejection reaction in a case where cells were differentiation-induced and allogeneically transplanted by disrupting the HLA allele of iPS cells.

[Experimental Example 33]

(Cell transplantation experiment 1)

(1) 585A1 iPS cells, and (2) the clone #3-1 which was produced in Experimental Example 13 and in which the A*24:02 allele, B*07:02 allele, B*52:01 allele, and C*12:02 allele of the 585A1 iPS cells were knocked out, were respectively differentiation-induced into EB-derived blood-cell-like cells. Subsequently, the respective EB-derived blood-cell-like cells were labeled with luciferase.

Subsequently, one hour before the start of the experiment, each of the cell was transplanted into immunodeficient mice (NOD.Cg-Rag1^{tm1mom}Il2rg^{tm1Wjl}/SzJ (NRG), The Jackson Laboratory) by intraperitoneal administration. Subsequently, at the start of the experiment, effector CD8-positive T cells which were prepared in the same manner as in Experimental Example 32 and were derived from peripheral blood mononuclear cells (PBMC, type "LP_329," CTL) or a medium (control) were further intraperitoneally administered.

Then, at the time of cell transplantation (before T cell injection), 5 hours, 1 day, 3 days, and 7 days after the start of the experiment, luciferin, which is a substrate of luciferase, was administered to each mouse. Using an IVIS imaging system (trade name "IVIS Spectrum," SPI), fluorescence of the EB-derived blood-cell-like cells labeled with luciferase was captured, and a survival rate of the transplanted cells was measured.

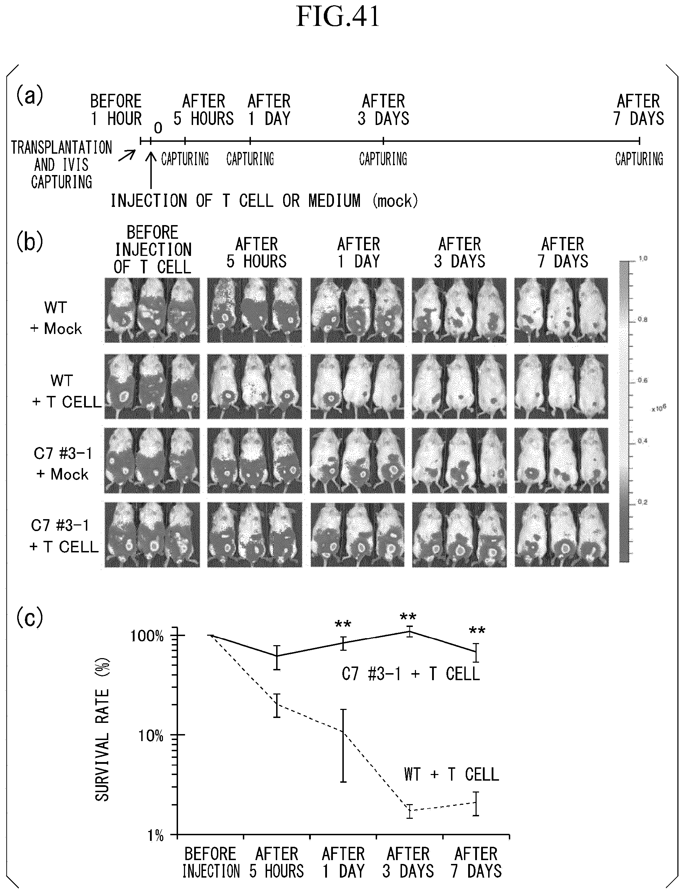

Fig. 41(a) is a diagram illustrating an experimental schedule. Furthermore, Fig. 41(b) shows a photograph showing results of capturing fluorescence of EB-derived blood-cell-like cells in each mouse. Furthermore, Fig. 41(c) is graph of a relative survival curve showing the results of calculating a survival rate of each EB-derived blood-cell-like cell in a case where the effector CD8-positive T cells were administered to the mouse, with a fluorescence intensity before T cell injection being 100%.

In Figs. 41(b) and 41(c), "WT" indicates the results of EB-derived blood-cell-like cells differentiation-induced from the 585A1 iPS cells of (1), "C7 #3-1" indicates the results of EB-derived blood-cell-like cells differentiation-induced from the clone #3-1 of (2), "Mock" indicates the results of administration of the medium as a control, and "T cell" indicates the results of administration of the effector CD8-positive T cells derived from peripheral blood mononuclear cells (PBMC, type "LP_329," CTL).

As a result, as shown in Fig. 41(c), it was confirmed that the EB-derived blood-cell-like cells differentiation-induced from 585A1 iPS cells had a reduced survival rate after administration of the effector CD8-positive T cells. On the other hand, it became clear that the EB-derived blood-cell-like cells differentiation-induced from the clone #3-1 in which the A*24:02 allele, B*07:02 allele, B*52:01 allele, and C*12:02 allele of 585A1 iPS cells were knocked out maintained a high survival rate even after the administration of the effector CD8-positive T cells.

These results further support that it is possible to reduce a rejection reaction in a case where cells were differentiation-induced and allogeneically transplanted by disrupting the HLA allele of iPS cells.

[Experimental Example 34]

(Reactivity test 2 of NK cells)

(1) 585A1 iPS cells, (2) clone #3-1 which was produced in Experimental Example 13 and in which A*24:02 allele, B*07:02 allele, B*52:01 allele, and C*12:02 allele of 585A1 iPS cells had been knocked out, and (3) cells (not subcloned bulk cells) produced in Experimental Example 46 to be described later and in which the B2M allele of 585A1 iPS cells had been knocked out, were prepared. Table 46 shows HLA haplotypes of 585A1 iPS cells, and Table 47 shows HLA haplotypes of 1383D2 iPS cells.

[Table 46]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

[Table 47]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

In addition, CD56-positive cells (NK cells) were recovered from PBMC having an HLA haplotype shown in Table 48 (type "LP_275," CTL) by sorting. Subsequently, the recovered NK cells were co-cultured with each of cells of (1) to (3) in the presence of the anti-CD107a antibody. As a result, when the NK cells were activated, they became CD107a-positive. After co-culture, flow cytometric analysis was performed to measure a percentage of activated NK cells.

[Table 48]

| HLA haplotype of PBMC (LP_275, CTL) | | | | | |
|---|---|---|---|---|---|
| HLA-A | | HLA-B | | HLA-C | |
| A*02:06 | A*33:03 | B*40:01 | B*58:01 | C*03:02 | C*07:02 |

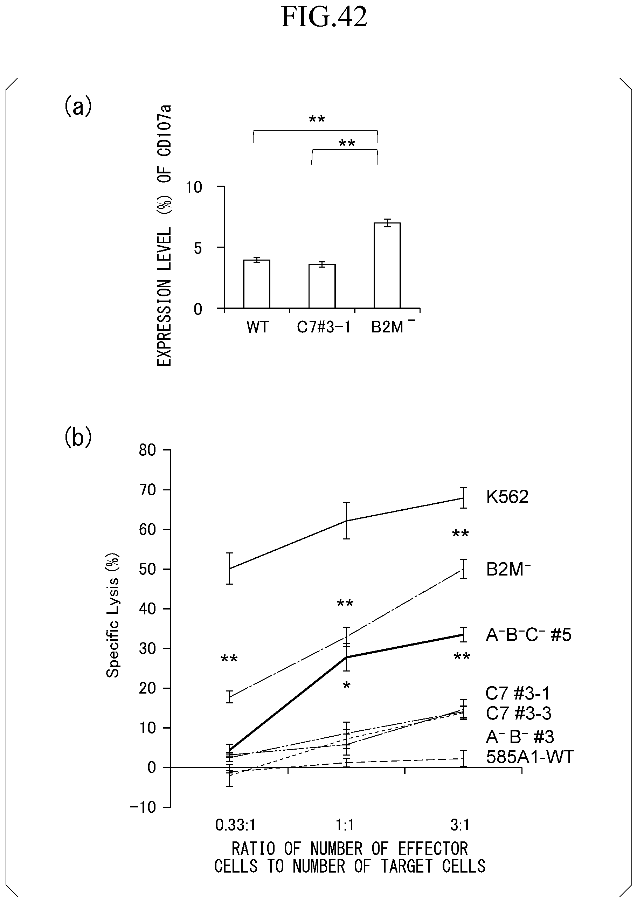

Fig. 42(a) is a graph showing results of flow cytometric analysis. In Fig. 42(a), the symbol "**" indicates that there is a significant difference at $P < 0.01$, "WT" indicates the results of the 585A1 iPS cells of (1), "C7#3-1" indicates the results of the clone #3-1 of (2), and "B2M⁻" indicates the results of the cells in which the B2M allele of the 585A1 iPS cells of (3) had been knocked out.

As a result, it was revealed that the 585A1 iPS cells and the clone #3-1 were able to significantly inhibit activation of NK cells, as compared to cells in which the B2M allele was knocked out. Then, NK cells activated with cells in which the B2M allele was knocked out were sorted and recovered.

Subsequently, for (4) K562 cells known not to express HLA antigen; (5) cells which were produced in Experimental Example 46 to be described later and in which B2M allele of 585A1 iPS cells was knocked out (not subcloned bulk cells); (6) cells which were produced in Experimental Example 47 to be described later and in which the HLA-A allele, HLA-B allele, and HLA-C allele of 585A1 iPS cells were knocked out (where clones #3-5, HLA-E, HLA-F, and HLA-G alleles remained); (7) the clone # 3-1 which was produced in Experimental Example 13 and in which the A*24:02 allele, B*07:02 allele, B*52:01 allele, and C*12:02 allele of 585A1 iPS cells were knocked out; (8) the same clone #3-3; (9) cells which were produced in Experimental Example 13 and in which the HLA-A and HLA-B alleles of the 585A1 iPS cells were knocked out (where clone #3, HLA-C, HLA-E, HLA-F, and HLA-G alleles remained); and (10) 585A1 iPS cells, activated NK cells were added to each of cells at various ratios, and a $^{51}$Cr release assay was performed.

That is, the above-mentioned activated NK cells were used as effector cells, and the above-mentioned cells of (4) to (10) were used as target cells. A percentage of the number of NK cells to be added was 0.33, 1, and 3 with respect to the target cells 1.

Fig. 42(b) is a graph showing a ratio of the cells of (4) to (10) described above that were damaged and lysed. In Fig. 42(b), the symbol "*" indicates that there is a significant difference at $P < 0.05$, the symbol "**" indicates that there is a significant difference at $P < 0.01$, "K562" indicates the results of the K562 cells of (4), "B2M⁻" indicates the results of cells in which the B2M allele of the 585A1 iPS cell of (5) was knocked out, "A⁻B⁻C⁻#5" indicates the results of the cells (clone #3-5) in which the HLA-A allele, HLA-B allele, and HLA-C allele of (6) were knocked out, "C7#3-1" indicates the results of the clone #3-1 of (7), "C7#3-3" indicates the results of the clone #3-3 of (8), "A⁻B⁻#3" indicates the results of the cells (clone #3) in which the HLA-A allele and the HLA-B allele of (9) were knocked out, and "585A1-WT" indicates the results of the 585A1 iPS cells of (10).

As a result, it was revealed that the cells in which the HLA-C allele remained could significantly avoided attack by NK cells, as compared to cells in which B2M was knocked out, and cells in which the HLA-A allele, HLA-B allele, and HLA-C alleles were knocked out.

[Experimental Example 35]

(Reactivity test 3 of NK cells)

(1) 585A1 iPS cells, (2) clone #3-1 which was produced in Experimental Example 13 and in which A*24:02 allele, B*07:02 allele, B*52:01 allele, and C*12:02 allele of 585A1 iPS cells had been knocked out, and (3) cells (not subcloned bulk cells) produced in Experimental Example 46 to be described later and in which the B2M allele of 585A1 iPS cells had been knocked out, were prepared. Table 49 shows HLA haplotypes of 585A1 iPS cells, and Table 50 shows HLA haplotypes of 1383D2 iPS cells.

[Table 49]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

[Table 50]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

In addition, CD3-negative and CD56-positive cells (NK cells) were recovered from PBMC having an HLA haplotype shown in Table 51 (type "LP _329," CTL) by sorting. Subsequently, the recovered NK cells were co-cultured with each of cells of (1) to (3) in the presence of the anti-CD107a antibody. As a result, when the NK cells were activated, they became CD107a-positive. After co-culture, flow cytometric analysis was performed to measure a percentage of activated NK cells.

[Table 51]

| HLA haplotype of PBMC (LP_329, CTL) | | | | | |
|---|---|---|---|---|---|
| HLA-A | | HLA-B | | HLA-C | |
| A*29:02 | A*68:01 | B*39:06 | B*40:02 | C*03:05 | C*07:02 |

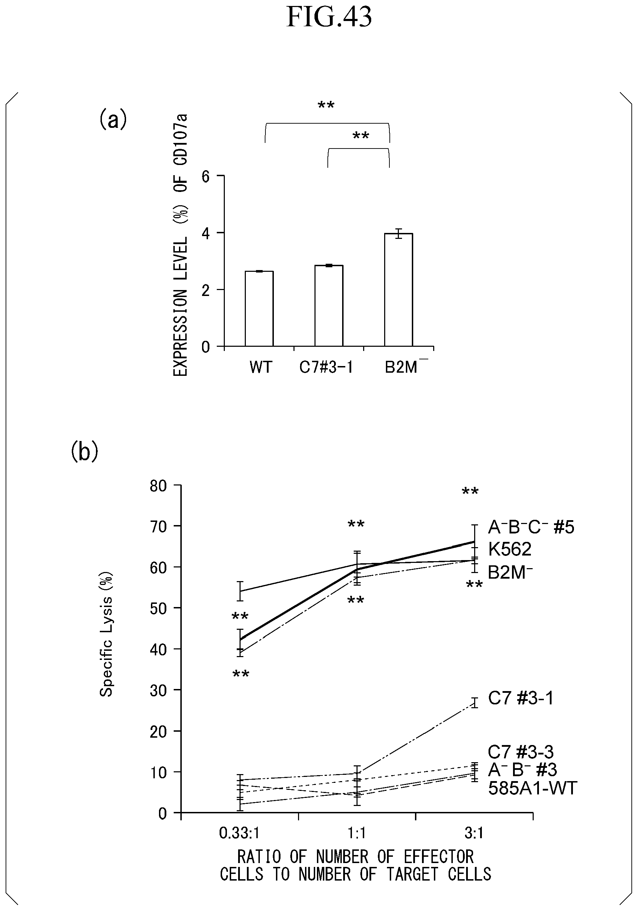

Fig. 43(a) is a graph showing results of flow cytometric analysis. In Fig. 43(a), the symbol "**" indicates that there is a significant difference at P < 0.01, "WT" indicates the results of the 585A1 iPS cells of (1), "C7#3-1" indicates the results of the clone #3-1 of (2), and "B2M⁻" indicates the results of the cells in which the B2M allele of the 585A1 iPS cells of (3) had been knocked out.

As a result, it was revealed that the 585A1 iPS cells and the clone #3-1 were able to significantly inhibit activation of NK cells, as compared to cells in which the B2M allele was knocked out. Then, NK cells activated with cells in which the B2M allele was knocked out were sorted and recovered.

Subsequently, for (4) K562 cells known not to express HLA antigen; (5) cells which were produced in Experimental Example 46 to be described later and in which B2M allele of 585A1 iPS cells was knocked out (not subcloned bulk cells); (6) cells which were produced in Experimental Example 47 to be described later and in which the HLA-A allele, HLA-B allele, and HLA-C allele of 585A1 iPS cells were knocked out (where clones #3-5, HLA-E, HLA-F, and HLA-G alleles remained); (7) the clone # 3-1 which was produced in Experimental Example 13 and in which the A*24:02 allele, B*07:02 allele, B*52:01 allele, and C*12:02 allele of 585A1 iPS cells were knocked out; (8) the same clone #3-3; (9) cells which were produced in Experimental Example 13 and in which the HLA-A and HLA-B alleles of the 585A1 iPS cells were knocked out (where clone #3, HLA-C, HLA-E, HLA-F, and HLA-G alleles remained); and (10) 585A1 iPS cells, activated NK cells were added to each of cells at various ratios, and a $^{51}$Cr release assay was performed.

That is, the above-mentioned activated NK cells were used as effector cells, and the above-mentioned cells of (4) to (10) were used as target cells. A percentage of the number of NK cells to be added was 0.33, 1, and 3 with respect to the target cells 1.

Fig. 43(b) is a graph showing a ratio of the cells of (4) to (10) described above that were damaged and lysed.

In Fig. 43(b), the symbol "**" indicates that there is a significant difference at P < 0.01, "K562" indicates the results of the K562 cells of (4), "B2M⁻" indicates the results of cells in which the B2M allele of the 585A1 iPS cell of (5) was knocked out, "A⁻B⁻C⁻#5" indicates the results of the cells (clone #5) in which the HLA-A allele, HLA-B allele, and HLA-C allele of (6) were knocked out, "C7#3-1" indicates the results of the clone #3-1 of (7), "C7#3-3" indicates the results of the clone #3-3 of (8), "A⁻B⁻#3" indicates the results of the cells (clone #3) in which the HLA-A allele and the HLA-B allele of (9) were knocked out, and "585A1-WT" indicates the results of the 585A1 iPS cells of (10).

As a result, it was revealed that the cells in which the HLA-C allele remained could significantly avoided attack by NK cells, as compared to cells in which B2M was knocked out, and cells in which the HLA-A allele, HLA-B allele, and HLA-C alleles were knocked out.

[Experimental Example 36]

(Reactivity test 4 of NK cells)

(1) 585A1 iPS cells, (2) clone #3-1 which was produced in Experimental Example 13 and in which A*24:02 allele, B*07:02 allele, B*52:01 allele, and C*12:02 allele of 585A1 iPS cells had been knocked out, (3) cells (not subcloned bulk cells) produced in Experimental Example 46 to be described later and in which the B2M allele of 585A1 iPS cells had been knocked out, and (4) K562 cells known not to express HLA antigen, were prepared. Table 52 shows HLA haplotypes of 585A1 iPS cells, and Table 53 shows HLA haplotypes of 1383D2 iPS cells.

[Table 52]

| HLA haplotype of 585A1 iPS cell | | | | | |
|---|---|---|---|---|---|
| HLA-A | | HLA-B | | HLA-C | |
| A*24:02 | A*24:02 | B*07:02 | B*52:01 | C*07:02 | C*12:02 |

[Table 53]

| HLAhaplotype of 1383D2 iPS cell | | | | | |
|---|---|---|---|---|---|
| HLA-A | | HLA-B | | HLA-C | |
| A*02:07 | A*32:01 | B*15:02 | B*51:01 | C*08:01 | C*14:02 |

In addition, CD3-negative and CD56-positive cells (NK cells) were respectively recovered from PBMC having HLA haplotypes shown in Table 54 (type "LP_118," CTL) and PBMC having HLA haplotypes shown in Table 55 (type "LP 266," CTL) by sorting. Subsequently, the recovered NK cells were co-cultured with each of cells of (1) to (4) in the presence of the anti-CD107a antibody. As a result, when the NK cells were activated, they became CD107a-positive. After co-culture, flow cytometric analysis was performed to measure a percentage of activated NK cells.

[Table 54]

| HLA haplotype of PBMC (LP_118, CTL) | | | | | |
|---|---|---|---|---|---|
| HLA-A | | HLA-B | | HLA-C | |
| A*03:01 | A*24:02 | B*07:02 | B*35:01 | C*07:02 | C*07:02 |

[Table 55]

| HLA haplotype of PBMC (LP 266, CTL) | | | | | |
|---|---|---|---|---|---|
| HLA-A | | HLA-B | | HLA-C | |
| A*03:01 | A*24:02 | B*07:02 | B*40:02 | C*03:05 | C*07:02 |

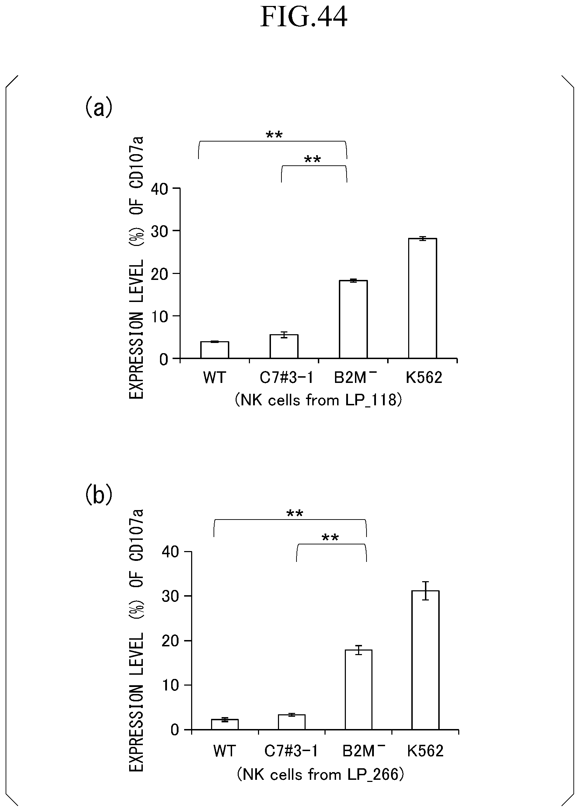

Fig. 44(a) and Fig. 44(b) are graphs showing results of flow cytometric analysis. Fig. 44(a) shows the results of NK cells derived from PBMC having HLA haplotypes shown in Table 54 (type "LP_118," CTL), and Fig. 44(b) shows the results of NK cells derived from PBMC having HLA haplotypes shown in Table 55 (type "LP 266," CTL). In Figs. 44(a) and 44(b), the symbol "**" indicates that there is a significant difference at P < 0.01, "WT" indicates the results of the 585A1 iPS cells of (1), "C7#3-1" indicates the results of the clone #3-1 of (2), "B2M⁻" indicates the results of the cells in which the B2M allele of the 585A1 iPS cells of (3) had been knocked out, and "K562" indicates the results of the K562 cells of (4).

As a result, it was revealed that the 585A1 iPS cells and the clone #3-1 were able to significantly inhibit activation of NK cells, as compared to cells in which the B2M allele was knocked out.

[Experimental Example 37]

(Cell transplantation experiment 2)

(1) Cells in which a B2M gene of 585A1 iPS cell was knocked out, and (2) the clone #3-1 which was produced in Experimental Example 13 and in which the A*24:02 allele, B*07:02 allele, B*52:01 allele, and C*12:02 allele of the 585A1 iPS cells were knocked out, were respectively differentiation-induced into EB-derived blood-cell-like cells. Subsequently, the respective EB-derived blood-cell-like cells were labeled with luciferase.

Subsequently, one hour before the start of the experiment, each of the cell was transplanted into immunodeficient mice (NOD.Cg-Rag1$^{tm1Mom}$Il2rg$^{tm1Wj1}$/SzJ (NRG), The Jackson Laboratory) by intraperitoneal administration. Subsequently, at the start of the experiment, activated NK cells which were prepared in the same manner as in Experimental Examples 34 and 35 and were derived from peripheral blood mononuclear cells (PBMC, type "LP _329," CTL) or a medium (control) were further intraperitoneally administered. Table 56 shows HLA haplotypes of PBMC (type "LP _329," CTL).

[Table 56]

| HLA haplotype of PBMC (LP _329, CTL) | | | | | |
|---|---|---|---|---|---|
| HLA-A | | HLA-B | | HLA-C | |
| A*29:02 | A*68:01 | B*39:06 | B*40:02 | C*03:05 | C*07:02 |

Then, at the time of cell transplantation (before NK cells injection), 5 hours, 1 day, 3 days, 5 days, and 7 days after the start of the experiment, luciferin, which is a substrate of luciferase, was administered to each mouse. Using an IVIS imaging system (trade name "IVIS Spectrum," SPI), fluorescence of the EB-derived blood-cell-like cells labeled with luciferase was captured, and a survival rate of the transplanted cells was measured.

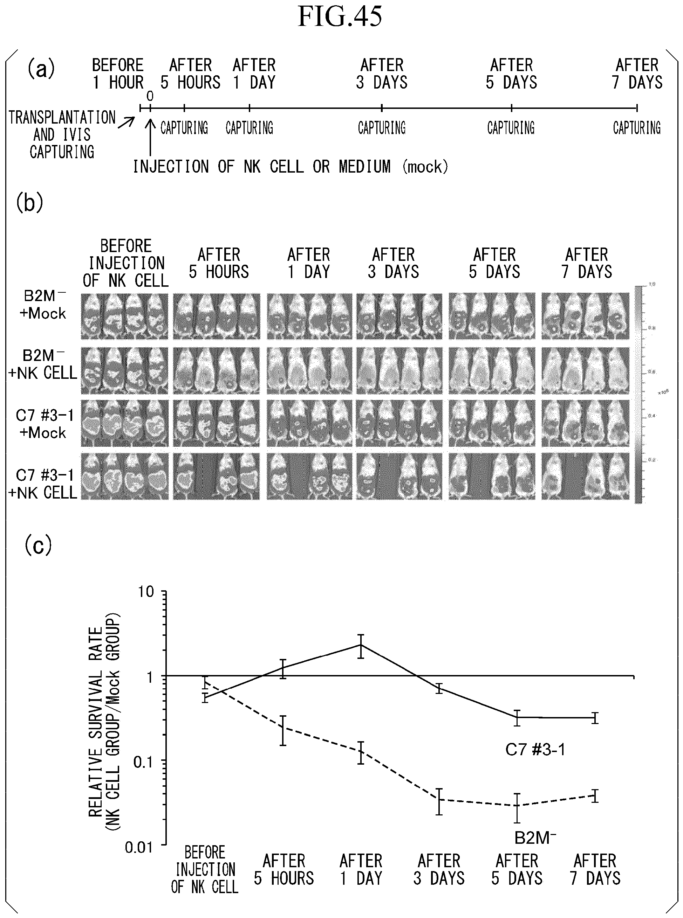

Fig. 45(a) is a diagram illustrating an experimental schedule. Furthermore, Fig. 45(b) shows a photograph showing results of capturing fluorescence of EB-derived blood-cell-like cells in each mouse. Furthermore, Fig. 45(c) is graph showing the results of calculating a survival rate of each EB-derived blood-cell-like cell in a case where the NK cells were administered to the mouse, with a fluorescence intensity before NK cell injection being 100%, and furthermore, calculating a relative survival rate at each measurement time by Formula (F1). In Fig. 45(c), a unit of a vertical axis is (%).

$$\text{Relative survival rate (\%)} = \text{survival rate (\%) of NK cell administration group/survival rate (\%) of control group} \times 100 \quad (F1)$$

In Figs. 45(b) and 45(c), "B2M⁻" indicates the results of EB-derived blood-cell-like cells differentiation-induced from cells in which a B2M gene of the 585A1 iPS cells of (1) was knocked out, "C7 #3-1" indicates the results of EB-derived blood-cell-like cells differentiation-induced from the clone #3-1 of (2), "Mock" indicates the results of administration of the medium as a control, and "NK cell" indicates the results of administration of the activated NK cells derived from peripheral blood mononuclear cells (PBMC, type "LP _329," CTL).

As a result, as shown in Fig. 45(c), it was confirmed that the EB-derived blood-cell-like cells differentiation-induced from the cells in which a B2M gene was knocked out had a reduced survival rate after administration of the NK cells. On the other hand, it was recognized that the EB-derived blood-cell-like cells differentiation-induced from the clone #3-1 tended to maintain a higher survival rate even after administration of activated NK cells.

These results further support that by partially leaving the HLA allele, it is possible to reduce the attack by NK cells in the case of allogeneic transplantation.

[Experimental Example 38]

(Cytotoxicity test using T cells and NK cells)

(1) 585A1 iPS cells, (2) clone #3-1 which was produced in Experimental Example 13 and in which A*24:02 allele, B*07:02 allele, B*52:01 allele, and C*12:02 allele of 585A1 iPS cells had been knocked out, and (3) cells (not subcloned bulk cells) produced in Experimental Example 46 to be described later and in which the B2M allele of 585A1 iPS cells had been knocked out, were respectively differentiation-induced into EB-derived blood-cell-like cells.

Subsequently, in a medium for each of the EB-derived blood-cell-like cells and at various proportions, effector CD8-positive T cells recovered by sorting from peripheral blood mononuclear cells (PBMC, type "LP _275," CTL) were added in the same manner as in Experimental Example 31, and activated NK cells recovered by sorting from PBMC (type "LP _275," CTL) were added in the same manner as in Experimental Example 34, and a $^{51}$Cr release assay was performed.

That is, target cells were EB-derived blood-cell-like cells that had been differentiation-induced from the cells of (1) to (3), and the effector cells were the above-mentioned CD8-positive T cells and activated NK cells. A ratio of the number of CD8-positive T cells to be added as effectors was 1, 3, and 9 with respect to EB-derived blood-cell-like cells 1. In addition, a ratio of the number of activated NK cells to be added was 0.33, 1, and 3 with respect to EB-derived blood-cell-like cells 1.

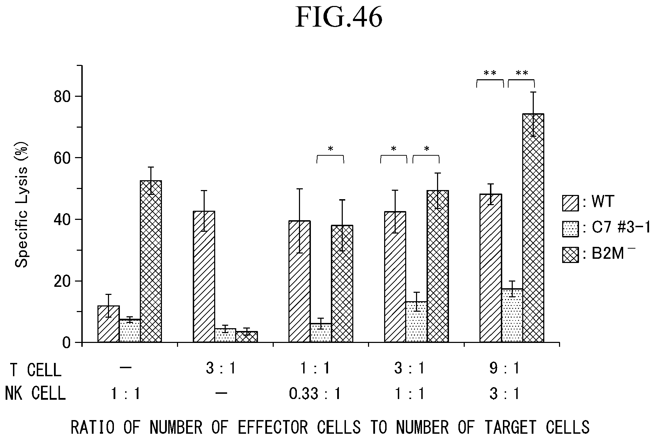

Fig. 46 is a graph showing a ratio of each EB-derived blood-cell-like cell damaged and lysed. In Fig. 46, the symbol "*" indicates that there is a significant difference at $P < 0.05$, the symbol "**" indicates that there is a significant difference at $P < 0.01$, "WT" indicates the results of EB-derived blood-cell-like cells differentiation-induced from the 585A1 iPS cells of (1), "C7#3-1" indicates the results of the EB-derived blood-cell-like cells differentiation-induced from the clone #3-1 of (2), and "B2M⁻" indicates the results of the EB-derived blood-cell-like cells differentiation-induced from cells in which the B2M allele of the 585A1 iPS cells of (3) had been knocked out.

As a result, it was confirmed that when EB-derived blood-cell-like cells were mixed alone with the effector CD8-positive T cells, only EB-derived blood-cell-like cells that were differentiation-induced from 585A1 iPS cells were damaged.

In addition, it was confirmed that when each of the EB-derived blood-cell-like cells was mixed with the activated NK cell alone, only the EB-derived blood-cell-like cell differentiation-induced from the cells in which the B2M allele was knocked out, was damaged.

In addition, it was revealed that when each of the effector CD8-positive T cells and the activated NK cells are mixed with each of the EB-derived blood-cell-like cells, both EB-derived blood-cell-like cells differentiation-induced from 585A1 iPS cells and EB-derived blood-cell-like cells differentiation-induced from cells in which the B2M allele was knocked out were damaged, but a degree of damage was significantly suppressed in the EB-derived blood-cell-like cells that were differentiation-induced from C7#3-1 cells.

These results indicate that damage caused by both the effector CD8-positive T cells and the activated NK cells can be reduced by disrupting an HLA allele present in donor cells but not present in recipient cells and by leaving HLA alleles matching in the donor cells and the recipient cells.

[Experimental Example 39]

(Production of CIITA gene knockout iPS cell)

A CIITA gene is a gene encoding a Class II Major Histocompatibility Complex Transactivator protein. As described above, expression of the CIITA gene is known to be essential for expression of class II HLA protein.

A CIITA gene of the clone #3-1 (hereinafter, may be referred to as "585A1-C7 residual cells"), which was produced in Experimental Example 13 and in which the A*24:02 allele, B*07:02 allele, B*52:01 allele, and C*12:02 allele of 585A1 iPS cells were knocked out, was knocked out.

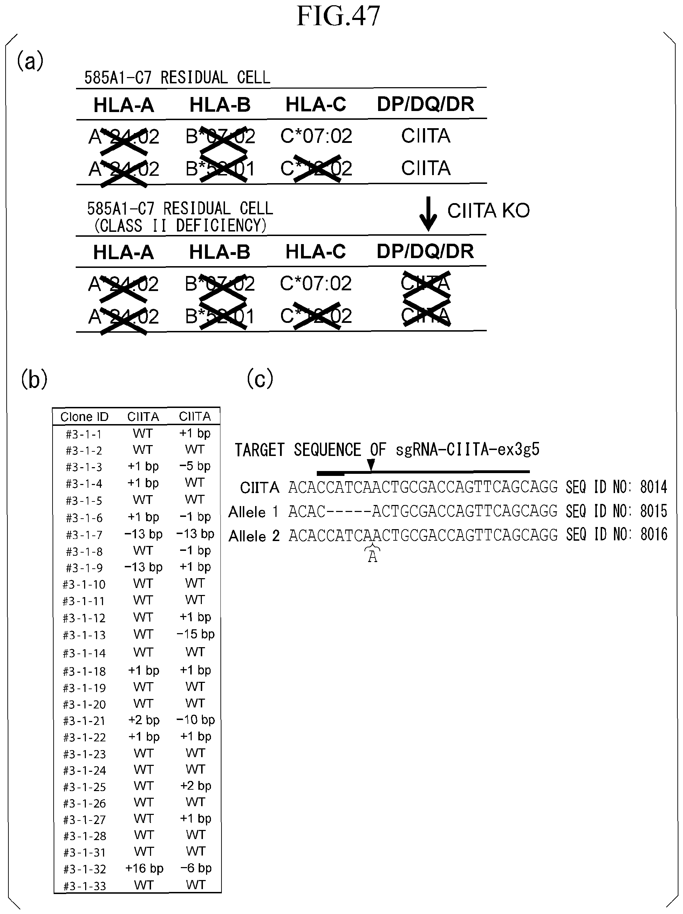

Fig. 47(a) is a table showing 585A1-C7 residual cells and an HLA haplotype of the 585A1-C7 residual cells in which a CIITA gene was knocked out. By knocking out the CIITA gene, class II HLA proteins such as HLA-DP, HLA-DQ, and HLA-DR can be deleted.

Specifically, the 585A1-C7 residual cells were transfected with a purified Cas9 protein together with CIITA-ex3g5 that is an sgRNA that cleaves a CIITA allele (where a target base sequence is set forth in SEQ ID NO: 8017), and subclones of 28 strains were obtained. A commercially available kit (trade name "4D-Nucleofector," model "V4XP-4032," Lonza) was used for transfection.

Then, a base sequence of the CIITA allele of the genome of each subclone was analyzed. Fig. 47(b) is a table showing a base mutation pattern of each subclone. In Fig. 47(b), "Clone ID" indicates the subclone name, "WT" indicates that it was a wild-type base sequence, "-" had a deletion mutation, and "+" indicates that it had an insertion mutation.

As a result, as shown in Fig. 47(b), it was confirmed that a deletion mutation or an insertion mutation was introduced into at least one of CIITA alleles in 15 strains among subclones of 28 strains. In Fig. 47(c), a base sequence of clone #3-1-3 is shown as a representative example.

[Experimental Example 40]

(Test of CD4-positive T cell activation)

CD4-positive T cells were recovered from peripheral blood mononuclear cells (PBMC) derived from healthy volunteer #21 by sorting, and were stained with CFSE (Cayman Chemical Co.).

Subsequently, the above CD4-positive T cells were mixed and co-cultured for 1 week with (1) 585A1 iPS cells, (2) clone #3-1 which was produced in Experimental Example 13 and in which A*24:02 allele, B*07:02 allele, B*52:01 allele, and C*12:02 allele of 585A1 iPS cells had been knocked out (hereinafter, also referred to as "585A1-C7 residual cells"), and (3) the cells which were produced in Experimental Example 39 and in which a CIITA gene of 585A1-C7 residual cells was knocked out (clone #3-1-3). In addition, for comparison, a group in which the above-mentioned CD4-positive T cells of (4) were cultured alone for 1 week was prepared.

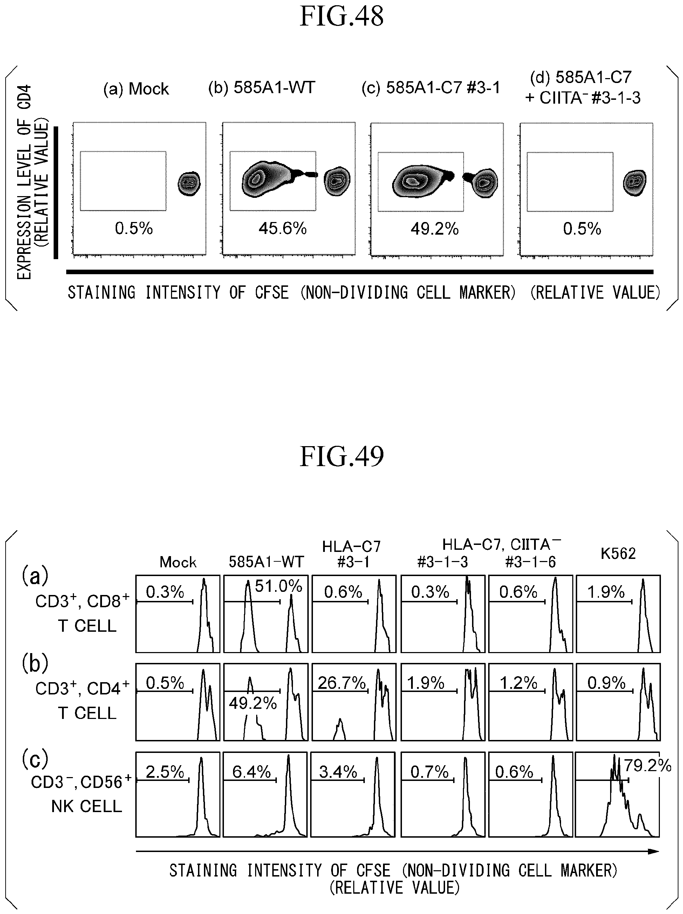

After co-culture, flow cytometric analysis was performed to examine fluorescence of CFSE. Figs. 48(a) to 48(d) are graphs showing results of flow cytometry. In Figs. 48(a) to 48(d), "Mock" indicates the results of culturing only the CD4-positive T cells of (4), "585A1-WT" indicates the results of 585A1 iPS cells of (1), "585A1-C7 #3-1" indicates the results of the 585A1-C7 residual cells of (2), and "585A1-C7 + CIITA #3-1-3" indicates the results of the cells (clone #3-1-3) in which the CIITA gene of the 585A1-C7 residual cells of (3) was knocked out.

As a result, as shown in Fig. 48(a), it was revealed that the CD4-positive T cells were not activated even when only the CD4-positive T cells were cultured alone.

Furthermore, as shown in Fig. 48(b), it became clear that when the CD4-positive T cells derived from healthy volunteer #21 and the 585A1 iPS cells were co-cultured, 45.6% of the CD4-positive T cells were activated and became CFSE-negative.

Furthermore, as shown in Fig. 48(c), it became clear that when the CD4-positive T cells derived from healthy volunteer #21 and the 585A1-C7 residual cells were co-cultured, 49.2% of the CD4-positive T cells were activated and became CFSE-negative.

Furthermore, as shown in Fig. 48(d), it became clear that even when the CD4-positive T cells derived from healthy volunteer #21 and the cells (clone #3-1-3) in which the CIITA gene of 585A1-C7 residual cells was knocked out were co-cultured, the CD4-positive T cells were not activated.

[Experimental Example 41]

(Test of activation of CD4-positive T cells, CD8-positive T cells, and NK cells)

Peripheral blood mononuclear cells (PBMC, type "LP _329," CTL) were stained with CFSE (Cayman Chemical). Table 57 shows HLA haplotypes of PBMC (type "LP _329," CTL).

[Table 57]

| HLA haplotype of PBMC (LP _329, CTL) | | | | | |
|---|---|---|---|---|---|
| HLA-A | | HLA-B | | HLA-C | |
| A*29:02 | A*68:01 | B*39:06 | B*40:02 | C*03:05 | C*07:02 |

Subsequently, the above PBMC was mixed and co-cultured for 1 week with (1) 585A1 iPS cells, (2) clone #3-1 which was produced in Experimental Example 13 and in which A*24:02 allele, B*07:02 allele, B*52:01 allele, and C*12:02 allele of 585A1 iPS cells had been knocked out (hereinafter, also referred to as "585A1-C7 residual cells"), (3) the cells which were produced in Experimental Example 39 and in which a CIITA gene of 585A1-C7 residual cells was knocked out (clone #3-1-3), (4) EB-derived blood-cell-like cells differentiated from each cell of the clone #3-1-6, and (5) K562 cells known not to express HLA antigen. In addition, for comparison, a group in which the above-mentioned PBMC of (6) were cultured alone for 1 week was prepared.

After co-culture, flow cytometric analysis was performed to examine fluorescence of CFSE. Figs. 49(a) to 49(c) are graphs showing results of flow cytometry. In Figs. 49(a) to 49(c), "Mock" indicates the results of culturing only PBMC of (6) alone; "585A1-WT" indicates the results of EB-derived blood-cell-like cells differentiation-induced from the 585A1 iPS cells of (1); "585A1-C7 #3-1" indicates the results of EB-derived blood-cell-like cells differentiation-induced from the 585A1-C7 residual cells of (2); "585A1-C7 + CIITA# 3-1-3" indicates the results of EB-derived blood-cell-like cells

49

differentiation-induced from the cells (clone #3-1-3) in which the CIITA gene of the 585A1-C7 residual cells of (3) was knocked out; "585A1-C7 + CIITA⁻ #3-1-6" indicates the results of EB-derived blood-cell-like cells differentiation-induced from the cells (clone #3-1-6) in which the CIITA gene of the 585A1-C7 residual cells of (4) was knocked out; and "K562" indicates the results of the K562 cells of (5).

As a result, as shown in Fig. 49(a), it became clear that the CD8-positive T cells reacted with the EB-derived blood-cell-like cells differentiation-induced from the 585A1 iPS cells, and 51.0% of the CD8-positive T cells were activated and became CFSE-negative. On the other hand, it was revealed that even when the CD8-positive T cells were co-cultured with cells other than the EB-derived blood-cell-like cells differentiation-induced from 585A1 iPS cells, only 2% or less of the CD8-positive T cells were activated.

Furthermore, as shown in Fig. 49(b), it became clear that the CD4-positive T cells reacted with the EB-derived blood-cell-like cells differentiation-induced from 585A1 iPS cells and the EB-derived blood-cell-like cells differentiation-induced from 585A1-C7 residual cells, and for each of the cases, 49.2% and 26.7% of the CD4-positive T cells were activated and became CFSE-negative. On the other hand, it was revealed that even when the CD4-positive T cells were co-cultured with other cells, only 2% or less of the CD4-positive T cells were activated.

Furthermore, as shown in Fig. 49(c), it was revealed that the NK cells reacted with the K562 cells, and 79.2% of the NK cells were activated and became CFSE-negative. On the other hand, it was revealed that even when the NK cells were co-cultured with other cells, only 7% or less of the NK cells were activated.

[Experimental Example 42]

(Production of iPS cells with residual HLA-C7 and deficient CIITA)

An HLA-A allele, an HLA-B allele, and a CIITA allele were knocked out from iPS stock cells (Ff-Xt-28s05 iPS cells) which has the third most frequent HLA allele among Japanese homozygously. Knockout was performed by one-stage genome editing and two-stage genome editing. Table 58 shows HLA haplotypes of Ff-Xt-28s05 iPS cells.

[Table 58]

| HLAhaplotype of Ff-Xt-28s05 iPS cells | | | | | |
|---|---|---|---|---|---|
| HLA-A | | HLA-B | | HLA-C | |
| A*24:02 | A*24:02 | B*07:02 | B*07:02 | C*07:02 | C*07:02 |

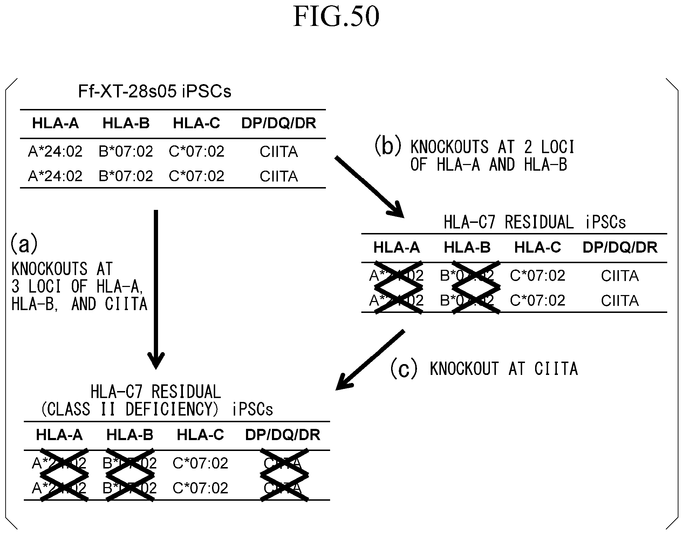

Figs. 50(a) to 50(c) are diagrams illustrating a procedure of the present experimental example. Fig. 50(a) is a diagram illustrating a procedure in which the HLA-A allele, the HLA-B allele, and the CIITA allele were knocked out in one stage. Furthermore, Figs. 50(b) and 50(c) are diagrams illustrating a procedure in which the HLA-A allele, the HLA-B allele, and the CIITA allele were knocked out in two stages.

In the one-stage knockout, as shown in Fig. 50(a), first, Ff-Xt-28s05 iPS cells were transfected with A-ex3-g1 which is an sgRNA capable of targeting the HLA-A allele (where a target base sequence is set forth in SEQ ID NO: 55), B-ex2-g1 which is an sgRNA capable of targeting the HLA-B allele (where a target base sequence is set forth in SEQ ID NO: 53), and CIITA-ex3g5 which is an sgRNA capable of targeting the CIITA allele (where a target base sequence is set forth in SEQ ID NO: 8017), and a purified Cas9 protein. A commercially available kit (trade name "4D-Nucleofector," model "V4XP-4032," Lonza) was used for transfection.

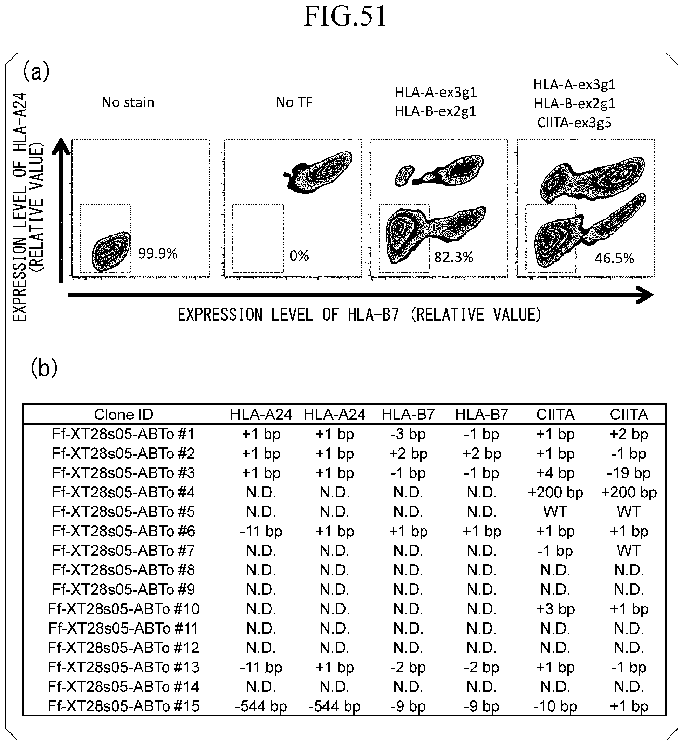

Then, iPS cells were stimulated with 50 ng/mL IFN-γ for 48 hours and subjected to flow cytometric analysis to examine the expression of HLA-A24 antigen and HLA-B7 antigen. Fig. 51(a) is a graph showing results of flow cytometry. In Fig. 51(a), "No stain" indicates the analysis result of iPS cells that were not stained with an antibody, "No TF" indicates the analysis results of iPS cells into which sgRNA was not introduced, "HLA-A-ex3g1" indicates the results of introduction of A-ex3-g1 (where a target base sequence is set forth in SEQ ID NO: 55), "HLA-B-ex2g1" indicates the results of introducing B-ex2-g1 (where a target base sequence is set forth in SEQ ID NO: 53), and CIITA-ex3g5 indicates the results of introducing CIITA-ex3g5 (where a target base sequence is set forth in SEQ ID NO: 8017).

Subsequently, cell populations in which both HLA-A24 antigen and HLA-B7 antigen were negative were sorted and recovered, and subclones of 15 strains were obtained. Then, a base sequence of the HLA-A24 allele, HLA-B7 allele, and CIITA allele of the genome of each subclone was analyzed. Fig. 51(b) is a table showing a base mutation pattern of each subclone. In Fig. 51(b), "Clone ID" indicates the subclone name, "WT" indicates that it was a wild-type base sequence, "-" had a deletion mutation, "+" indicates that it had an insertion mutation, and "N.D." indicates that a base sequence was not determined. Furthermore, "ABTo" in the subclone name means that the HLA-A allele, HLA-B allele, and CIITA allele were knocked out.

As a result, as shown in Fig. 51(b), clones #1, #2, #3, #6, #13, and #15 were obtained in which insertion deletion mutations (Indel mutations) were detected in all of the HLA-A allele, HLA-B allele, and CIITA allele.

In the two-stage knockout, as shown in Fig. 50(b), first, Ff-Xt-28s05 iPS cells were transfected with A-ex3-g1 which is an sgRNA capable of targeting the HLA-A allele (where a target base sequence is set forth in SEQ ID NO: 55), B-ex2-g1 which is an sgRNA capable of targeting the HLA-B allele (where a target base sequence is set forth in SEQ ID NO: 53), and a purified Cas9 protein. Thereby, subclones of 17 strains were obtained. A commercially available kit (trade name "4D-Nucleofector," model "V4XP-4032," Lonza) was used for transfection.

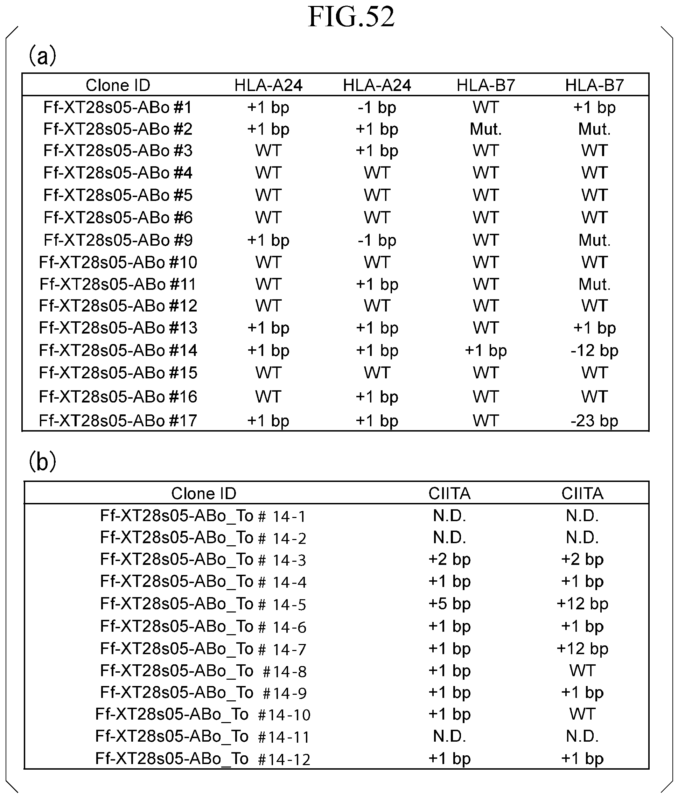

Then, a base sequence of the HLA-A24 allele and HLA-B7 allele of the genome of each subclone was analyzed. Fig. 52(a) is a table showing a base mutation pattern of each subclone. In Fig. 52(a), "Clone ID" indicates the subclone name, "WT" indicates that it was a wild-type base sequence, "-" had a deletion mutation, "+" indicates that it had an insertion mutation, and "Mut." indicates that it had a substitution mutation. Furthermore, "ABo" in the subclone name means that the HLA-A allele and HLA-B allele were knocked out.

As a result, as shown in Fig. 52(a), clone #14 was obtained in which insertion deletion mutations (Indel mutations) were detected in both of the HLA-A allele and the HLA-B allele.

Subsequently, as shown in Fig. 50(c), the obtained clone #14 was transfected with CIITA-ex3g5 (where a target base sequence is set forth in SEQ ID NO: 8017), which is an sgRNA capable of targeting the CIITA allele, and a purified Cas9 protein. Thereby, subclones of 12 strains were obtained. A commercially available kit (trade name "4D-Nucleofector," model "V4XP-4032," Lonza) was used for transfection.

Then, a base sequence of the CIITA allele of the genome of each subclone was analyzed. Fig. 52(b) is a table showing a base mutation pattern of each subclone. In Fig. 52(b), "Clone ID" indicates the subclone name, "WT" indicates that it was a wild-type base sequence, "-" had a deletion mutation, "+" indicates that it had an insertion mutation, and "N.D." indicates that a base sequence was not determined. Furthermore, "ABo To" in the subclone name means that the HLA-A and HLA-B alleles were knocked out, subcloned once, and then the CIITA allele was knocked out.

As a result, as shown in Fig. 52(b), clones #14-3, #14-4, #14-6, and #14-12 were obtained in which insertion deletion mutations (Indel mutations) that were frameshift mutations were detected in both alleles of CIITA.

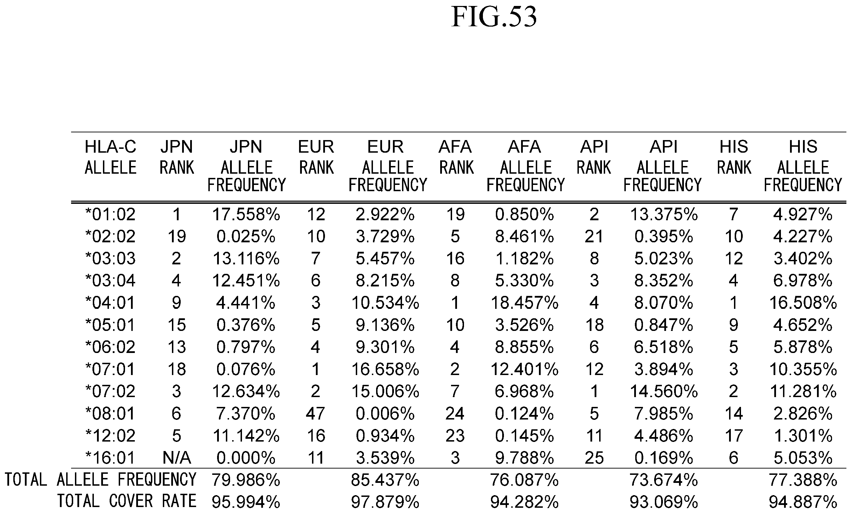

Fig. 53 is a table showing allele frequency of an HLC-C allele in various races. In Fig. 53, "JPN" indicates Japanese, "EUR" indicates European American, "AFA" indicates African American, "API" indicates Asian, and "HIS" indicates Hispanic. In addition, "allele frequency" indicates frequency (%) of an allele in each race, and "rank" indicates the order of frequency from the highest frequency.

As shown in Fig. 53, it is possible to cover 93% or more of the population in any of the above races by producing 12 kinds of cells, which are HLA-C*01:02, HLA-C*02:02, HLA-C*03:03, HLA-C*03:04, HLA-C*04:01, HLA-C*05:01, HLA-C*06:02, HLA-C*07:01, HLA-C*07:02, HLA-C*08:01, HLA-C*12:02, and HLA-C*16:01.

That is, cells deficient in the HLA-A and HLA-B alleles and having any of the above 12 kinds of the HLA-C alleles, or cells deficient in the HLA-A allele, HLA-B allele, and CIITA allele and having any of the above 12 kinds of the HLA-C alleles are very useful as cells for cell therapy and regenerative medicine. The cells are not particularly limited as long as they are cells used for cell therapy, and they may be pluripotent stem cells or differentiated cells. Specifically, for example, iPS cells, T cells, hematopoietic stem cells, mesenchymal stem cells, and the like may be used.

[Experimental Example 43]

(Production 3 of B2M knockout iPS cell)

AB2M allele was knocked out from the iPS stock cells (Ff-Xt-28s05 iPS cells) also used in Experimental Example 41.

Ff-Xt-28s05 iPS cells were transfected with B2M-g2, which is an sgRNA capable of targeting the B2M allele (where a target base sequence is set forth in SEQ ID NO: 60) and a purified Cas9 protein. Thereby, subclones of 5 strains were obtained. A commercially available kit (trade name "4D-Nucleofector," model "V4XP-4032," Lonza) was used for transfection.

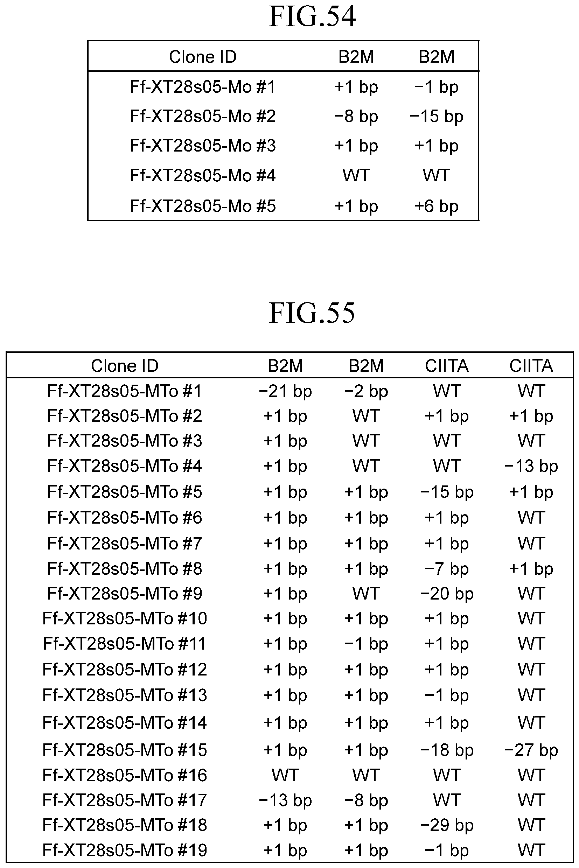

Then, a base sequence of the B2M allele of the genome of each subclone was analyzed. Fig. 54 is a table showing a base mutation pattern of each subclone. In Fig. 54, "Clone ID" indicates the subclone name, "WT" indicates that it was a wild-type base sequence, "-" had a deletion mutation, and "+" indicates that it had an insertion mutation. Furthermore, "Mo" in the subclone name means that the B2M allele was knocked out.

As a result, as shown in Fig. 54, clones #1 and #3 having frameshift mutations in the B2M allele were obtained.

[Experimental Example 44]

(Preparation of B2M and CIITA knockout iPS cells)

AB2M allele and a CIITA allele were knocked out at the same time from the iPS stock cells (Ff-Xt-28s05 iPS cells) also used in Experimental Example 41.

Ff-Xt-28s05 iPS cells were transfected with B2M-g2 which is an sgRNA capable of targeting the B2M allele (where a target base sequence is set forth in SEQ ID NO: 60), CIITA-ex3g5 which is an sgRNA capable of targeting the CIITA allele (where a target base sequence is set forth in SEQ ID NO: 8017), and a purified Cas9 protein. Thereby, subclones of 19 strains were obtained. A commercially available kit (trade name "4D-Nucleofector," model "V4XP-4032," Lonza) was used for transfection.

Then, a base sequence of the B2M and CIITA alleles of the genome of each subclone was analyzed. Fig. 55 is a table showing a base mutation pattern of each subclone. In Fig. 55, "Clone ID" indicates the subclone name, "WT" indicates that it was a wild-type base sequence, "-" had a deletion mutation, and "+" indicates that it had an insertion mutation. Furthermore, "MTo" in the subclone name means that the B2M allele and the CIITA allele were knocked out in one stage.

As a result, as shown in Fig. 55, clone #8 having a frameshift mutation in both the B2M allele and the CIITA allele was obtained.

Furthermore, it was revealed that in clone #2, only one of the B2M alleles was knocked out, and both of the CIITA alleles were knocked out. Accordingly, the clone #2 was obtained as a cell strain in which substantially only the CIITA allele was knocked out.

[Experimental Example 45]

(Examination of HLA protein expression in B2M and CIITA knockout iPS cells)

Expression of HLA-ABC protein in Ff-Xt-28s05-MTo #2 iPS cells produced in Experimental Example 44 was examined.

First, Ff-Xt-28s05-MTo #2 iPS cells were stimulated with 50 ng/mL IFN-γ for 48 hours. Thereafter, the cells were stained with an anti-HLA-ABC antibody (product number:311418, BIOLEGEND) to be subjected to flow cytometric analysis, and expression of HLA-A protein, HLA-B protein, and HLA-C protein was examined.

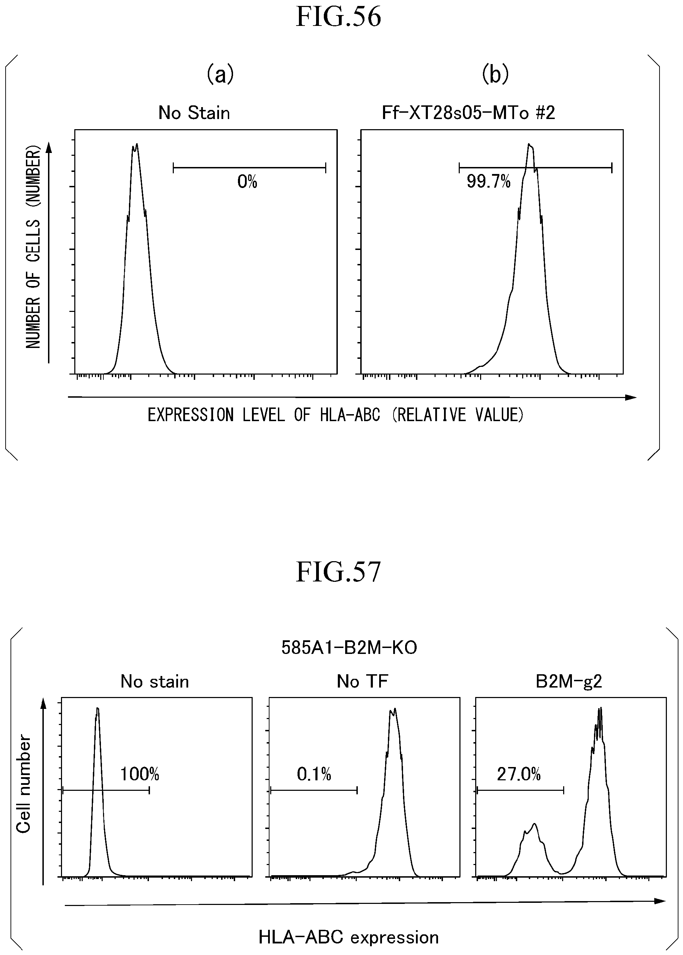

Fig. 56(a) and 56(b) are graphs showing results of flow cytometry. In Fig. 56(a), "No stain" indicates the analysis results of iPS cells not stained with the antibody. Furthermore, in Fig. 16(b), "Ff-Xt-28s05-MTo #2" indicates the analysis results of Ff-Xt-28s05-MTo #2 iPS cells.

As a result, it was revealed that the Ff-Xt-28s05-MTo #2 iPS cells maintained an expression ability of HLA-A protein, HLA-B protein, and HLA-C protein.

[Experimental Example 46]

(Production 4 of B2M knockout iPS cell)

585A1 iPS cells were transfected with a plasmid vector expressing Cas9 and B2M-specific sgRNA. B2M-g2 (where a target base sequence is set forth in SEQ ID NO: 60) was used as sgRNA.

Subsequently, cells stimulated with 50 ng/mL IFN-γ for 48 hours were stained with an anti-HLA-ABC antibody, and flow cytometric analysis was performed.

Fig. 57 is a graph showing results of flow cytometry. In Fig. 57, "No stain" indicates the analysis results of iPS cells that were not stained with an antibody, "No TF" indicates the analysis results of iPS cells into which sgRNA was not introduced, and "B2M-g2" indicates the analysis results of iPS cells into which this sgRNA was introduced and in which the B2M allele was knocked out.

As a result, a proportion of the cell population in which all of HLA-A protein, HLA-B protein, and HLA-C protein were negative was 27.0%. Subsequently, a cell population which was obtained by the introduction of B2M-g2 sgRNA and in which all of HLA-A, HLA-B, and HLA-C were negative was recovered by sorting. No subcloning was performed herein.

[Experimental Example 47]

(HLA-allele-specific knockout 10)

HLA-A, HLA-B, and HLA-C alleles of 585A1 iPS cells were knocked out to produce cells. Specifically, a purified Cas9 protein, an sgRNA specific to C*07:02 allele, and an sgRNA specific to C*12:02 allele were transfected to the cells (clone # 3) which were produced in Experimental Example 13 and in which the A*24:02 allele, B*07:02 allele, and B*52:01 allele of the 585A1 iPS cells were knocked out.

C0702-ex3-g3 (where a target base sequence is set forth in SEQ ID NO: 49) was used as the sgRNA specific to the C*07:02 allele. In addition, C1202-ex3-g1 (where a target base sequence is set forth in SEQ ID NO: 50) was used as the sgRNA specific to the C*12:02 allele. A commercially available kit (trade name "4D-Nucleofector," model "V4XP-4032," Lonza) was used for transfection.

Thereafter, subcloning was performed to obtain subclones of six strains. Subsequently, genomic DNA was extracted from each subclone. Subsequently, base sequences of the HLA-C*07:02 allele and the HLA-C*12:02 allele were analyzed by Sanger sequence.

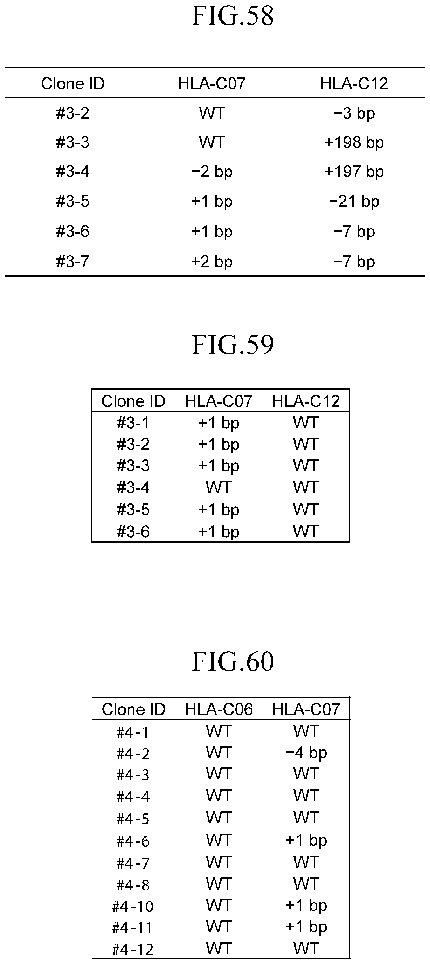

Fig. 58 is a table showing a base mutation pattern of each clone recovered. In Fig. 58, "Clone ID" indicates the subclone name, "HLA-C07" indicates the HLA-C*07:02 allele, "HLA-C12" indicates the HLA-C*12:02 allele, "WT" indicates that it was a wild-type base sequence, "-" had a deletion mutation, and "+" indicates that it had an insertion mutation. As a result, it was confirmed that both HLA-C alleles were knocked out in clones #3-4, #3-5, #3-6, and #3-7.

[Experimental Example 48]

(HLA-allele-specific knockout 11)

A purified Cas9 protein and an sgRNA specific to C*07:02 allele were transfected to the cells (clone # 3) which were produced in Experimental Example 13 and in which the A*24:02 allele, B*07:02 allele, and B*52:01 allele of the 585A1 iPS cells were knocked out.

C0702-ex3-g3 (where a target base sequence is set forth in SEQ ID NO: 49) was used as the sgRNA specific to the C*07:02 allele. A commercially available kit (trade name "4D-Nucleofector," model "V4XP-4032," Lonza) was used for transfection.

Thereafter, subcloning was performed to obtain subclones of six strains. Subsequently, genomic DNA was extracted from each subclone. Subsequently, base sequences of the HLA-C*07:02 allele and the HLA-C*12:02 allele were analyzed by Sanger sequence.

Fig. 59 is a table showing a base mutation pattern of each clone recovered. In Fig. 59, "Clone ID" indicates the subclone name, "HLA-C07" indicates the HLA-C*07:02 allele, "HLA-C12" indicates the HLA-C*12:02 allele, "WT" indicates that it was a wild-type base sequence, and "+" indicates that it had an insertion mutation. As a result, it was confirmed that the HLA-C*07:02 allele was knocked out and only the HLA-C*12:02 allele was left in the clones #3-1, #3-2, #3-3, #3-5, and #3-6.

As described above with reference to Fig. 53, by preparing 12 kinds of cells, which are HLA-C*01:02, HLA-C*02:02, HLA-C*03:03, HLA-C*03:04, HLA-C*04:01, HLA-C*05:01, HLA-C*06:02, HLA-C*07:01, HLA-C*07:02, HLA-C*08:01, HLA-C*12:02, and HLA-C*16:01, it is possible to cover a population of 93% or more in any race of Japanese, European American, African American, Asian, and Hispanic.

The cells produced in the present experimental example can be used as one of these 12 kinds of cells.

[Experimental Example 49]

(HLA-allele-specific knockout 12)

A purified Cas9 protein and an sgRNA specific to C*07:02 allele were transfected to the cells (clone # 4) which were produced in Experimental Example 14 and in which the A*01:01 allele, A*24:02 allele, B*07:02 allele, and B*37:01 allele of the 604B1 iPS cells were knocked out.

C0702-ex3-g3 (where a target base sequence is set forth in SEQ ID NO: 49) was used as the sgRNA specific to the C*07:02 allele. A commercially available kit (trade name "4D-Nucleofector," model "V4XP-4032," Lonza) was used for transfection.

Thereafter, subcloning was performed to obtain subclones of 11 strains. Subsequently, genomic DNA was extracted from each subclone. Subsequently, base sequences of the HLA-C*06:02 allele and the HLA-C*12:02 allele were analyzed by Sanger sequence.

Fig. 60 is a table showing a base mutation pattern of each clone recovered. In Fig. 60, "Clone ID" indicates the subclone name, "HLA-C06" indicates the HLA-C*06:02 allele, "HLA-C07" indicates the HLA-C*07:02 allele, "WT" indicates that it was a wild-type base sequence, "-" had an insertion mutation, and "+" indicates that it had an insertion mutation. As a result, it was confirmed that the HLA-C*07:02 allele was knocked out and only the HLA-C*06:02 allele was left in the clones #4-2, #4-6, #4-10, and #4-11.

As described above with reference to Fig. 53, by preparing 12 kinds of cells, which are HLA-C*01:02, HLA-C*02:02, HLA-C*03:03, HLA-C*03:04, HLA-C*04:01, HLA-C*05:01, HLA-C*06:02, HLA-C*07:01, HLA-C*07:02, HLA-C*08:01, HLA-C*12:02, and HLA-C*16:01, it is possible to cover a population of 93% or more in any race of Japanese, European American, African American, Asian, and Hispanic.

The cells produced in the present experimental example can be used as one of these 12 kinds of cells.

Industrial Applicability

According to the present invention, it is possible to provide a technique for producing a low-antigenic cell in which a rejection reaction is reduced in a case where the cell is allogeneically transplanted into a recipient.

**Claims**

1. A method for producing, from a donor cell, a low-antigenic cell in which a rejection reaction is reduced in a case where the cell is allogeneically transplanted into a recipient, the method comprising:

   determining human leukocyte antigen (HLA) alleles for the donor cell and the recipient, respectively;
   specifying an HLA allele that is present in the donor cell but is not present in the recipient; and
   disrupting or modifying the specified HLA allele to obtain a cell population including a cell not expressing an HLA protein specific to the donor cell,
   wherein the cell not expressing the HLA protein specific to the donor cell is the low-antigenic cell.

2. The production method according to claim 1, wherein the HLA allele determined in the determining the HLA alleles for the donor cell and the recipient, respectively, includes an HLA-A allele, an HLA-B allele, and an HLA-C allele.

3. The production method according to claim 1 or 2, further comprising:

   recovering the cell not expressing the HLA protein specific to the donor cell from the cell population after the obtaining of the cell population including the cell not expressing the HLA protein specific to the donor cell,
   wherein the recovering includes

   bringing the cell population into contact with an HLA protein expression-inducing agent, and
   recovering the cell not expressing the HLA protein specific to the donor cell from the cell population using, as an index, expression of the HLA protein specific to the donor cell in the cell population brought into contact with the HLA protein expression-inducing agent.

4. The production method according to claim 3, wherein the HLA protein expression-inducing agent is interferon (IFN)-$\gamma$, IFN-$\alpha$, IFN-$\beta$, interleukin (IL)-4, granulocyte macrophage colony-stimulating factor (GM-CSF), transforming growth factor (TGF)-$\alpha$, or TGF-$\beta$.

5. The production method according to any one of claims 1 to 4, wherein the low-antigenic cell is a pluripotent stem cell.

6. A kit for detecting a low-antigenic cell in which a rejection reaction is reduced in a case where the cell is allogeneically transplanted into a recipient, the kit comprising an HLA protein expression-inducing agent.

7. The kit according to claim 6, wherein the HLA protein expression-inducing agent is IFN-$\gamma$, IFN-$\alpha$, IFN-$\beta$, IL-4, GM-CSF, TGF-$\alpha$, or TGF-$\beta$.

8. The kit according to claim 6 or 7, wherein the low-antigenic cell is a pluripotent stem cell.

9. An HLA protein expression-inducing agent consisting of IFN-$\gamma$, IFN-$\alpha$, IFN-$\beta$, IL-4, GM-CSF, TGF-$\alpha$, or TGF-$\beta$.

10. A cell in which at least one HLA allele is disrupted or modified and at least one kind of HLA protein can be expressed.

11. The cell according to claim 10, wherein the HLA allele includes an HLA-A allele, an HLA-B allele, or an HLA-C allele.

12. The cell according to claim 10 or 11, which is a low-antigenic cell in which a rejection reaction is reduced in a case where the cell is allogeneically transplanted into a recipient, and in which the disrupted or modified HLA allele is an HLA allele that is not present in the recipient.

13. The cell according to any one of claims 10 to 12, which is a pluripotent stem cell.

14. The cell according to any one of claims 10 to 13, wherein at least one allele of a class II major histocompatibility complex transactivator (CIITA) allele, a regulatory factor X5 (RFX5) allele, a regulatory factor X associated protein (RFXAP) allele, or a regulatory factor X associated ankyrin containing protein (RFXANK) allele is further disrupted or modified.

15. The cell according to any one of claims 10 to 14, in which the HLA-A allele and the HLA-B allele are disrupted and at least one kind of HLA-C protein can be expressed.

16. The cell according to claim 15, wherein the HLA-C protein is a protein encoded by one kind of allele selected from the group consisting of an HLA-C*01:02 allele, an HLA-C*02:02 allele, an HLA-C*03:03 allele, an HLA-C*03:04 allele, an HLA-C*04:01 allele, an HLA-C*05:01 allele, an HLA-C*06:02 allele, an HLA-C*07:01 allele, an HLA-C*07:02 allele, an HLA-C*08:01 allele, an HLA-C*12:02 allele, and an HLA-C*16:01 allele.

17. A method for producing, from a donor cell, a low-antigenic cell in which a rejection reaction is reduced in a case where the cell is allogeneically transplanted into a recipient, the method comprising:

disrupting or modifying an allele including at least one of a CIITA allele, an RFX5 allele, an RFXAP allele, and an RFXANK allele of the donor cell,
wherein a cell in which the CIITA allele, the RFX5 allele, the RFXAP allele, or the RFXANK allele is disrupted or modified is the low-antigenic cell.

18. A cell according to claim 17, wherein the low-antigenic cell is a pluripotent stem cell.

19. A cell in which an allele including at least one of a CIITA allele, an RFX5 allele, an RFXAP allele, and an RFXANK allele is disrupted or modified.

20. The cell according to claim 19, which is a pluripotent stem cell.

21. A gRNA targeting, as a target base sequence, a base sequence that is mapped to only one target HLA haplotype in a case where the base sequence is mapped to base sequence data of genomic DNA of all HLA haplotypes, but that is not mapped in a case where the base sequence is mapped to base sequence data of all genomic DNA except for HLA alleles.

22. The gRNA according to claim 21,
wherein the target base sequence consists of

a base sequence set forth in any of SEQ ID NOs: 3, 4, 7, 45 to 52, and 72 to 2459, or
a base sequence in which one or several bases are deleted, substituted, or added at a 5' end of a base sequence set forth in any of SEQ ID NOs: 3, 4, 7, 45 to 52, and 72 to 2459.

23. The gRNA according to claim 21 or 22,
wherein the target base sequence consists of

a base sequence set forth in any of SEQ ID NOs: 3, 4, 7, and 45 to 52, or
a base sequence in which one or several bases are deleted, substituted, or added at a 5' end of a base sequence set forth in any of SEQ ID NOs: 3, 4, 7, and 45 to 52.

24. The gRNA according to any one of claims 21 to 23, wherein the target base sequence is mapped to an exon 2 or 3 of an HLA gene.

25. A gRNA targeting, as a target base sequence, a base sequence that is mapped to two or more target HLA haplotypes in a case where the base sequence is mapped to base sequence data of genomic DNA of all HLA haplotypes, but that is not mapped in a case where the base sequence is mapped to base sequence data of all genomic DNA except for HLA alleles.

26. The gRNA according to claim 25,
wherein the target base sequence consists of

a base sequence set forth in any of SEQ ID NOs: 53 to 55 and 2460 to 8013, or

a base sequence in which one or several bases are deleted, substituted, or added at a 5' end of a base sequence set forth in any of SEQ ID NOs: 53 to 55 and 2460 to 8013.

27. The gRNA according to claim 25 or 26,
wherein the target base sequence consists of

a base sequence set forth in any of SEQ ID NOs: 53 to 55, or
a base sequence in which one or several bases are deleted, substituted, or added at a 5' end of a base sequence set forth in any of SEQ ID NOs: 53 to 55.

28. The gRNA according to any one of claims 25 to 27, wherein the target base sequence is mapped to an exon 2 or 3 of an HLA gene.

29. A method for specifying a target base sequence for producing, by genome editing, a low-antigenic cell in which a rejection reaction is reduced in a case where the cell is allogeneically transplanted into a recipient, the method comprising:

mapping a candidate base sequence to base sequence data of genomic DNA of all HLA haplotypes;
mapping the candidate base sequence to base sequence data of all genomic DNA except for HLA alleles; and
specifying, as a target base sequence, the candidate base sequence that is mapped to only one target HLA haplotype in a case where the base sequence is mapped to base sequence data of genomic DNA of all HLA haplotypes, but that is not mapped in a case where the base sequence is mapped to base sequence data of all genomic DNA except for the HLA alleles.

30. A method for specifying a target base sequence for producing, by genome editing, a low-antigenic cell in which a rejection reaction is reduced in a case where the cell is allogeneically transplanted into a recipient, the method comprising:

mapping a candidate base sequence to base sequence data of genomic DNA of all HLA haplotypes;
mapping the candidate base sequence to base sequence data of all genomic DNA except for HLA alleles; and
specifying, as a target base sequence, the candidate base sequence that is mapped to two or more target HLA haplotypes in a case where the base sequence is mapped to base sequence data of genomic DNA of all HLA haplotypes, but that is not mapped in a case where the base sequence is mapped to base sequence data of all genomic DNA except for the HLA alleles.

FIG.1

(a)

(b)

Unique k-mers

FIG.2

NUMBER OF CELLS (RELATIVE VALUE)

$-10^3$   0   $10^3$   $10^4$   $10^5$

EXPRESSION LEVEL OF HLA-ABC
(RELATIVE VALUE)

: no cytokine

: LPS (100 ng/ml), 48 hr

: TNF-α (100 ng/ml), 48 hr

: IFN-γ (50ng/ml), 48 hr

FIG.3

NUMBER OF CELLS (RELATIVE VALUE)

$-10^3$   0   $10^3$   $10^4$   $10^5$

EXPRESSION LEVEL OF HLA-ABC
(RELATIVE VALUE)

: no cytokine

: IFN-γ (10 ng/ml), 48 hr

: IFN-γ (50 ng/ml), 48 hr

: IFN-γ (100 ng/ml), 48 hr

FIG.4

(a)

NUMBER OF CELLS (RELATIVE VALUE)

: no IFN-γ
: 4 hr on
: 8 hr on
: 16 hr on
: 24 hr on
: 48 hr on

$-10^3$　$0$　$10^3$　$10^4$　$10^5$

EXPRESSION LEVEL OF HLA-A2
(RELATIVE VALUE)

(b)

ANALYSIS

$0$　　　$24$　　$32$　$40$ $44$ $48$ hr

# FIG.5

(a)

| | | |
|---|---|---|
| A*02:07 allele | GTACCACCAGTACGCCTACGACGGCAA | SEQ ID NO: 1 |
| A*32:01 allele | GTACCAGCAGGACGCCTACGACGGCAA | SEQ ID NO: 2 |
| A0207-ex3-g1 ── | TACCACCAGTACGCCTACGAC<u>GG</u> | SEQ ID NO: 3 |
| A0207-ex3-g2 ── | <u>CCA</u>CCAGTACGCCTACGAC<u>GG</u>CA | SEQ ID NO: 4 |
| | | |
| A*02:07 allele | TCACACCGTCCAGAGGATGTGTGGCTG | SEQ ID NO: 5 |
| A*32:01 allele | TCACACCATCCAGATGATGTATGGCTG | SEQ ID NO: 6 |
| A0207-ex3-g4 ── | CACACCGTCCAGAGGATGTG<u>TGG</u> | SEQ ID NO: 7 |

HLA-A

Exon1  Exon2    Exon3         Exon4 Exon5  Exon6 Exon7 Exon8

(b)

| T7E1 | − + | − + | − + | − + | − + | − + |
|---|---|---|---|---|---|---|
| sgRNA | ex3-g1 | ex3-g2 | ex3-g4 | − | DMD#1 | − |
| Indel(%) | 15% | 13% | 28% | | 23% | |

60

# FIG.6

(a)

| No-treatment | IFN-γ treated |
| --- | --- |

604B1　(A1/A24)

1383D2　(A2/A32)

404C2　(A2/A2)

$-10^3$　$0$　$10^3$　$10^4$　$10^5$　$-10^3$　$0$　$10^3$　$10^4$　$10^5$

EXPRESSION LEVEL OF HLA-A2 (RELATIVE VALUE)

(b)

| 1383D2<br>+ DMD sgRNA#1 | 1383D2<br>+ A0207-ex3-g1 | 1383D2<br>+ A0207-ex3-g4 |
| --- | --- | --- |

Not treated

NUMBER OF CELLS (NUMBER)

$0$　$10^2$　$10^3$　$10^4$　　　$0$　$10^2$　$10^3$　$10^4$　　　$0$　$10^2$　$10^3$　$10^4$　$10^5$

EXPRESSION LEVEL OF HLA-A2 (RELATIVE VALUE)

IFN-γ treated

NUMBER OF CELLS (NUMBER)

0.2%　　　9.1%　　　62%

$0$　$10^2$　$10^3$　$10^4$　　　$0$　$10^2$　$10^3$　$10^4$　　　$0$　$10^2$　$10^3$　$10^4$　$10^5$

EXPRESSION LEVEL OF HLA-A2 (RELATIVE VALUE)

## FIG.7

# FIG.8

(a)

No transfection    A0101-ex3-g1    A0101-ex3-g2

NUMBER OF CELLS (NUMBER)

0.03%    11.2%    18.9%

EXPRESSION LEVEL OF HLA-A1 (RELATIVE VALUE)

(b)

No transfection    B0702-ex2-g1    B0702-ex2-g2

NUMBER OF CELLS (NUMBER)

0.08%    11.6%    0.70%

EXPRESSION LEVEL OF HLA-B7 (RELATIVE VALUE)

# FIG.9

(a)

EXPRESSION LEVEL OF HLA-B7 (RELATIVE VALUE)

No transfection

0.2%

EXPRESSION LEVEL OF HLA-A1 (RELATIVE VALUE)

(b)

EXPRESSION LEVEL OF HLA-B7 (RELATIVE VALUE)

A0101-ex3-g2
B0702-ex2-g1

1.0%

EXPRESSION LEVEL OF HLA-A1 (RELATIVE VALUE)

# FIG.10

(a)

| CLONE NO. | HLA-A1 | HLA-B7 |
|-----------|--------|--------|
| #1  | −5 bp  | −6 bp  |
| #2  | −5 bp  | −6 bp  |
| #3  | WT     | WT     |
| #5  | −5 bp  | −6 bp  |
| #6  | −5 bp  | −6 bp  |
| #7  | WT     | WT     |
| #8  | −4 bp  | −2 bp  |
| #10 | −14 bp | −11 bp |
| #11 | +18 bp | −1 bp  |
| #12 | −5 bp  | −6 bp  |
| #13 | −4 bp  | −2 bp  |
| #14 | WT     | WT     |
| #15 | −4 bp  | −2 bp  |

(b)

HLA-A*01:01

```
WT   GGGTACCGGCAGGACGCCT   SEQ ID NO: 8
#8   GGGTACCGG----CCGCCT   SEQ ID NO: 9
#10  GG--------------CCT
```

HLA-B*07:02

```
WT   ACAAGGCCCAGGCACA   SEQ ID NO: 10
#8   ACAA--CCCAGGCACA   SEQ ID NO: 11
#10  ACA----------CA
```

# FIG.11

No stain   No transfection   B0702-ex2-g1 C0702-ex3-g3

100%   6.1%   44.0%

NUMBER OF CELLS (NUMBER)

EXPRESSION LEVEL OF HLA-B7 (RELATIVE VALUE)

# FIG.12

(a)

| CLONE NO. | HLA-B7 | HLA-C7 |
|-----------|--------|--------|
| #1  | −6 bp | −9 bp |
| #2  | −6 bp | +2 bp |
| #3  | −6 bp | WT |
| #4  | −6 bp | WT |
| #5  | −6 bp | −9 bp |
| #6  | −6 bp | WT |
| #7  | −6 bp | WT |
| #8  | −6 bp | WT |
| #9  | −6 bp | +2 bp |
| #10 | −3 bp | +1 bp |
| #11 | −3 bp | +1 bp |
| #12 | −6 bp | WT |
| #13 | −3 bp | +1 bp |
| #14 | −3 bp | +1 bp |
| #16 | −6 bp | WT |
| #17 | −6 bp | +2 bp |
| #18 | −6 bp | +2 bp |
| #19 | −6 bp | +2 bp |
| #20 | −6 bp | +2 bp |
| #21 | −6 bp | +2 bp |
| #22 | −6 bp | +2 bp |
| #23 | −6 bp | +2 bp |
| #24 | −6 bp | +2 bp |
| #25 | −6 bp | +2 bp |
| #26 | −2 bp | +1 bp |
| #27 | −6 bp | +2 bp |
| #28 | −6 bp | +2 bp |

(b)

|     | HLA-B*07:02 | HLA-C*07:02 |
|-----|-------------|-------------|
| WT  | AAGGCCCAG | GGACACC |
| #2  | A------AG | GGACACC (AA) |
| #11 | A---CCCAG | GGACACC (A) |
| #26 | AA--CCCAG | GGACACG (A) |

# FIG.13

(a)

| CLONE NO. | HLA-C12 |
|-----------|---------|
| #1 | −1 bp |
| #2 | −3 bp |
| #3 | −28 bp |
| #4 | −28 bp |
| #5 | −3 bp |
| #6 | −28 bp |
| #7 | −1 bp |
| #8 | −9 bp |
| #9 | −2 bp |
| #10 | −40 bp |
| #11 | −28 bp |
| #12 | +1 bp |
| #13 | −1 bp |
| #14 | −3 bp |
| #16 | −3 bp |
| #17 | −1 bp |

(b)

| CLONE NO. | HLA-B52 |
|-----------|---------|
| #1-1 | Mut |
| #1-2 | −12 bp |
| #1-3 | WT |
| #1-4 | WT |
| #1-5 | WT |
| #1-6 | Mut |
| #1-7 | WT |
| #1-8 | WT |
| #1-9 | WT |
| #1-10 | −11 bp |
| #1-11 | WT |
| #1-12 | WT |

(c)

|  | HLA-C*12:02 | HLA-B*52:02 |
|------|-------------|-------------|
| WT | GAGGATG | GCATAAC |
| #1-1 | GAG−ATG | GTATGAC |

# FIG.14

(a)

| CLONE NO. | HLA-A01 | HLA-C06 |
|-----------|---------|---------|
| #3 | -8 bp | +8 bp |
| #4 | WT | WT |
| #6 | -24 bp | -28 bp |
| #7 | -8 bp | -4 bp |
| #9 | WT | WT |

(b)

```
A*01:01    GGCAGGACGCCTA   SEQ ID NO: 12      C*06:02    GGATGT
  #3       GGC--------TA                        #3       GGATGT    SEQ ID NO: 13
                                                        ⌐TGTGAGAG⌐
```

# FIG.15

(a)

No Stain          No transfection          B-ex2-g1

NUMBER OF CELLS (NUMBER)

0%          99.7%          31.1%

EXPRESSION LEVEL OF HLA-B7 (RELATIVE VALUE)

(b)

| CLONE NO. | HLA-B07 | HLA-B37 |
|-----------|---------|---------|
| #3-1 | Mut | -9 bp |
| #3-2 | Mut | Mut |
| #3-3 | Mut | Mut |
| #3-4 | WT | WT |
| #3-5 | Mut | -4 bp |
| #3-6 | Mut | -4 bp |
| #3-7 | WT | WT |
| #3-8 | Mut | Mut |
| #3-9 | WT | WT |
| #3-10 | Mut | -3 bp |
| #3-11 | WT | +1 bp |
| #3-12 | WT | -1 bp |

(c)

```
         HLA-B*07:02              HLA-B*37:01
WT       AGTTCGT        WT        AGTTCGT
#3-11    AGTTCGT        #3-11     AGTTCGT
                                   ⌐T⌐
#3-12    AGTTCGT        #3-12     AGT-CGT
```

# FIG.16

(a)

HLA-A

A-ex3-g1

Exon1　Exon2　Exon3　Exon4

HLA-B

Exon1　Exon2　Exon3　Exon4

BC-ex2-g1

HLA-C

Exon1　Exon2　Exon3　Exon4

(b)

No stain

100%

No transfection

0.2%

BC-ex2-g1

14.1%

NUMBER OF CELLS (NUMBER)

EXPRESSION LEVEL OF HLA-BC (RELATIVE VALUE)

(c)

No stain

99.7%

No transfection

0.1%

A-ex3-g1

6.1%

NUMBER OF CELLS (NUMBER)

EXPRESSION LEVEL OF HLA-ABC (RELATIVE VALUE)

# FIG.17

(a)

| CLONE NO. | HLA-A01 | HLA-A24 | HLA-B07 | HLA-B37 | HLA-C06 | HLA-C07 |
|---|---|---|---|---|---|---|
| #1 | +1 bp | +1 bp | −1 bp | −6 bp | +9 bp | −14 bp |

(b)

A*01:01

WT   TCTTGGACCGCGG  SEQ ID NO: 14
#1   TCTTGGACCGCGG  SEQ ID NO: 15
          A

A*24:02

WT   TCTTGGACCGCGG  SEQ ID NO: 16
#1   TCTTGGACCGCGG  SEQ ID NO: 17
          A

B*07:02

WT   AGTATTGGGACCGG  SEQ ID NO: 18
#1   AGTATTGG-ACCGG  SEQ ID NO: 19

B*37:01

WT   TGGGACCGGGAGAC  SEQ ID NO: 20
#1   TGGGA------GAC

C*06:02

WT   GTATTGGGACCGGG  SEQ ID NO: 21
#1   GTATTGGGACCGGGG  SEQ ID NO: 22
        CAATTCTGT

C*07:02

WT   GAGGGGCCGGAGTATTG  SEQ ID NO: 23
#1   GA-------------G

# FIG.18

(a)

(b)

| CLONE NO. | HLA-A24 | HLA-A24 | HLA-B07 | HLA-B52 |
|---|---|---|---|---|
| #1 | +9 bp | +10 bp | −7 bp | −20 bp |
| #3 | −9 bp | −10 bp | −1 bp | +1 bp |
| #5 | +1 bp | +1 bp | WT | +1 bp |
| #7 | +1 bp | +1 bp | +1 bp | −545 bp |
| #8 | +1 bp | ? bp | −4 bp | +1 bp |
| #10 | +1 bp | +1 bp | +1 bp | +1 bp |

(c)

| A*24:02 | GGACCTGCGCTCTTGGACCGCGGCGGACATGGCGGCTCAG | SEQ ID NO: 24 |
| #3 | GGAYCTGASCTCTKGGAAMTGGSSGGTTCRAATCACCARS | SEQ ID NO: 25 |

B*07:02    CACCCAGTTCGTGAGG    SEQ ID NO: 26        B*52:01    CACCCAGTTCGTGA    SEQ ID NO: 28
#3    CACCCAGT−CGTGAGG    SEQ ID NO: 27        #3    CACCCAGTTCGTGA    SEQ ID NO: 29
                                                                         T

(d)

| CLONE NO. | HLA-C07 | HLA-C12 |
|---|---|---|
| #3-1 | WT | −34 bp |
| #3-2 | WT | −1 bp |
| #3-3 | WT | −1 bp |
| #3-4 | WT | WT |

(e)

| C*12:02 | CAGAGGATGTACGGCTGCGACCTGGGGCCCGACGGG | SEQ ID NO: 30 |
| #3-1 | C−−−−−−−−−−−−−−−−−−−−−−−−−−−−−−−−−−−G | |
| #3-2 | CAGAG−ATGTACGGCTGCGACCTGGGGCCCGACGGG | SEQ ID NO: 31 |

# FIG.19

(a)

(b)

(c)

| CLONE NO. | HLA-A01 | HLA-A24 | HLA-B07 | HLA-B37 |
|---|---|---|---|---|
| #1 | −2 bp | +1 bp | +355 bp | +1 bp |
| #3 | +1 bp | +1 bp | +1 bp | WT |
| #4 | −2 bp | +1 bp | +1 bp | −1 bp |
| #9 | +1 bp | +1 bp | −4 bp | +1 bp |
| #14 | +1 bp | +1 bp | +1 bp | +145 bp |
| #17 | −1 bp | +3 bp | +1 bp | −4 bp |
| #23 | +1 bp | −25 bp | +1 bp | +1 bp |

(d)

A*01:01 TTGGACCG    A*24:02 TTGGACCG
#4   TTG--CCG     #4   TTGGACCG
                    A

B*07:02 CCAGTTCG    B*37:01 CCAGTTCG
#4   CCAGTTCG     #4   CCAGT-CG
        A

71

# FIG.20

(a)

(b)

(c)

# FIG.21

# FIG.22

# FIG.23

(a)

(b)

(c)

```
WT   GCCGAGATGTCTCGCTCCGTGGCCTTAGCTGTGCTCGC   SEQ ID NO: 32
#1   GCCGAGATGTCTCGC--CKYGGYCTYCTYTGYTGTCST   SEQ ID NO: 33
```

# FIG.24

(a)

NUMBER OF CELLS (NUMBER)

| No stain | No transfection | B2M-g2 |

100%    0.1%    14.0%

EXPRESSION LEVEL OF HLA-ABC (RELATIVE VALUE)

(b)

```
WT    TCGGGCCGAGATGTCTCGCTCCGTGGCCTT    SEQ ID NO: 34
#1    TCGGGCCGAGATGTCTCGCTCCGTGGCCTT    SEQ ID NO: 35
                         T
#2    TCGGGCCGAGAT------SKCKCTWGTCTC     SEQ ID NO: 36
#3    TCGGGCCGAGATGTCTCGCTCCGTGGCCTT    SEQ ID NO: 37
                         T
#4    TCGGGCCGAGATGTCTCG-----TGGCCTT     SEQ ID NO: 38
```

# FIG.25

# FIG.26

(a)585A1    (b) 585A1-B7⁻C7⁻#2    (c) 585A1-B7⁻C7⁻#11

(d) 585A1-B7⁻C7⁻#26    (e) 1383D2    (f) 604B1-B2M⁻#1

EXPRESSION LEVEL OF CD8 (RELATIVE VALUE)

STAINING INTENSITY OF CFSE (NON-DIVIDING CELL MARKER) (RELATIVE VALUE)

FIG.27

# FIG.28

604B1 iPS cells

HLA-A
HLA-B
HLA-C

Differentiate
CD45+ cells

CD8+ T Cells from PBMCs

Culture with
IL-2 and IFN-r

Proliferation

Sorting

HLA-
reacting
T cells

FIG.29

# FIG.30

HLA-A
HLA-B
HLA-C

Non-edited iPSC

Edited A1⁻ B7⁻ clones

Expanded HLA-reacting T cells

Differentiation

Cardiomyocytes

IFN-γ

$^{51}$Cr

$^{51}$Cr labeling

# FIG.31

# FIG.32

# FIG.33

# FIG.34

(a)  604B1-B2M⁻ #1          (b)  604B1

STAINING INTENSITY OF CFSE (NON-DIVIDING CELL MARKER)
(RELATIVE VALUE)

(c)

RATIO OF NUMBER OF EFFECTOR CELLS TO NUMBER OF TARGET CELLS

## FIG.35

(a) NK cell only  (b) 604B1-wt  (c) 604B1-A1⁻B7⁻ #8  (d) 604B1-A1⁻B7⁻ #10
(e) 1383D2  (f) 604B1-B2M⁻#1  (g) K562

EXPRESSION LEVEL OF CD56 (RELATIVE VALUE)

EXPRESSION LEVEL OF CD107a (RELATIVE VALUE)

## FIG.36

EXPRESSION LEVEL OF NANOG (RELATIVE VALUE)

UNTREATED  IMMEDIATELY AFTER IFN  AFTER 5 DAYS  UNTREATED  IMMEDIATELY AFTER IFN  AFTER 5 DAYS

1383D2 iPS CELL      604B1 iPS CELL

# FIG.37

(a) 604B1

1 mm

(b) 604B1-A1⁻B37⁻C6⁻ #3-11

1 mm

(c) 604B1-A1⁻B37⁻C6⁻ #3-12

1 mm

(d) 604B1-B2M⁻#1

1 mm

(e) 1383D2

1 mm

(f) 1383D2-B2M⁻#1

1 mm

# FIG.38

(a) 604B1

1 mm

(b) 604B1-A1⁻B37⁻C6⁻ #3-11

1 mm

(c) 604B1-A1⁻B37⁻C6⁻ #3-12

1 mm

(d) 604B1-B2M⁻#1

1 mm

(e) 1383D2

1 mm

(f) 1383D2-B2M⁻#1

1 mm

# FIG.39

(a) 1383D2 B2M-KO #1  (b) 585A1-WT

EXPRESSION LEVEL OF CD8 (RELATIVE VALUE)

0.0%

24.4%

STAINING INTENSITY OF CFSE
(NON-DIVIDING CELL MARKER)
(RELATIVE VALUE)

(c)

Specific Lysis (%)

WT

C7 #3-1
B2M⁻
C7 #3-3

RATIO OF NUMBER OF EFFECTOR CELLS TO NUMBER OF TARGET CELLS

# FIG.40

(a) 1383D2 B2M-KO #1  (b) 585A1-WT

EXPRESSION LEVEL OF CD8 (RELATIVE VALUE)

0.8%    11.7%

STAINING INTENSITY OF CFSE
(NON-DIVIDING CELL MARKER)
(RELATIVE VALUE)

(c)

WT

B2M⁻

C7 #3-3

C7 #3-1

RATIO OF NUMBER OF EFFECTOR CELLS TO NUMBER OF TARGET CELLS

# FIG.41

(a) Timeline showing: BEFORE 1 HOUR, 0, AFTER 5 HOURS, AFTER 1 DAY, AFTER 3 DAYS, AFTER 7 DAYS; TRANSPLANTATION AND IVIS CAPTURING; CAPTURING at each timepoint; INJECTION OF T CELL OR MEDIUM (mock)

(b) IVIS images for WT + Mock, WT + T CELL, C7 #3-1 + Mock, C7 #3-1 + T CELL at BEFORE INJECTION OF T CELL, AFTER 5 HOURS, AFTER 1 DAY, AFTER 3 DAYS, AFTER 7 DAYS

(c) Graph of SURVIVAL RATE (%) for C7 #3-1 + T CELL and WT + T CELL across timepoints (BEFORE INJECTION, AFTER 5 HOURS, AFTER 1 DAY, AFTER 3 DAYS, AFTER 7 DAYS)

# FIG.42

# FIG.43

(a)

(b)

# FIG.44

(a)

(b)

# FIG.45

EP 3 754 018 A1

FIG.46

95

# FIG.47

(a)

585A1-C7 RESIDUAL CELL

| HLA-A | HLA-B | HLA-C | DP/DQ/DR |
|-------|-------|-------|----------|
| A*24:02 | B*07:02 | C*07:02 | CIITA |
| A*24:02 | B*52:01 | C*12:02 | CIITA |

585A1-C7 RESIDUAL CELL
(CLASS II DEFICIENCY)      ↓ CIITA KO

| HLA-A | HLA-B | HLA-C | DP/DQ/DR |
|-------|-------|-------|----------|
| A*24:02 | B*07:02 | C*07:02 | CIITA |
| A*24:02 | B*52:01 | C*12:02 | CIITA |

(b)

| Clone ID | CIITA | CIITA |
|----------|-------|-------|
| #3-1-1 | WT | +1 bp |
| #3-1-2 | WT | WT |
| #3-1-3 | +1 bp | −5 bp |
| #3-1-4 | +1 bp | WT |
| #3-1-5 | WT | WT |
| #3-1-6 | +1 bp | −1 bp |
| #3-1-7 | −13 bp | −13 bp |
| #3-1-8 | WT | −1 bp |
| #3-1-9 | −13 bp | +1 bp |
| #3-1-10 | WT | WT |
| #3-1-11 | WT | WT |
| #3-1-12 | WT | +1 bp |
| #3-1-13 | WT | −15 bp |
| #3-1-14 | WT | WT |
| #3-1-18 | +1 bp | +1 bp |
| #3-1-19 | WT | WT |
| #3-1-20 | WT | WT |
| #3-1-21 | +2 bp | −10 bp |
| #3-1-22 | +1 bp | +1 bp |
| #3-1-23 | WT | WT |
| #3-1-24 | WT | WT |
| #3-1-25 | WT | +2 bp |
| #3-1-26 | WT | WT |
| #3-1-27 | WT | +1 bp |
| #3-1-28 | WT | WT |
| #3-1-31 | WT | WT |
| #3-1-32 | +16 bp | −6 bp |
| #3-1-33 | WT | WT |

(c)

TARGET SEQUENCE OF sgRNA-CIITA-ex3g5

| | | |
|---|---|---|
| CIITA | ACACCATCAACTGCGACCAGTTCAGCAGG | SEQ ID NO: 8014 |
| Allele 1 | ACAC-----ACTGCGACCAGTTCAGCAGG | SEQ ID NO: 8015 |
| Allele 2 | ACACCATCAACTGCGACCAGTTCAGCAGG | SEQ ID NO: 8016 |

# FIG.48

# FIG.49

# FIG.50

Ff-XT-28s05 iPSCs

| HLA-A | HLA-B | HLA-C | DP/DQ/DR |
|-------|-------|-------|----------|
| A*24:02 | B*07:02 | C*07:02 | CIITA |
| A*24:02 | B*07:02 | C*07:02 | CIITA |

(b) KNOCKOUTS AT 2 LOCI OF HLA-A AND HLA-B

(a) KNOCKOUTS AT 3 LOCI OF HLA-A, HLA-B, AND CIITA

HLA-C7 RESIDUAL iPSCs

| HLA-A | HLA-B | HLA-C | DP/DQ/DR |
|-------|-------|-------|----------|
| A*~~24~~:02 | B*~~07~~:02 | C*07:02 | CIITA |
| A*~~24~~:02 | B*~~07~~:02 | C*07:02 | CIITA |

(c) KNOCKOUT AT CIITA

HLA-C7 RESIDUAL (CLASS II DEFICIENCY) iPSCs

| HLA-A | HLA-B | HLA-C | DP/DQ/DR |
|-------|-------|-------|----------|
| A*~~24~~:02 | B*~~07~~:02 | C*07:02 | ~~CIITA~~ |
| A*~~24~~:02 | B*~~07~~:02 | C*07:02 | ~~CIITA~~ |

# FIG.51

(a)

(b)

| Clone ID | HLA-A24 | HLA-A24 | HLA-B7 | HLA-B7 | CIITA | CIITA |
|---|---|---|---|---|---|---|
| Ff-XT28s05-ABTo #1 | +1 bp | +1 bp | -3 bp | -1 bp | +1 bp | +2 bp |
| Ff-XT28s05-ABTo #2 | +1 bp | +1 bp | +2 bp | +2 bp | +1 bp | -1 bp |
| Ff-XT28s05-ABTo #3 | +1 bp | +1 bp | -1 bp | -1 bp | +4 bp | -19 bp |
| Ff-XT28s05-ABTo #4 | N.D. | N.D. | N.D. | N.D. | +200 bp | +200 bp |
| Ff-XT28s05-ABTo #5 | N.D. | N.D. | N.D. | N.D. | WT | WT |
| Ff-XT28s05-ABTo #6 | -11 bp | +1 bp | +1 bp | +1 bp | +1 bp | +1 bp |
| Ff-XT28s05-ABTo #7 | N.D. | N.D. | N.D. | N.D. | -1 bp | WT |
| Ff-XT28s05-ABTo #8 | N.D. | N.D. | N.D. | N.D. | N.D. | N.D. |
| Ff-XT28s05-ABTo #9 | N.D. | N.D. | N.D. | N.D. | N.D. | N.D. |
| Ff-XT28s05-ABTo #10 | N.D. | N.D. | N.D. | N.D. | +3 bp | +1 bp |
| Ff-XT28s05-ABTo #11 | N.D. | N.D. | N.D. | N.D. | N.D. | N.D. |
| Ff-XT28s05-ABTo #12 | N.D. | N.D. | N.D. | N.D. | N.D. | N.D. |
| Ff-XT28s05-ABTo #13 | -11 bp | +1 bp | -2 bp | -2 bp | +1 bp | -1 bp |
| Ff-XT28s05-ABTo #14 | N.D. | N.D. | N.D. | N.D. | N.D. | N.D. |
| Ff-XT28s05-ABTo #15 | -544 bp | -544 bp | -9 bp | -9 bp | -10 bp | +1 bp |

# FIG.52

(a)

| Clone ID | HLA-A24 | HLA-A24 | HLA-B7 | HLA-B7 |
|---|---|---|---|---|
| Ff-XT28s05-ABo #1 | +1 bp | -1 bp | WT | +1 bp |
| Ff-XT28s05-ABo #2 | +1 bp | +1 bp | Mut. | Mut. |
| Ff-XT28s05-ABo #3 | WT | +1 bp | WT | WT |
| Ff-XT28s05-ABo #4 | WT | WT | WT | WT |
| Ff-XT28s05-ABo #5 | WT | WT | WT | WT |
| Ff-XT28s05-ABo #6 | WT | WT | WT | WT |
| Ff-XT28s05-ABo #9 | +1 bp | -1 bp | WT | Mut. |
| Ff-XT28s05-ABo #10 | WT | WT | WT | WT |
| Ff-XT28s05-ABo #11 | WT | +1 bp | WT | Mut. |
| Ff-XT28s05-ABo #12 | WT | WT | WT | WT |
| Ff-XT28s05-ABo #13 | +1 bp | +1 bp | WT | +1 bp |
| Ff-XT28s05-ABo #14 | +1 bp | +1 bp | +1 bp | -12 bp |
| Ff-XT28s05-ABo #15 | WT | WT | WT | WT |
| Ff-XT28s05-ABo #16 | WT | +1 bp | WT | WT |
| Ff-XT28s05-ABo #17 | +1 bp | +1 bp | WT | -23 bp |

(b)

| Clone ID | CIITA | CIITA |
|---|---|---|
| Ff-XT28s05-ABo_To # 14-1 | N.D. | N.D. |
| Ff-XT28s05-ABo_To # 14-2 | N.D. | N.D. |
| Ff-XT28s05-ABo_To # 14-3 | +2 bp | +2 bp |
| Ff-XT28s05-ABo_To # 14-4 | +1 bp | +1 bp |
| Ff-XT28s05-ABo_To # 14-5 | +5 bp | +12 bp |
| Ff-XT28s05-ABo_To # 14-6 | +1 bp | +1 bp |
| Ff-XT28s05-ABo_To # 14-7 | +1 bp | +12 bp |
| Ff-XT28s05-ABo_To #14-8 | +1 bp | WT |
| Ff-XT28s05-ABo_To #14-9 | +1 bp | +1 bp |
| Ff-XT28s05-ABo_To #14-10 | +1 bp | WT |
| Ff-XT28s05-ABo_To #14-11 | N.D. | N.D. |
| Ff-XT28s05-ABo_To #14-12 | +1 bp | +1 bp |

# FIG.53

| HLA-C ALLELE | JPN RANK | JPN ALLELE FREQUENCY | EUR RANK | EUR ALLELE FREQUENCY | AFA RANK | AFA ALLELE FREQUENCY | API RANK | API ALLELE FREQUENCY | HIS RANK | HIS ALLELE FREQUENCY |
|---|---|---|---|---|---|---|---|---|---|---|
| *01:02 | 1 | 17.558% | 12 | 2.922% | 19 | 0.850% | 2 | 13.375% | 7 | 4.927% |
| *02:02 | 19 | 0.025% | 10 | 3.729% | 5 | 8.461% | 21 | 0.395% | 10 | 4.227% |
| *03:03 | 2 | 13.116% | 7 | 5.457% | 16 | 1.182% | 8 | 5.023% | 12 | 3.402% |
| *03:04 | 4 | 12.451% | 6 | 8.215% | 8 | 5.330% | 3 | 8.352% | 4 | 6.978% |
| *04:01 | 9 | 4.441% | 3 | 10.534% | 1 | 18.457% | 4 | 8.070% | 1 | 16.508% |
| *05:01 | 15 | 0.376% | 5 | 9.136% | 10 | 3.526% | 18 | 0.847% | 9 | 4.652% |
| *06:02 | 13 | 0.797% | 4 | 9.301% | 4 | 8.855% | 6 | 6.518% | 5 | 5.878% |
| *07:01 | 18 | 0.076% | 1 | 16.658% | 2 | 12.401% | 12 | 3.894% | 3 | 10.355% |
| *07:02 | 3 | 12.634% | 2 | 15.006% | 7 | 6.968% | 1 | 14.560% | 2 | 11.281% |
| *08:01 | 6 | 7.370% | 47 | 0.006% | 24 | 0.124% | 5 | 7.985% | 14 | 2.826% |
| *12:02 | 5 | 11.142% | 16 | 0.934% | 23 | 0.145% | 11 | 4.486% | 17 | 1.301% |
| *16:01 | N/A | 0.000% | 11 | 3.539% | 3 | 9.788% | 25 | 0.169% | 6 | 5.053% |
| TOTAL ALLELE FREQUENCY | | 79.986% | | 85.437% | | 76.087% | | 73.674% | | 77.388% |
| TOTAL COVER RATE | | 95.994% | | 97.879% | | 94.282% | | 93.069% | | 94.887% |

EP 3 754 018 A1

# FIG.54

| Clone ID | B2M | B2M |
|----------|-----|-----|
| Ff-XT28s05-Mo #1 | +1 bp | −1 bp |
| Ff-XT28s05-Mo #2 | −8 bp | −15 bp |
| Ff-XT28s05-Mo #3 | +1 bp | +1 bp |
| Ff-XT28s05-Mo #4 | WT | WT |
| Ff-XT28s05-Mo #5 | +1 bp | +6 bp |

# FIG.55

| Clone ID | B2M | B2M | CIITA | CIITA |
|----------|-----|-----|-------|-------|
| Ff-XT28s05-MTo #1 | −21 bp | −2 bp | WT | WT |
| Ff-XT28s05-MTo #2 | +1 bp | WT | +1 bp | +1 bp |
| Ff-XT28s05-MTo #3 | +1 bp | WT | WT | WT |
| Ff-XT28s05-MTo #4 | +1 bp | WT | WT | −13 bp |
| Ff-XT28s05-MTo #5 | +1 bp | +1 bp | −15 bp | +1 bp |
| Ff-XT28s05-MTo #6 | +1 bp | +1 bp | +1 bp | WT |
| Ff-XT28s05-MTo #7 | +1 bp | +1 bp | +1 bp | WT |
| Ff-XT28s05-MTo #8 | +1 bp | +1 bp | −7 bp | +1 bp |
| Ff-XT28s05-MTo #9 | +1 bp | WT | −20 bp | WT |
| Ff-XT28s05-MTo #10 | +1 bp | +1 bp | +1 bp | WT |
| Ff-XT28s05-MTo #11 | +1 bp | −1 bp | +1 bp | WT |
| Ff-XT28s05-MTo #12 | +1 bp | +1 bp | +1 bp | WT |
| Ff-XT28s05-MTo #13 | +1 bp | +1 bp | −1 bp | WT |
| Ff-XT28s05-MTo #14 | +1 bp | +1 bp | +1 bp | WT |
| Ff-XT28s05-MTo #15 | +1 bp | +1 bp | −18 bp | −27 bp |
| Ff-XT28s05-MTo #16 | WT | WT | WT | WT |
| Ff-XT28s05-MTo #17 | −13 bp | −8 bp | WT | WT |
| Ff-XT28s05-MTo #18 | +1 bp | +1 bp | −29 bp | WT |
| Ff-XT28s05-MTo #19 | +1 bp | +1 bp | −1 bp | WT |

# FIG.56

(a)

(b)

No Stain

Ff-XT28s05-MTo #2

NUMBER OF CELLS (NUMBER)

0%

99.7%

EXPRESSION LEVEL OF HLA-ABC (RELATIVE VALUE)

# FIG.57

585A1-B2M-KO

No stain

No TF

B2M-g2

Cell number

100%

0.1%

27.0%

HLA-ABC expression

# FIG.58

| Clone ID | HLA-C07 | HLA-C12 |
|----------|---------|---------|
| #3-2 | WT | −3 bp |
| #3-3 | WT | +198 bp |
| #3-4 | −2 bp | +197 bp |
| #3-5 | +1 bp | −21 bp |
| #3-6 | +1 bp | −7 bp |
| #3-7 | +2 bp | −7 bp |

# FIG.59

| Clone ID | HLA-C07 | HLA-C12 |
|----------|---------|---------|
| #3-1 | +1 bp | WT |
| #3-2 | +1 bp | WT |
| #3-3 | +1 bp | WT |
| #3-4 | WT | WT |
| #3-5 | +1 bp | WT |
| #3-6 | +1 bp | WT |

# FIG.60

| Clone ID | HLA-C06 | HLA-C07 |
|----------|---------|---------|
| #4-1 | WT | WT |
| #4-2 | WT | −4 bp |
| #4-3 | WT | WT |
| #4-4 | WT | WT |
| #4-5 | WT | WT |
| #4-6 | WT | +1 bp |
| #4-7 | WT | WT |
| #4-8 | WT | WT |
| #4-10 | WT | +1 bp |
| #4-11 | WT | +1 bp |
| #4-12 | WT | WT |

## INTERNATIONAL SEARCH REPORT

| International application No. |
| --- |
| PCT/JP2019/005524 |

A. CLASSIFICATION OF SUBJECT MATTER
Int.Cl. C12N15/09(2006.01)i, C12N5/10(2006.01)i, C12N15/11(2006.01)i, C12Q1/04(2006.01)i, G01N33/53(2006.01)i

According to International Patent Classification (IPC) or to both national classification and IPC

B. FIELDS SEARCHED

Minimum documentation searched (classification system followed by classification symbols)
Int.Cl. C12N15/09, C12N5/10, C12N15/11, C12Q1/04, G01N33/53

| Documentation searched other than minimum documentation to the extent that such documents are included in the fields searched | |
| --- | --- |
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/REGISTRY (STN), GenBank/EMBL/DDBJ/GeneSeq

C. DOCUMENTS CONSIDERED TO BE RELEVANT

| Category* | Citation of document, with indication, where appropriate, of the relevant passages | Relevant to claim No. |
| --- | --- | --- |
| X | WO 2016/201047 A1 (EDITAS MEDICINE, INC.) 15 December 2016, claims, page 176, examples & JP 2018-523977 A & US 2018/0296603 A1 & EP 3307887 A1 & KR 10-2018-0031671 A & CN 108026526 A | 1-2, 5, 10-13, 15, 21, 24-25, 29-30 |
| Y | | 1-30 |
| X | WO 2016/183041 A2 (PRESIDENT AND FELLOWS OF HARVARD COLLEGE) 17 November 2016, claims, examples & JP 2018-515139 A & EP 3294342 A2 & CN 107921148 A | 6-13, 17-21, 25 |
| Y | | 1-30 |

☒ Further documents are listed in the continuation of Box C.　　☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 23 April 2019 (23.04.2019) | 14 May 2019 (14.05.2019) |

| Name and mailing address of the ISA/ Japan Patent Office 3-4-3, Kasumigaseki, Chiyoda-ku, Tokyo 100-8915, Japan | Authorized officer Telephone No. |
| --- | --- |

Form PCT/ISA/210 (second sheet) (January 2015)

<div align="center">

**INTERNATIONAL SEARCH REPORT**

</div>

| | International application No. |
|---|---|
| | PCT/JP2019/005524 |

C (Continuation).    DOCUMENTS CONSIDERED TO BE RELEVANT

| Category* | Citation of document, with indication, where appropriate, of the relevant passages | Relevant to claim No. |
|---|---|---|
| X<br>Y | JP 2016-513460 A (SANGAMO BIOSCIENCES, INC.) 16 May 2016, example 8 & WO 2014/165177 A1, example 8 & US 2015/0037304 A1 & EP 2970886 A1 & CN 105283539 A | 6-13<br>1-30 |
| X<br>Y | JP 2017-523813 A (COLLEGE OF MEDICINE POCHON CHA UNIVERSITY INDUSTRY-ACADEMIC COOPERATION FOUNDATION) 24 August 2017, claims, examples & WO 2016/021972 A1, claims, examples & US 2017/0327795 A1 & EP 3194578 A1 & KR 10-2016-0018425 A & CN 106536721 A | 10-13, 15,<br>1-30 |

Form PCT/ISA/210 (continuation of second sheet) (January 2015)

**REFERENCES CITED IN THE DESCRIPTION**

**Patent documents cited in the description**

- JP 2018026421 A

- WO 2012145384 PCT